# EUROPEAN PATENT SPECIFICATION

(11) **EP 1 942 933 B1**
(45) Date of publication and mention of the grant of the patent: **17.10.2012**
(21) Application number: 06789953.4
(22) Date of filing: 23.08.2006
(51) Int. Cl.: A61K 39/145

(54) **CANINE INFLUENZA VACCINES**
HUNDE-INFLUENZA-IMPFSTOFFE
VACCINS CONTRE LA GRIPPE CANINE

(30) Priority: 25.08.2005 US 211983; 01.11.2005 US 264622
(43) Date of publication of application: 16.07.2008
(73) Proprietor: Merial Limited, Duluth, GA 30096 (US)
(72) Inventor: MINKE, Jules, Maarten, F-69906 Corbas (FR); KARACA, Kemal, Athens, GA 30306 (US); YAO, Jiansheng, North York, ON M2H 3H1 (CA)
(74) Representative: Harding, Charles Thomas
(86) International application number: PCT/US2006/032914
(87) International publication number: WO 2007/024947

(56) References cited:
- WO-A-99/44633
- WO-A1-2006/116082
- "The Detectives" CORNELL VETERINARY MAGAZINE, [Online] 2004, pages 10-13, XP002427524 Retrieved from the Internet: URL:http://www.vet.cornell.edu/news/cvmaga zine/Fall04/detectives.pdf>
- WOOD J M ET AL: "The standardization of inactivated equine influenza vaccines by single-radial immunodiffusion" JOURNAL OF BIOLOGICAL STANDARDIZATION, ACADEMIC PRESS, LONDON, GB, vol. 11, no. 2, April 1983 (1983-04), pages 133-136, XP002418529 ISSN: 0092-1157
- STEPHENSEN CB ET AL: "Canine Distemper Virus (CDV) infection of ferrets as a model for testing Morbillivirus strategies: NYVAC- and ALVAC-based CDV recombinants protect against symptomatic infection" JOURNAL OF VIROLOGY, THE AMERICAN SOCIETY FOR MICROBIOLOGY, US, vol. 71, no. 2, February 1997 (1997-02), pages 1506-1513, XP002099275 ISSN: 0022-538X
- DUBOVI EJ, CRAWFORD PC, DONIS RO, CASTELMAN WL, STEPHENSON I, GIBBS EPJ: "Isolation of Equine Influenza Virus from Racing Greyhounds with Fatal Hemorrhagic Pneumonia" PROCEEDINGS OF THE 47TH ANNUAL MEETING OF AMERICAN ASSOCIATION OF VETERINARY LABORATORY DIAGNOSTICIANS, October 2004 (2004-10), page 158, XP009081246
- "UF Researchers: Equine Influenza Virus Likely Involved In Recent Respiratory Disease Outbreak In Racing Greyhounds" THE VETERINARY PAGE - NEWS FROM THE UNIVERSITY OF FLORIDA, COLLEGE OF VETERINARY MEDICINE, [Online] April 2004 (2004-04), XP002427526 Retrieved from the Internet: URL:http://www.vetmed.ufl.edu/pr/vp/2004/a pr04.pdf>
- KARACA KEMAL ET AL: "Evaluation of the ability equine influenza virus of canarypox-vectored vaccines to induce humoral immune responses against canine influenza viruses in dogs" AMERICAN JOURNAL OF VETERINARY RESEARCH, vol. 68, no. 2, February 2007 (2007-02), pages 208-212, XP009081173 ISSN: 0002-9645
- ENSERINK MARTIN: "Epidemiology. Horse flu virus jumps to dogs" SCIENCE, AMERICAN ASSOCIATION FOR THE ADVANCEMENT OF SCIENCE,, US, vol. 309, no. 5744, 30 September 2005 (2005-09-30), page 2147, XP002418480 ISSN: 0036-8075
- CRAWFORD P C ET AL: "Transmission of Equine influenza virus to dogs" SCIENCE, AMERICAN ASSOCIATION FOR THE ADVANCEMENT OF SCIENCE,, US, vol. 310, no. 5747, 21 October 2005 (2005-10-21), pages 482-485, XP003003531 ISSN: 0036-8075
- EDLUND TOULEMONDE C ET AL: "Efficacy of a recombinant equine influenza vaccine against challenge with an American lineage H3N8 influenza virus responsible for the 2003 outbreak in the United Kingdom." THE VETERINARY RECORD 19 MAR 2005, vol. 156, no. 12, 19 March 2005 (2005-03-19), pages 367-371, XP009081274 ISSN: 0042-4900 cited in the application
- TUMOVA ET AL: "Equine influenza - A segment in influenza virus ecology", COMPARATIVE IMMUNOLOGY, MICROBIOLOGY AND INFECTIOUS DISEASES, PERGAMON PRESS, OXFORD, GB, vol. 3, no. 1-2, 1 January 1980 (1980-01-01), pages 45-59, XP023686506, ISSN: 0147-9571, DOI: 10.1016/0147-9571(80)90038-7 [retrieved on 1980-01-01]

## Description

### INCORPORATION BY REFERENCE

This application claims priority from U.S. Application Serial No. 11/264,622 filed November 1, 2005 which claims priority from U.S. Application Serial No. 11/211,983 filed August 25, 2005.

### FIELD OF THE INVENTION

The present description encompasses influenza vaccines, in particular canine influenza vaccines. The vaccine may be a recombinant poxvirus vaccine or an inactivated vaccine.

### BACKGROUND OF THE INVENTION

Respiratory diseases resembling influenza has infected thousands of dogs in the U.S. Recurrent outbreaks of severe respiratory disease characterized by coughing and fever have occurred in greyhounds at racing kennels. Pathological findings included severe pulmonary and plural hemorrhage, accurate to subacute erosive to hyperplastic tracheitis, bronchitis and bronchiolitis and bronchopneumonia. In 2004, eight greyhounds in Jacksonville, Florida were killed by an equine influenza virus that jumped the species barrier from horses to dogs.

Equine influenza is a disease of horses, and the virus is in the same group of viruses that cause flu in people. The disease is present in horse populations throughout Europe, North America and parts of Asia, with horses typically developing a fever and a dry hacking cough. In the early stages of the disease, horses are reluctant to eat or drink for several days, but usually recover in two to three weeks.

H3N8 subtypes of equine influenza were previously isolated from lungs of two dogs from Florida and one dog fromTexas who had died from the infection. Genetic sequence analyses and phylogenetic comparisons determined that all three canine isolates were closely related and evolved from contemporary strains of equine influenza H3N8. Immunohistochemistry demonstrated influenza antigen in bronchial gland epithelial cells, bronchial and bronchiolar epithelial cells and in alveolar macrophages. Seroconversion to canine influenza virus was demonstrated by hemagglutination inhibition and microneutralization assays.

Molecular and antigenic analyses of three influenza viruses isolated from outbreaks of severe respiratory disease in racing greyhounds revealed that they are closely related to H3N8 equine influenza virus (see, e.g., Crawford et al., Science. 2005 Oct 21;310(5747):482-5. Epub 2005 Sep 26). Phylogenetic analysis indicated that the canine influenza virus genomes form a monophyletic group, consistent with a single interspecies virus transfer. Molecular changes in the hemagglutinin suggested adaptive evolution in the new host. The etiologic role of this virus in respiratory disease was supported by the temporal association of rising antibody titers with disease and by experimental inoculation studies. The geographic expansion of the infection and its persistence for several years indicate efficient transmission of canine influenza virus among greyhounds. Evidence of infection in pet dogs suggests that this infection may also become enzootic in this population.

In 2005, a mutated form of the Bird Flu (H3N8 Virus) was reported to have killed greyhounds in Massachusetts

("The detectives", Cornell Veterinary Magazine, 2004, p. 10-13, URL:http://www.vet.cornell.edu/news/cvmagazine/Fall04/ detectives.pdf)

Accordingly, there is a need for an effective vaccine against influenza in canines. Citation or identification of any document in this application is not an admission that such document is available as prior art to the present invention.

### SUMMARY OF THE INVENTION

The present invention provides the use of a formulation comprising an avipox expression vector comprising a polynucleotide encoding an equine influenza antigen and a pharmaceutically or veterinarily acceptable carrier, excipient or vehicle in an effective amount for eliciting a protective immune response, for the manufacture of a medicament for eliciting a protective immune response against influenza in a canine;
wherein the avipox expression vector is an attenuated avipox expression vector, wherein the avipox expression vector is a canarypox vector and wherein the canarypox vector is ALVAC; and
wherein the equine influenza antigen is a hemagglutinin wherein the hemagglutinin is H3.

In another aspect, the present invention provides a formulation comprising an avipox expression vector comprising a polynucleotide encoding an equine influenza antigen and a pharmaceutically or veterinarily acceptable carrier, excipient or vehicle in an effective amount for eliciting a protective immune response, for use in eliciting a protective immune response against influenza in a canine;
wherein the avipox expression vector is an attenuated avipox expression vector, wherein the avipox expression vector is a canarypox vector and wherein the canarypox vector is ALVAC; and
wherein the influenza antigen is a hemagglutinin wherein the hemagglutinin is H3.

In a further aspect, the present invention provides the use of a formulation comprising an inactivated influenza vaccine and a pharmaceutically acceptable carrier in an effective amount for eliciting a protective immune response in a canine, for the manufacture of a medicament for eliciting a protective immune response against influenza in a canine; wherein said inactivated influenza vaccine is an inactivated equine influenza of serotype H3; wherein the inactivated influenza vaccine is an inactivated equine influenza isolate and wherein the equine influenza isolate is chosen from the group consisting of an Ohio equine influenza isolate, a Kentucky equine influenza isolate, a Newmarket equine influenza isolate or mixtures thereof.

The present invention provides, in another aspect a formulation comprising an inactivated influenza vaccine and a pharmaceutically acceptable carrier in an effective amount for eliciting a protective immune response in a canine, for use in eliciting a protective immune response against influenza in a canine; wherein said inactivated influenza vaccine is an inactivated equine influenza of serotype H3; wherein the inactivated influenza vaccine is an inactivated equine influenza isolate and wherein the equine influenza isolate is chosen from the group consisting of an Ohio equine influenza isolate, a Kentucky equine influenza isolate, a Newmarket equine influenza isolate or mixtures thereof.

The present description encompasses canine influenza vaccines, which may be a recombinant canine influenza vaccine or an inactivated canine influenza vaccine.

In an embodiment wherein the canine influenza vaccine is a recombinant vaccine, advantageously, the vector is an avipox expression vector which may comprise a polynucleotide encoding an influenza antigen, epitope or immunogen. The influenza antigen, epitope or immunogen may be a hemagglutinin, matrix protein, neuraminidase, nonstructural protein, nucleoprotein, polymerase or any fragment thereof.

In an advantageous embodiment, the canine influenza antigen, epitope or immunogen is derived from a canine infected with influenza. For example, but not by limitation, influenza virus may be isolated from the broncho alveolar lavage and/or lung tissues of an affected dog. Isolation and characterization of the nucleotide sequence of the influenza infecting the dog may be done by routine experimentation by a person of ordinary skill in the art.

The canine influenza antigen, epitope or immunogen may be isolated from an equine influenza. The equine influenza may be an Ohio equine influenza, Kentucky equine influenza or a Newmarket equine influenza.

The avipox expression vector may be an attenuated avipox expression vector. In one embodiment, the avipox expression vector may be a canarypox vector, advantageously ALVAC. The influenza antigen, epitope or immunogen may be a hemagglutinin, such as H3. The canarypox vector may be CP 2242, CP1529 or CP1533.

The present description also encompasses an inactivated influenza vaccine. The inactivated influenza vaccine may be an inactivated canine influenza. In another embodiment, the inactivated influenza vaccine may be an equine influenza, advantageously an Ohio equine influenza, Kentucky equine influenza or a Newmarket equine influenza. The vaccine may be inactivated with formalin or beta-propiolactone.

The description also relates to method of eliciting an immune response against influenza in a canine, which may comprise administering a formulation comprising any one of the above recombinant influenza vaccine or inactivated vaccine and a pharmaceutically or veterinarily acceptable carrier, excipient or vehicle in an effective amount for eliciting an immune response. In an advantageous embodiment, an adjuvant may be added. The adjuvant may be aluminum hydroxide, alumimum phosphate, a carbomer or an oil-water-emulsion and optionally may comprise CpG. Advantageously, the administration may be subcutaneous or intramuscular.

The description further relates to method of inducing an immune response against influenza in a canine, which may comprise administering a formulation comprising any one of the above recombinant influenza vaccine or inactivated vaccine and a pharmaceutically or veterinarily acceptable carrier, excipient or vehicle in an effective amount for inducing an immune response. In an advantageous embodiment, an adjuvant may be added. The adjuvant may be aluminum hydroxide, alumimum phosphate, a carbomer or an oil-water-emulsion and optionally may comprise CpG. Advantageously, the administration may be subcutaneous or intramuscular.

The description further encompasses a kit for performing a method of eliciting or inducing an immune response which may comprise any one of the recombinant influenza vaccines or inactivated vaccines and instructions for performing the method.

These and other embodiments are disclosed or are obvious from and encompassed by, the following Detailed Description.

### BRIEF DESCRIPTION OF THE DRAWINGS

The following detailed description, given by way of example, but not intended to limit the invention solely to the specific embodiments described, may best be understood in conjunction with the accompanying drawings, in which:
FIG. 1 illustrates the sequence of the insert in PJT004 (SEQ ID NO: 1);
FIG. 2 illustrates the sequence of the insert in PJT005 (SEQ ID NO: 2);
FIG. 3 illustrates a comparison of the amino acid sequence of EIV Ohio 03 strain HA to that of New Market strain H3 HA (SEQ ID NOS: 3 and 4);
FIG. 4A illustrates a construction of an ALVAC donor plasmid for generation of an ALVAC recombinant expressing codon optimized EIV H3 HA (Ohio 03);
FIG. 4B illustrates pALVAC C5 H6p-synthetic EIV H3 HA, pJY1571.1;
FIG. 5A illustrates a predicted amino acid sequence of product(s): EIV H3 HA;
FIG. 5B illustrates a nucleotide sequence of arms and insert with translation
FIG. 6 illustrates a vCP2242 Western blot analysis. A 1/1,000 dilution of pooled anti-EIV antibody was used for the analysis. Lane 1: 5 µl Fermentas Prestain protein marker, lane 2: 15 µl ALVAC cell pellet, lane 3: 15 µl vCP2242. cell pellet, lane 4: 15 µl vCP2242. cell pellet, lane 5: 15 µl vCP1533 cell pellet, lane 6: space, lane 7: 40 µl ALVAC supernatant, lane 8: 40 µl vCP2242. supernatant, lane 9: 40 µl vCP2242. supernatant and lane 10: 40 µl vCP1533 supernatant.

### DETAILED DESCRIPTION

The present invention is based, in part, on Applicants' studies demonstrating a recombinant canarypox expressing equine influenza HA is immunogenic in dogs.

The present description encompasses any influenza antigen, epitope or immunogen that elicits an immunogenic response in an animal, advantageously a vertebrate, more advantageously a dog. The influenza antigen, epitope or immunogen may be any influenza antigen, epitope or immunogen, such as, but not limited to, a protein, peptide or fragment thereof, that elicits, induces or stimulates a response in an animal, advantageously a vertebrate, more advantageously a dog.

In an advantageous embodiment, the canine influenza antigen, epitope or immunogen is derived from a canine infected with influenza. For example, but not by limitation, influenza virus may be isolated from the broncho alveolar lavage and/or lung tissues of an affected dog. Isolation and characterization of the nucleotide sequence of the influenza infecting the dog may be done by routine experimentation by a person of ordinary skill in the art.

In another advantageous embodiment, the canine influenza, antigen, epitope or immunogen may be derived from an equine infected with influenza or an equine influenza strain. Advantageously, the equine influenza strain is an Ohio equine influenza, Kentucky equine influenza strain or a Newmarket equine influenza strain. The canine influenza antigen, epitope or immunogen may be determined by one of ordinary skill of the art from the nucleotide sequences of Examples 4 and 5. Advantageously, the canine influenza antigen, epitope or immunogen is a hemagglutinin (HA) (e.g., HA precursor, H1, H2, protein, matrix protein (e.g., matrix protein M1 or M2), neuraminidase, nonstructural (NS) protein (e.g., NS1 or NS2), nucleoprotein (NP) and polymerase (e.g., PA polymerase, PB1 polymerase 1 or PB2 polymerase 2).

Examples of Kentucky equine influenza strains that may be used in methods of the present description include, but are not limited to, equine influenza strains A/eq/Kentucky/98 (see, e.g., Crouch et al., Vaccine. 2004 Dec 2;23(3):418-25), A/Equi 2 (Kentucky 81) (see, e.g., Short et al., J Vet Pharmacol Ther. 1986 Dec;9(4):426-32, Homer & Ledgard, N Z Vet J. 1988 Dec;36(4):205-6), A/equine/Kentucky/1/81 (Eq/Ky) (see, e.g., Breathnach et al., Vet Immunol Immunopathol. 2004 Apr;98(3-4):127-36), A/Equine/Kentucky/1/81 (H3N8) (see, e.g., Olsen et al., Vaccine. 1997 Jul;15(10):1149-56, Morley et al. Vet Microbiol. 1995 Jun;45(1):81-92, Ozaki et al., Vet Microbiol. 2001 Sep 20;82(2):111-9, Sugiura et al., J Virol Methods. 2001 Oct;98(1):1-8, see, e.g., Sugiura et al., J Virol Methods. 2001 Oct;98(1):1-8, Goto et al., J Vet Med Sci. 1993 Feb;55(1):33-7, Goto et al., J Vet Med Sci. 1993 Feb;55(1):33-7), A/Equine/Kentucky/1/91 (H3N8) (see, e.g., Youngner et al., Am J Vet Res. 2001 Aug;62(8):1290-4), A/Equine/Kentucky/1277/90 (Eq/Kentucky) (see, e.g., Webster & Thomas, Vaccine. 1993;11(10):987-93), A/Equine/Kentucky/2/91 (H3N8) (see, e.g., Donofrio et al., J Vet Diagn Invest. 1994 Jan;6(1):39-43), A/Equine/Kentucky/79 (H3N8) (see, e.g., Donofrio et al., J Vet Diagn Invest. 1994 Jan;6(1):39-43), A/equine/Kentucky/81 (see, e.g., Sugiura et al., J Virol Methods. 2001 Oct;98(1):1-8), A/equine/Kentucky/91 (H3N8) (see, e.g., Gross et al., Equine Vet J. 1998 Nov;30(6):489-97), A/equine-2/Kentucky/95 (H3N8) (see, e.g., Heldens et al., Vet J. 2004 Mar;167(2):150-7) and A/equine-2/Kentucky/98 (see, e.g., Chambers et al., Equine Vet J. 2001 Nov;33(7):630-6),

Examples of Newmarket equine influenza strains that may be used in methods of the present description include, but are not limited to, equine influenza strains A/eq/Newmarket/1/77 (see, e.g., Lindstrom et al., Arch Virol. 1998;143(8):1585-98), A/eq/Newmarket/5/03 (see, e.g., Edlund Toulemonde et al., Vet Reo. 2005 Mar 19;156(12):367-71), A/Equi 2 (H3N8), Newmarket 1/93 (see, e.g., Mohler et al., Biotechnol Bioeng. 2005 Apr 5;90(1):46-58, Nayak et al., J Chromatogr B Analyt Technol Biomed Life Sci. 2005 Jul 8), A/equi-2/Newmarket-1/93 (see, e.g., Heldens et al., J Immunol Methods. 2002 Jun 1;264(1-2):11-7), A/equine/Newmarket/2/93 (see, e.g., Wattrang et al., Viral Immunol. 2003;16(1):57-67), A/equine/Newmarket/79 (H3N8) (see, e.g., Duhaut & Dimmock, Virology. 2000 Sep 30;275(2):278-85, Noble & Dimmock, J Gen Virol. 1994 Dec;75 (Pt 12):3485-91, Duhaut & Dimmock, Virology. 1998 Sep 1;248(2):241-53, Hannant & Mumford, Vet Immunol Immunopathol. 1989 Jul;21(3-4):327-37, Hannant et al., Vet Microbiol. 1989 Apr;19(4):293-303, Hannant et al., Vet Rec. 1988 Feb 6;122(6):125-8, Richards et al., Vet Immunol Immunopathol. 1992 Jun;33(1-2):129-43, Heldens et al., Vet J. 2004 Mar;167(2):150-7), A/equine/Newmarket/1/77 (H7N7) (see, e.g., Goto et al., J Vet Med Sci. 1993 Feb;55(1):33-7, Sugiura et al., J Virol Methods. 2001 Oct;98(1):1-8, Sugiura et al., J Virol Methods. 2001 Oct;98(1):1-8) and A/equine-2/Newmarket-2/93 (see, e.g., Heldens et al., Vet J. 2004 Mar;167(2):150-7).

The present description also encompasses other equine influenza viruses, such as, but not limited to, equine influenza virus A/eq/Miami/63 (H3N8) (see, e.g., van Maanen et al., Vet Microbiol. 2003 Jun 10;93(4):291-306), A/equi 1 (Prague strain) (see, e.g., Homer & Ledgard, N Z Vet J. 1988 Dec;36(4):205-6, Short et al., J Vet Pharmacol Ther. 1986 Dec;9(4):426-32), A/Equi 2 (Miami) (see, e.g., Short et al., J Vet Pharmacol Ther. 1986 Dec;9(4):426-32), A/equi-1/Prague/56 (Pr/56) (see, e.g., Heldens et al., J Immunol Methods. 2002 Jun 1;264(1-2):11-7), A/equi-2/Suffolk/89 (Suf/89) (see, e.g., Heldens et al., J Immunol Methods. 2002 Jun 1;264(1-2):11-7), A/Equine 2/Sussex/89 (H3N8) (see, e.g., Mumford et al., Vet Rec. 1994 Feb 12;134(7):158-62), A/equine/Sussex/89 (see, e.g., Wattrang et al., Viral Immunol. 2003;16(1):57-67), A/equine-2/Saskatoon/90 (see, e.g., Chambers et al., Equine Vet J. 2001 Nov;33(7):630-6), A/Equine/Prague/1/56 (H7N7) (see, e.g., Donofrio et al., J Vet Diagn Invest. 1994 Jan;6(1):39-43, Morley et al. Vet Microbiol. 1995 Jun;45(1):81-92),, A/equine/Miami/1/63 (H3N8) (see, e.g., Morley et al. Vet Microbiol. 1995 Jun;45(1):81-92, Ozaki et al., Vet Microbiol. 2001 Sep 20;82(2):111-9, Thomson et al, Vet Rec. 1977 May 28;100(22):465-8, Mumford et al., Epidemiol Infect. 1988 Jun;100(3):501-10, Donofrio et al., J Vet Diagn Invest. 1994 Jan;6(1):39-43, Mumford et al., J Hyg (Lond). 1983 Jun;90(3):385-95), A/Aichi/2/68 (H3N2) (see, e.g., Ozaki et al., Vet Microbiol. 2001 Sep 20;82(2):111-9), A/equine/Tokyo/2/71 (H3N8) (see, e.g., Goto et al., J Vet Med Sci. 1993 Feb;55(1):33-7), A/eq/LaPlata/1/88 (see, e.g., Lindstrom et al., Arch Virol. 1998;143(8):1585-98), A/Equine/Jilin/1/89 (Eq/Jilin) (see, e.g., Webster & Thomas, Vaccine. 1993;11(10):987-93), A/Equine/Alaska/1/91 (H3N8) (see, e.g., Webster & Thomas, Vaccine. 1993;11(10):987-93), A/equine/Saskatoon/1/91 (H3N8) (see, e.g., Morley etal. Vet Microbiol. 1995 Jun;45(1):81-92), A/equine/Rome/5/91 (H3N8) (see, e.g., Sugiura et al., J Virol Methods. 2001 Oct;98(1):1-8), A/equine/La Plata/1/93 (H3N8) (see, e.g., Ozaki et al., Vet Microbiol. 2001 Sep 20;82(2):111-9), A/equine/La Plata/1/93 (LP/93) (see, e.g., Sugiura et al., J Virol Methods. 2001 Oct;98(1):1-8), A/eq/Holland/1/95 (H3N8) (see, e.g., van Maanen et al., Vet Microbiol. 2003 Jun 10;93(4):291-306) and A/eq/Holland/2/95 (H3N8) (see, e.g., van Maanen et al., Vet Microbiol. 2003 Jun 10;93(4):291-306)

In another advantageous embodiment, the canine influenza, antigen, epitope or immunogen may be derived from an equine infected with influenza or an equine influenza strain derived from a recent isolate.

The influenza antigen, epitope or immunogen may also be isolated from any influenza strain such as, but not limited to, avian H5N1 influenza virus, A/Hong Kong/156/97 (A/HK/156/97) (see, e.g., Leneva et al., Antimicrob Agents Chemother. 2001 Apr;45(4):1216-24), avian H7N1 influenza strain (see, e.g., Foni et al., New Microbiol. 2005 Jan;28(1):31-5), avian H9N2 influenza virus (see, e.g., Leneva et al., Antimicrob Agents Chemother. 2001 Apr;45(4):1216-24), avian influenza virus A/Chicken/HK/G9/97 (H9N2) (see, e.g., Leneva et aL, Antimicrob Agents Chemother. 2001 Apr;45(4):1216-24), avian influenza virus A/Quail/HK/G1/97 (H9N2) (see, e.g., Leneva et al., Antimicrob Agents Chemother. 2001 Apr;45(4):1216-24), avian influenza virus A/Teal/HK/W312/97 (H6N1) (see, e.g., Leneva et al., Antimicrob Agents Chemother. 2001 Apr;45(4):1216-24), avian pandemic influenza A viruses of avian origin (see, e.g., Audsley & Tannock, Expert Opin Biol Ther. 2004 May;4(5):709-17), cold-adapted (ca) and temperature sensitive (ts) master donor strain, A/Leningrad/134/17/57 (H2N2) (see, e.g., Youil et al., Virus Res. 2004 Jun 15;102(2):165-76), equine influenza virus (A/Equi 2 (H3N8), Newmarket 1/93) (see, e.g., Mohler et al., Biotechnol Bioeng. 2005 Apr 5;90(1):46-58; Nayak et al., J Chromatogr B Analyt Technol Biomed Life Sci. 2005 Jul 8), equine-2 influenza virus (EIV; subtype H3N8) (see, e.g., Virology. 2001 Aug 15;287(1):202-13), equine-2 influenza virus, A/Equine/Kentucky/1/91 (H3N8) (see, e.g., Youngner et al., Am J Vet Res. 2001 Aug;62(8):1290-4), human influenza virus A(H3N2) isolates (see, e.g., Abed et al., J Infect Dis. 2002 Oct 15;186(8):1074-80), human influenza virus A/Memphis/1/71 (H3N2) (see, e.g., Suzuki et al., Biochem J. 1996 Sep 1;318 (Pt 2):389-93), human influenza virus A/Nanchang/933/95 (H3N2) virus (see, e.g., Scholtissek et al., J Virol. 2002 Feb;76(4):1781-6), human influenza virus A/PR/8/34 (H1N1) virus (see, e.g., Scholtissek et al., J Virol. 2002 Feb;76(4):1781-6), human influenza virus A/Singapore/57 (H2N2) virus (see, e.g., Scholtissek et al., J Virol. 2002 Feb;76(4):1781-6), influenza virus A (see, e.g., Chare et al., J Gen Virol. 2003 Oct;84(Pt 10):2691-703), influenza virus A PR/8/34 (PR8) virus (H1N1 subtype) (see, e.g., Mantani et al., Planta Med. 2001 Apr;67(3):240-3), influenza virus A/Aichi/2/68(H3N2) (see, e.g., Miyamoto et al., Antiviral Res. 1998 Aug;39(2):89-100), influenza virus A/Ann Arbor/6/60 cold-adapted virus (see, e.g., Treanor et al., J Virol. 1994 Dec;68(12):7684-8), influenza virus A/Beijing 32/92 (H3N2) (see, e.g., Zakay-Rones et al., J Altern Complement Med. 1995 Winter;1(4):361-9), influenza virus A/Charlottesville/31/95 (H1N1) (see, e.g., Gubareva et al., J Gen Virol. 2002 Nov;83(Pt 11):2683-92), influenza virus A/Kawasaki/86 (H1N1) virus (see, e.g., Staschke et al., Virology. 1998 Sep 1;248(2):264-74), influenza virus A/Korea/82 (H3N2) (see, e.g., Treanor et al., J Virol. 1994 Dec;68(12):7684-8), influenza virus A/Leningrad/134/57 (see, e.g., Egorov et al., J Virol. 1998 Aug;72(8):6437-41), influenza virus A/NWS/33 (H1N1) (see, e.g., Sidwell et al., Antiviral Res. 1998 Feb;37(2):107-20), influenza virus A/PR/8/34(H1N1) (see, e.g., Miyamoto et al., Antiviral Res. 1998 Aug;39(2):89-100), influenza virus A/PR8/34 (see, e.g., Nunes-Correia et al., Biochemistry. 1999 Jan 19;38(3):1095-101, Tree et al., Vaccine. 2001 May 14;19(25-26):3444-50), influenza virus A/Puerto Rico (PR)/8/34 (see, e.g., Egorov et al., J Virol. 1998 Aug;72(8):6437-41), influenza virus A/Puerto Rico/8-Mount Sinai (see, e.g., Mazanec et al., J Virol. 1995 Feb;69(2):1339-43), influenza virus A/Shangdong 9/93 (H3N2) (see, e.g., Zakay-Rones et al., J Altern Complement Med. 1995 Winter; 1(4):361-9, Sidwell et al., Antiviral Res. 1998 Feb;37(2):107-20), influenza virus A/Shingapol/1/57(H2N2) (see, e.g., Miyamoto et al., Antiviral Res. 1998 Aug;39(2):89-100), influenza virus A/Singapore 6/86 (H1N1) (see, e.g., Zakay-Rones et al., J Altern Complement Med. 1995 Winter;1(4):361-9), influenza virus A/Singapore/1/57 (H2N2) (see, e.g., Bantia et al., Antimicrob Agents Chemother. 1998 Apr;42(4):801-7), influenza virus A/Texas 36/91 (H1N1) (see, e.g., Zakay-Rones et al., J Altern Complement Med. 1995 Winter;1(4):361-9), influenza virus A/Texas/36/91 (H1N1) virus (see, e.g., Gubareva et al., J Infect Dis. 2001 Feb 15;183(4):523-31, Halperin et al., Vaccine. 1998 Aug;16(13):1331-5), influenza virus A/Texas/36/91(H1N1) (see, e.g., Hayden et al., Antiviral Res. 1994 Oct;25(2):123-31), influenza virus A/Udorn/72 virus infection (see, e.g., Shimizu et al., Virology. 1999 Feb 15;254(2):213-9), influenza virus A/Victoria/3/75 (H3N2) (see, e.g., Sidwell et al., Antiviral Res. 1998 Feb;37(2):107-20), influenza virus A/Virginia/88(H3N2) (see, e.g., Hayden et al., Antiviral Res. 1994 Oct;25(2):123-31), influenza virus A/WSN/33 (H1N1) (see, e.g., Lu et al., Arch Virol. 2002;147(2):273-84), influenza virus A/WSN/33 (see, e.g., Gujuluva et al., Virology. 1994 Nov 1;204(2):491-505), influenza virus B (see, e.g., Chare et al., J Gen Virol. 2003 Oct;84(Pt 10):2691-703), influenza virus B/Ann Arbor 1/86 (see, e.g., Zakay-Rones et al., J Altern Complement Med. 1995 Winter;1(4):361-9), influenza virus B/Harbin/7/94 (see, e.g., Halperin et al., Vaccine. 1998 Aug;16(13):1331-5, influenza virus B/Hong Kong/5/72 (see, e.g., Sidwell et al., Antiviral Res. 1998 Feb;37(2):107-20), influenza virus B/Lee/40 (see, e.g., Miyamoto et al., Antiviral Res. 1998 Aug;39(2):89-100), influenza virus B/Victoria group (see, e.g., Nakagawa et al., J Virol Methods. 1999 Apr;79(1):113-20), influenza virus B/Yamagata 16/88 (see, e.g., Zakay-Rones et al., J Altern Complement Med. 1995 Winter; 1(4):361-9), influenza virus B/Yamagata group (see, e.g., Nakagawa et al., J Virol Methods. 1999 Apr;79(1):113-20); influenza virus B/Yamanashi/166/98 (see, e.g., Hoffmann et al., Proc Natl Acad Sci U S A. 2002 Aug 20;99(17):11411-6), influenza virus C (see, e.g., Chare et al., J Gen Virol. 2003 Oct;84(Pt 10):2691-703), influenza virus strain A/Equi/2/Kildare/89 (see, e.g., Quinlivan et al., J Clin Microbiol. 2004 Feb;42(2):759-63), influenza virus type B/Panama 45/90 (see, e.g., Zakay-Rones et al., J Altern Complement Med. 1995 Winter;1(4):361-9), live, cold-adapted, temperature-sensitive (ca/ts) Russian influenza A vaccines (see, e.g., Palker et al., Virus Res. 2004 Oct; 105(2): 183-94), Madin Darby Canine Kidney (MDCK)-derived cell line (see, e.g., Halperin et al., Vaccine. 2002 Jan 15;20(7-8):1240-7), mouse-adapted influenza virus A/Guizhou/54/89 (H3N2 subtype) (see, e.g., Nagai et al., Biol Pharm Bull. 1995 Feb;18(2):295-9), mouse-adapted influenza virus A/PR/8/34 (A/PR8) (see, e.g., Nagai et al., Antiviral Res. 1995 Jan;26(1):11-25), mouse-adapted influenza virus B/Ibaraki/2/85 (see, e.g., Nagai et al., Biol Pharm Bull. 1995 Feb;18(2):295-9), Russian live attenuated influenza vaccine donor strains A/Leningrad/134/17/57, A/Leningrad/134/47/57 and B/USSR/60/69 (see, e.g., Audsley & Tannock, J Virol Methods. 2005 Feb;123(2):187-93), swine H1 and H3 influenza viruses (see, e.g., Gambaryan et al., Virus Res. 2005 Jul 1), swine influenza A viruses (see, e.g., Landolt et al., Am J Vet Res. 2005 Jan;66(1):119-24), swine influenza virus (SIV) (see, e.g., Clavijo et al., Can J Vet Res. 2002 Apr;66(2):117-21), swine influenza virus A/Sw/Ger 2/81 (see, e.g., Zakay-Rones et al., J Altern Complement Med. 1995 Winter;1(4):361-9), swine influenza virus A/Sw/Ger 8533/91 (see, e.g., Zakay-Rones et al., J Altern Complement Med. 1995 Winter;1(4):361-9), turkey influenza virus A/Tur/Ger 3/91 (see, e.g., Zakay-Rones et al., J Altern Complement Med. 1995 Winter;1(4):361-9) and turkey influenza virus A/turkey/Minnesota/833/80 (H4N2) (see, e.g., Gubareva et al., J Virol. 1997 May;71(5):3385-90).

As used herein, the term "antigen" or "immunogen" means a substance that induces a specific immune response in a host animal. The antigen may comprise a whole organism, killed, attenuated or live; a subunit or portion of an organism; a recombinant vector containing an insert with immunogenic properties; a piece or fragment of DNA capable of inducing an immune response upon presentation to a host animal; a protein, a polypeptide, a peptide, an epitope, a hapten, or any combination thereof. Alternately, the immunogen or antigen may comprise a toxin or antitoxin.

The term "immunogenic protein or peptide" as used herein also refers includes peptides and polypeptides that are immunologically active in the sense that once administered to the host, it is able to evoke an immune response of the humoral and/or cellular type directed against the protein. Preferably the protein fragment is such that it has substantially the same immunological activity as the total protein. Thus, a protein fragment according to the description comprises or consists essentially of or consists of at least one epitope or antigenic determinant. The term epitope relates to a protein site able to induce an immune reaction of the humoral type (B cells) and/or cellular type (T cells).

The term "immunogenic protein or peptide" further contemplates deletions, additions and substitutions to the sequence, so long as the polypeptide functions to produce an immunological response as defined herein. In this regard, particularly preferred substitutions will generally be conservative in nature, i.e., those substitutions that take place within a family of amino acids. For example, amino acids are generally divided into four families: (1) acidic-aspartate and glutamate; (2) basic-lysine, arginine, histidine; (3) non-polar-alanine, valine, leucine, isoleucine, proline, phenylalanine, methionine, tryptophan; and (4) uncharged polar-glycine, asparagine, glutamine, cystine, serine threonine, tyrosine. Phenylalanine, tryptophan, and tyrosine are sometimes classified as aromatic amino acids. It is reasonably predictable that an isolated replacement of leucine with isoleucine or valine, or vice versa; an aspartate with a glutamate or vice versa; a threonine with a serine or vice versa; or a similar conservative replacement of an amino acid with a structurally related amino acid, will not have a major effect on the biological activity. Proteins having substantially the same amino acid sequence as the reference molecule but possessing minor amino acid substitutions that do not substantially affect the immunogenicity of the protein are, therefore, within the definition of the reference polypeptide.

The term "epitope" refers to the site on an antigen or hapten to which specific B cells and/or T cells respond. The term is also used interchangeably with "antigenic determinant" or "antigenic determinant site". Antibodies that recognize the same epitope can be identified in a simple immunoassay showing the ability of one antibody to block the binding of another antibody to a target antigen.

An "immunological response" to a composition or vaccine is the development in the host of a cellular and/or antibody-mediated immune response to a composition or vaccine of interest. Usually, an "immunological response" includes but is not limited to one or more of the following effects: the production of antibodies, B cells, helper T cells, , and/or cytotoxic T cells, directed specifically to an antigen or antigens included in the composition or vaccine of interest. Preferably, the host will display either a therapeutic or protective immunological response such that resistance to new infection will be enhanced and/or the clinical severity of the disease reduced. Such protection will be demonstrated by either a reduction or lack of symptoms normally displayed by an infected host, a quicker recovery time and/or a lowered viral titer in the infected host.

The terms "immunogenic" protein or polypeptide as used herein also refers to an amino acid sequence which elicits an immunological response as described above. An "immunogenic" protein or polypeptide, as used herein, includes the full-length sequence of the protein, analogs thereof, or immunogenic fragments thereof. By "immunogenic fragment" is meant a fragment of a protein which includes one or more epitopes and thus elicits the immunological response described above. Such fragments can be identified using any number of epitope mapping techniques, well known in the art. See, *e.g*., Epitope Mapping Protocols in Methods in Molecular Biology, Vol. 66 (Glenn E. Morris, Ed., 1996) Humana Press, Totowa, N.J. For example, linear epitopes may be determined by *e.g*., concurrently synthesizing large numbers of peptides on solid supports, the peptides corresponding to portions of the protein molecule, and reacting the peptides with antibodies while the peptides are still attached to the supports. Such techniques are known in the art and described in, *e.g*., U.S. Pat No. 4,708,871; Geysen et al. (1984) Proc. Natl. Acad. Sci. USA 81:3998-4002; Geysen et al. (1986) Molec. Immunol. 23:709-715. Similarly, conformational epitopes are readily identified by determining spatial conformation of amino acids such as by, *e.g*., x-ray crystallography and 2-dimensional nuclear magnetic resonance. See, *e.g*., Epitope Mapping Protocols, *supra.* Methods especially applicable to the proteins of *T*. *parva* are fully described in the PCT Application Serial No. PCT/US2004/022605.

Synthetic antigens are also included within the definition, for example, polyepitopes, flanking epitopes, and other recombinant or synthetically derived antigens. See, *e.g.,* Bergmann et al. (1993) Eur. J. Immunol. 23:2777-2781; Bergmann et al. (1996) J. Immunol. 157:3242-3249; Suhrbier, A. (1997) Immunol. and Cell Biol. 75:402-408; Gardner et al. (1998) 12th World AIDS Conference, Geneva, Switzerland, Jun. 28-Jul. 3, 1998. Immunogenic fragments, for purposes of the present description will usually include at least about 3 amino acids, preferably at least about 5 amino acids, more preferably at least about 10-15 amino acids, and most preferably 25 or more amino acids, of the molecule. There is no critical upper limit to the length of the fragment, which could comprise nearly the full-length of the protein sequence, or even a fusion protein comprising at least one epitope of the protein.

Accordingly, a minimum structure of a polynucleotide expressing an epitope is that it comprises or consists essentially of or consists of nucleotides to encode an epitope or antigenic determinant of an influenza protein or polyprotein. A polynucleotide encoding a fragment of the total protein or polyprotein, more advantageously, comprises or consists essentially of or consists of a minimum of 21 nucleotides, advantageously at least 42 nucleotides, and preferably at least 57, 87 or 150 consecutive or contiguous nucleotides of the sequence encoding the total protein or polyprotein. Epitope determination procedures, such as, generating overlapping peptide libraries (Hemmer B. et al., Immunology Today, 1998, 19 (4), 163-168), Pepscan (Geysen et al., (1984) Proc. Nat. Acad. Sci. USA, 81, 3998-4002; Geysen et al., (1985) Proc. Nat. Acad. Sci. USA, 82, 178-182; Van der Zee R. et al., (1989) Eur. J. Immunol, 19, 43-47; Geysen H.M., (1990) Southeast Asian J. Trop. Med. Public Health, 21, 523-533; Multipin.RTM. Peptide Synthesis Kits de Chiron) and algorithms (De Groot A. et al., (1999) Nature Biotechnology, 17, 533-561), and in PCT Application Serial No. PCT/US2004/022605, can be used in the practice of the invention, without undue experimentation. Other documents cited may also be consulted for methods for determining epitopes of an immunogen or antigen and thus nucleic acid molecules that encode such epitopes.

A "polynucleotide" is a polymeric form of nucleotides of any length, which contain deoxyribonucleotides, ribonucleotides, and analogs in any combination. Polynucleotides may have three-dimensional structure, and may perform any function, known or unknown. The term "polynucleotide" includes double-, single-stranded, and triple-helical molecules. Unless otherwise specified or required, any embodiment of the description described herein that is a polynucleotide encompasses both the double stranded form and each of two complementary forms known or predicted to make up the double stranded form of either the DNA, RNA or hybrid molecule.

The following are non-limiting examples of polynucleotides: a gene or gene fragment, exons, introns, mRNA, tRNA, rRNA, ribozymes, cDNA, recombinant polynucleotides, branched polynucleotides, plasmids, vectors, isolated DNA of any sequence, isolated RNA of any sequence, nucleic acid probes and primers. A polynucleotide may comprise modified nucleotides, such as methylated nucleotides and nucleotide analogs, uracyl, other sugars and linking groups such as fluororibose and thiolate, and nucleotide branches. The sequence of nucleotides may be further modified after polymerization, such as by conjugation, with a labeling component. Other types of modifications included in this definition are caps, substitution of one or more of the naturally occurring nucleotides with an analog, and introduction of means for attaching the polynucleotide to proteins, metal ions, labeling components, other polynucleotides or solid support. The polynucleotides can be obtained by chemical synthesis or derived from a microorganism.

The description further comprises a complementary strand to a polynucleotide encoding an influenza antigen, epitope or immunogen. The complementary strand can be polymeric and of any length, and can contain deoxyribonucleotides, ribonucleotides, and analogs in any combination.

The terms "protein", "peptide", "polypeptide" and "polypeptide fragment" are used interchangeably herein to refer to polymers of amino acid residues of any length. The polymer can be linear or branched, it may comprise modified amino acids or amino acid analogs, and it may be interrupted by chemical moieties other than amino acids. The terms also encompass an amino acid polymer that has been modified naturally or by intervention; for example disulfide bond formation, glycosylation, lipidation, acetylation, phosphorylation, or any other manipulation or modification, such as conjugation with a labeling or bioactive component.

An "isolated" polynucleotide or polypeptide is one that is substantially free of the materials with which it is associated in its native environment. By substantially free, is meant at least 50%, advantageously at least 70%, more advantageously at least 80%, and even more advantageously at least 90% free of these materials.

Hybridization reactions can be performed under conditions of different "stringency." Conditions that increase stringency of a hybridization reaction are well known. See for example, "Molecular Cloning: A Laboratory Manual", second edition (Sambrook et al. 1989). Examples of relevant conditions include (in order of increasing stringency): incubation temperatures of 25°C, 37°C, 50°C, and 68°C; buffer concentrations of 10 x SSC, 6 x SSC, 1 x SSC, 0.1 x SSC (where SSC is 0.15 M NaCl and 15 mM citrate buffer) and their equivalent using other buffer systems; formamide concentrations of 0%, 25%, 50%, and 75%; incubation times from 5 minutes to 24 hours; 1, 2 or more washing steps; wash incubation times of 1, 2, or 15 minutes; and wash solutions of 6 x SSC, 1 x SSC, 0.1 x SSC, or deionized water.

The description further encompasses polynucleotides encoding functionally equivalent variants and derivatives of the influenza polypeptides and functionally equivalent fragments thereof which may enhance, decrease or not significantly affect properties of the polypeptides encoded thereby. These functionally equivalent variants, derivatives, and fragments display the ability to retain influenza activity. For instance, changes in a DNA sequence that do not change the encoded amino acid sequence, as well as those that result in conservative substitutions of amino acid residues, one or a few amino acid deletions or additions, and substitution of amino acid residues by amino acid analogs are those which will not significantly affect properties of the encoded polypeptide. Conservative amino acid substitutions are glycine/alanine; valine/isoleucine/leucine; asparagine/glutamine; aspartic acid/glutamic acid; serane/threonine/methionine; lysine/arginine; and phenylalanine/tyrosine/tryptophan. In one embodiment, the variants have at least 50%, at least 55%, at least 60%, at least 65%, at least 70%, at least 75%, at least 80%, at least 85%, at least 86%, at least 87%, at least 88%, at least 89%, at least 90%, at least 91%, at least 92%, at least 93%, at least 94%, at least 95%, at least 96%, at least 97%, at least 98% or at least 99% homology or identity to the influenza polynucleotide or polypeptide of interest.

For the purposes of the present description, sequence identity or homology is determined by comparing the sequences when aligned so as to maximize overlap and identity while minimizing sequence gaps. In particular, sequence identity may be determined using any of a number of mathematical algorithms. A nonlimiting example of a mathematical algorithm used for comparison of two sequences is the algorithm of Karlin & Altschul, Proc. Natl. Acad. Sci. USA 1990; 87: 2264-2268, modified as in Karlin & Altschul, Proc. Natl. Acad. Sci. USA 1993;90: 5873-5877.

Another example of a mathematical algorithm used for comparison of sequences is the algorithm of Myers & Miller, CABIOS 1988;4: 11-17. Such an algorithm is incorporated into the ALIGN program (version 2.0) which is part of the GCG sequence alignment software package. When utilizing the ALIGN program for comparing amino acid sequences, a PAM120 weight residue table, a gap length penalty of 12, and a gap penalty of 4 can be used. Yet another useful algoritlun for identifying regions of local sequence similarity and alignment is the FASTA algorithm as described in Pearson & Lipman, Proc. Natl. Acad. Sci. USA 1988; 85: 2444-2448.

Advantageous for use according to the present description is the WU-BLAST (Washington University BLAST) version 2.0 software. WU-BLAST version 2.0 executable programs for several UNIX platforms can be downloaded from ftp ://blast.wustl.edu/blast/executables. This program is based on WU-BLAST version 1.4, which in turn is based on the public domain NCBI-BLAST version 1.4 (Altschul & Gish, 1996, Local alignment statistics, Doolittle ed., Methods in Enzymology 266: 460-480; Altschul et al., Journal of Molecular Biology 1990; 215: 403-410; Gish & States, 1993;Nature Genetics 3: 266-272; Karlin & Altschul, 1993;Proc. Natl. Acad. Sci. USA 90: 5873-5877,

In general, comparison of amino acid sequences is accomplished by aligning an amino acid sequence of a polypeptide of a known structure with the amino acid sequence of a the polypeptide of unknown structure. Amino acids in the sequences are then compared and groups of amino acids that are homologous are grouped together. This method detects conserved regions of the polypeptides and accounts for amino acid insertions and deletions. Homology between amino acid sequences can be determined by using commercially available algorithms (see also the description of homology above). In addition to those otherwise mentioned herein, mention is made too of the programs BLAST, gapped BLAST, BLASTN, BLASTP, and PSI-BLAST, provided by the National Center for Biotechnology Information. These programs are widely used in the art for this purpose and can align homologous regions of two amino acid sequences.

In all search programs in the suite the gapped alignment routines are integral to the database search itself. Gapping can be turned off if desired. The default penalty (Q) for a gap of length one is Q=9 for proteins and BLASTP, and Q=10 for BLASTN, but may be changed to any integer. The default per-residue penalty for extending a gap (R) is R=2 for proteins and BLASTP, and R=10 for BLASTN, but may be changed to any integer. Any combination of values for Q and R can be used in order to align sequences so as to maximize overlap and identity while minimizing sequence gaps. The default amino acid comparison matrix is BLOSUM62, but other amino acid comparison matrices such as PAM can be utilized.

Alternatively or additionally, the term "homology" or "identity", for instance, with respect to a nucleotide or amino acid sequence, can indicate a quantitative measure of homology between two sequences. The percent sequence homology can be calculated as

(N*_{ref}* - *N_{dif}*)*100/N_{*r*e*f*}, wherein N*_{dif}* is the total number of non-identical residues in the two sequences when aligned and wherein *N_{ref}* is the number of residues in one of the sequences. Hence, the DNA sequence AGTCAGTC will have a sequence identity of 75% with the sequence AATCAATC (*N_{ref}* = 8; *N_{dif}*=2).

Alternatively or additionally, "homology" or "identity" with respect to sequences can refer to the number of positions with identical nucleotides or amino acids divided by the number of nucleotides or amino acids in the shorter of the two sequences wherein alignment of the two sequences can be detennined in accordance with the Wilbur and Lipman algorithm (Wilbur & Lipman, Proc Natl Acad Sci USA 1983; 80:726), for instance, using a window size of 20 nucleotides, a word length of 4 nucleotides, and a gap penalty of 4, and computer-assisted analysis and interpretation of the sequence data including alignment can be conveniently performed using commercially available programs (e.g., Intelligenetics ™ Suite, Intelligenetics Inc. CA). When RNA sequences are said to be similar, or have a degree of sequence identity or homology with DNA sequences, thymidine (T) in the DNA sequence is considered equal to uracil (U) in the RNA sequence. Thus, RNA sequences are within the scope of the description and can be derived from DNA sequences, by thymidine (T) in the DNA sequence being considered equal to uracil (U) in RNA sequences.

And, without undue experimentation, the skilled artisan can consult with many other programs or references for determining percent homology.

The description further encompasses the influenza polynucleotides contained in a vector molecule or an expression vector and operably linked to a promoter element and optionally to an enhancer.

The vector is advantageously a poxvirus, particularly a vaccinia virus or an avipox virus, such as fowlpox virus and canarypox virus. Advantageously, the virus is a canarypox virus. Advantageous canarypox strains may be an attenuated strain. The vector can express at least one epitope from Kentucky strains, Newmarket strains and/or Ohio strains. Advantageous canarypox constructs include, but are not limited to, vCP 1529, vCP 1533 and vCP 2242. Recombinant avipox viruses (see, e.g., U.S. Patent Nos. 5,505,941 and 5,756,103), such as an attenuated recombinant canarypox virus, for instance ALVAC, or an attenuated fowlpox virus, for instance TROVAC, are especially advantageous. In one advantageous embodiment, the recombinant ALVAC vaccine described by Edlund Toulemonde et al., Vet Rec. 2005 Mar 19;156(12):367-71 may be used as a canine influenza vaccine. Other viruses that may be used in methods of the description include, but are not limited to, vaccinia viruses, such as an attenuated vaccinia virus, for instance NYVAC, adenoviruses, such as canine adenoviruses (CAV), and herpesviruses, such as canine herpesvirus (CHV) or a feline herpesvirus (FHV).

A "vector" refers to a recombinant DNA or RNA plasmid or virus that comprises a heterologous polynucleotide to be delivered to a target cell, either in vitro or in vivo. The heterologous polynucleotide may comprise a sequence of interest for purposes of therapy, and may optionally be in the form of an expression cassette. As used herein, a vector needs not be capable of replication in the ultimate target cell or subject. The term includes cloning vectors also included are viral vectors.

The term "recombinant" means a polynucleotide semisynthetic, or synthetic origin which either does not occur in nature or is linked to another polynucleotide in an arrangement not found in nature.

"Heterologous" means derived from a genetically distinct entity from the rest of the entity to which it is being compared. For example, a polynucleotide, may be placed by genetic engineering techniques into a plasmid or vector derived from a different source, and is a heterologous polynucleotide. A promoter removed from its native coding sequence and operatively linked to a coding sequence other than the native sequence is a heterologous promoter.

The polynucleotides of the description may comprise additional sequences, such as additional encoding sequences within the same transcription unit, controlling elements such as promoters, ribosome binding sites, polyadenylation sites, additional transcription units under control of the same or a different promoter, sequences that permit cloning, expression, homologous recombination, and transformation of a host cell, and any such construct as may be desirable to provide embodiments of this description.

Elements for the expression of an influenza antigen, epitope or immunogen are advantageously present in a vector. In minimum manner, this comprises, consists essentially of, or consists of an initiation codon (ATG), a stop codon and a promoter, and optionally also a polyadenylation sequence for certain vectors such as plasmid and certain viral vectors, e.g., viral vectors other than poxviruses. When the polynucleotide encodes a polyprotein fragment, e.g. an influenza peptide, advantageously, in the vector, an ATG is placed at 5' of the reading frame and a stop codon is placed at 3'. Other elements for controlling expression may be present, such as enhancer sequences, stabilizing sequences, such as intron and signal sequences permitting the secretion of the protein.

Methods for making and/or administering a vector or recombinants or plasmid for expression of gene products of genes of the description either *in vivo* or *in vitro* can be any desired method, e.g., a method which is by or analogous to the methods disclosed in, or disclosed in documents cited in: U.S. Patent Nos. 4,603,112; 4,769,330; 4,394,448; 4,722,848; 4,745,051; 4,769,331; 4,945,050; 5,494,807; 5,514,375; 5,744,140; 5,744,141; 5,756,103; 5,762,938; 5,766,599; 5,990,091; 5,174,993; 5,505,941; 5,338,683; 5,494,807; 5,591,639; 5,589,466; 5,677,178; 5,591,439; 5,552,143; 5,580,859; 6,130,066; 6,004,777; 6,130,066; 6,497,883; 6,464,984; 6,451,770; 6,391,314; 6,387,376; 6,376,473; 6,368,603; 6,348,196; 6,306,400; 6,228,846; 6,221,362; 6,217,883; 6,207,166; 6,207,165; 6,159,477; 6,153,199; 6,090,393; 6,074,649; 6,045,803; 6,033,670; 6,485,729; 6,103,526; 6,224,882; 6,312,682; 6,348,450 and 6; 312,683; U.S. patent application Serial No. 920,197, filed October 16,1986; WO 90/01543; W091/11525; WO 94/16716; WO 96/39491; WO 98/33510; EP 265785; EP 0 370 573; Andreansky et al., Proc. Natl. Acad. Sci. USA 1996;93:11313-I 13I 8; Ballay et al., EMBO J. 1993;4:3861-65; Felgaer et al., J. Biol. Chem. 1994;269:2550-2561; Frolov et al., Proc. Natl. Acad Sci. USA 1996;93:11371-11377; Graham, Tibtech 1990;8:85-87; Granhaus et al., Sem. Virol. 1992;3:237-52; Ju et al., Diabetologia 1998;41:736-739; Kitson et al, J. Virol. 1991;65:3068-3075; McClements et al., Proc. Natl. Acad. Sci. USA 1996;93:11414-11420; Moss, Proc. Natl. Acad. Sci. USA 1996;93:1134I-11348; Paoletti, Proc. Natl. Acad. Sci. USA 1996;93:11349-11353; Pennock et al, Mol. Cell. Biol. 1984;4:399-406; Richardson (Ed), Methods in Molecular Biology 1995;39, "Baculovirus Expression Protocols," Humana Press Inc.; Smith et al. (1983) Mol. Cell. Biol. 1983;3:2156-2165; Robertson et al., Proc. Natl. Acad. Sci. USA 1996;93:11334-11340; Robinson et al., Sem. Immunol. 1997;9:271; and Roizman, Proc. Natl. Acad. Sci. USA 1996;93:11307-11312. Thus, the vector in the description can be any suitable recombinant virus or virus vector, such as a poxvirus (e.g., vaccinia virus, avipox virus, canarypox virus, fowlpox virus, raccoonpox virus, swinepox virus, etc.), adenovirus (e.g., human adenovirus, canine adenovirus), herpesvirus (e.g. canine herpesvirus), baculovirus, retrovirus, etc. or the vector can be a plasmid. The herein cited documents, in addition to providing examples of vectors useful in the practice of the invention, can also provide sources for non-influenza peptides or fragments thereof to be expressed by vector or vectors in, or included in, the compositions of the description.

The present description also relates to preparations comprising vectors, such as expression vectors, e.g., therapeutic compositions. The preparations can comprise, consist essentially of, or consist of one or more vectors, e.g., expression vectors, such as *in vivo* expression vectors, comprising, consisting essentially or consisting of (and advantageously expressing) one or more of influenza antigens, epitopes or immunogens. Advantageously, the vector contains and expresses a polynucleotide that includes, consists essentially of, or consists of a polynucleofiide coding for (and advantageously expressing) an influenza antigen, epitope or immunogen, in a pharmaceutically or veterinarily acceptable carrier, excipient or vehicle. Thus, according to an embodiment of the description the other vector or vectors in the preparation comprises, consists essentially of or consists of a polynucleotide that encodes, and under appropriate circumstances the vector expresses one or more other proteins of an influenza antigen, epitope or immunogen (e.g., hemagglutinin, neuraminidase, nucleoprotein) or a fragment thereof.

According to another embodiment, the vector or vectors in the preparation comprise, or consist essentially of, or consist of polynucleotide(s) encoding one or more proteins or fragment(s) thereof of an influenza antigen, epitope or immunogen, the vector or vectors expressing the polynucleotide(s). The preparation advantageously comprises, consists essentially of, or consists of, at least two vectors comprising, consisting essentially of, or consisting of, and advantageously also expressing, advantageously *in vivo* under appropriate conditions or suitable conditions or in a suitable host cell, polynucleotides from different canine influenza isolates encoding the same proteins and/or for different proteins, but advantageously the same proteins. Preparations containing one or more vectors containing, consisting essentially of or consisting of polynucleotides encoding, and advantageously expressing, advantageously *in vivo,* an influenza antigen, fusion protein or an epitope thereof. The description is also directed at mixtures of vectors that contain, consist essentially of, or consist of coding for, and express, different influenza antigens, epitopes or immunogens, e.g., an influenza antigen, epitope or immunogen from different species such as, but not limited to, humans, , pigs, , in addition to avian species including chicken, ducks and geese.

According to one embodiment of the description the expression vector is a viral vector, in particular an *in vivo* expression vector. In an advantageous embodiment, the expression vector is an adenovirus vector. Advantageously, the adenovirus is a human Ad5 vector, an E1-deleted and/ or an E3-deleted adenovirus.

In one particular embodiment the viral vector is a poxvirus, e.g. a vaccinia virus or an attenuated vaccinia virus, (for instance, MVA, a modified Ankara strain obtained after more than 570 passages of the Ankara vaccine strain on chicken embryo fibroblasts; see Stickl & Hochstein-Mintzel, Munch. Med. Wschr., 1971,113,1149-1153; Sutter et al., Proc. Natl. Acad. Sci. U.S.A., 1992, 89, 10847-10851; available as ATCC VR-1508; or NYVAC, see U.S. Patent No. 5,494,807, for instance, Examples 1 to 6 and *et seq* of U.S. Patent No. 5,494,807 which discuss the construction of NYVAC, as well as variations of NYVAC with additional ORFs deleted from the Copenhagen strain vaccinia virus genome, as well as the insertion of heterologous coding nucleic acid molecules into sites of this recombinant and also, the use of matched promoters; see also WO96/40241), an avipox virus or an attenuated avipox virus (e.g., canarypox, fowlpox, dovepox, pigeonpox, quailpox, ALVAC or TROVAC; see, e.g., U.S. Patent No. 5,505,941, 5,494,807), swinepox, raccoonpox, camelpox, or myxomatosis virus.

According to another embodiment of the description the poxvirus vector is a canarypox virus or a fowlpox virus vector, advantageously an attenuated canarypox virus or fowlpox virus. In this regard, is made to the canarypox available from the ATCC under access number VR-111. Attenuated canarypox viruses are described in U.S. Patent No. 5,756,103 (ALVAC) and WO01/05934. Numerous fowlpox virus vaccination strains are also available, e.g. the DIFTOSEC CT strain marketed by MERIAL and the NOBILIS VARIOLE vaccine marketed by INTERVET; and, reference is also made to U.S. Patent No. 5,766,599 which pertains to the attenuated fowlpox strain TROVAC.

For information on the method to generate recombinants thereof and how to administer recombinants thereof, the skilled artisan can refer documents cited herein and to WO90/12882, e.g., as to vaccinia virus mention is made of U.S. Patents Nos. 4,769,330, 4,722,848, 4,603,112, 5,110,587, 5,494,807, and 5,762,938 *inter alia*; as to fowlpox, mention is made of U.S. Patents Nos. 5,174,993, 5,505,941 and US-5,766,599 *inter alia*; as to canarypox mentionis made of U.S. Patent No. 5,756,103 *inter alia*; as to swinepox mention is made of U.S. Patent No. 5,382,425 *inter alia*; and, as to raccoonpox, mention is made of WO00/03030 *inter alia*.

When the expression vector is a vaccinia virus, insertion site or sites for the polynucleotide or polynucleotides to be expressed are advantageously at the thymidine kinase (TK) gene or insertion site, the hemagglutinin (HA) gene or insertion site, the region encoding the inclusion body of the A type (ATI); see also documents cited herein, especially those pertaining to vaccinia virus. In the case of canarypox, advantageously the insertion site or sites are ORF(s) C3, C5 and/or C6; see also documents cited herein, especially those pertaining to canarypox virus. In the case of fowlpox, advantageously the insertion site or sites are ORFs F7 and/or F8; see also documents cited herein, especially those pertaining to fowlpox virus. The insertion site or sites for MVA virus area advantageously as in various publications, including Carroll M. W. et al., Vaccine, 1997, 15 (4), 387-394; Stittelaar K. J. et al., J. Virol., 2000, 74 (9), 4236-4243; Sutter G. et al., 1994, Vaccine, 12 (11), 1032-1040; and, in this regard it is also noted that the complete MVA genome is described in Antoine G., Virology, 1998, 244, 365-396, which enables the skilled artisan to use other insertion sites or other promoters.

Advantageously, the polynucleotide to be expressed is inserted under the control of a specific poxvirus promoter, e.g., the vaccinia promoter 7.5 kDa (Cochran et al., J. Virology, 1985, 54, 30-35), the vaccinia promoter I3L (Riviere et al., J. Virology, 1992, 66, 3424-3434), the vaccinia promoter HA (Shida, Virology, 1986, 150, 451-457), the cowpox promoter ATI (Funahashi et al., J. Gen. Virol., 1988, 69, 35-47), the vaccinia promoter H6 (Taylor J. et al., Vaccine, 1988, 6, 504-508; Guo P. et al. J. Virol., 1989, 63, 4189-4198; Perkus M. et al., J. Virol., 1989, 63, 3829-3836), *inter alia*.

In a particular embodiment the viral vector is an adenovirus, such as a human adenovirus (HAV) or a canine adenovirus (CAV).

In one embodiment the viral vector is a human adenovirus, in particular a serotype 5. adenovirus, rendered incompetent for replication by a deletion in the E1 region of the viral genome, in particular from about nucleotide 459 to about nucleotide 3510 by reference to the sequence of the hAd5 disclosed in Genbank under the accession number M73260 and in the referenced publication J. Chroboczek et al Virol. 1992, 186, 280-285. The deleted adenovirus is propagated in E1-expressing 293 (F. Graham et al J. Gen. Virol. 1977, 36, 59-72) or PER cells, in particular PER.C6 (F. Falloux et al Human Gene Therapy 1998, 9, 1909-1917). The human adenovirus can be deleted in the E3 region, in particular from about nucleotide 28592 to about nucleotide 30470. The deletion in the E1 region can be done in combination with a deletion in the E3 region (*see*, *e.g*. J. Shriver et al. Nature, 2002,415, 331-335, F. Graham et al Methods in Molecular Biology Vol .7: Gene Transfer and Expression Protocols Edited by E. Murray, The Human Press Inc, 1991, p 109-128; Y. Ilan et al Proc. Natl. Acad. Sci. 1997, 94, 2587-2592; US6,133,028; US6,692,956; S. Tripathy et al Proc. Natl. Acad. Sci. 1994, 91, 11557-11561; B. Tapnell Adv. Drug Deliv. Rev.1993, 12, 185-199;X. Danthinne et al Gene Thrapy 2000, 7, 1707-1714; K. Berkner Bio Techniques 1988, 6, 616-629; K. Berkner et al Nucl. Acid Res. 1983, 11, 6003-6020; C. Chavier et al J. Virol. 1996, 70, 4805-4810). The insertion sites can be the E1. and/or E3 loci (region) eventually after a partial or complete deletion of the E1 and/or E3 regions. Advantageously, when the expression vector is an adenovirus, the polynucleotide to be expressed is inserted under the control of a promoter functional in eukaryotic cells, such as a strong promoter, preferably a cytomegalovirus immediate-early gene promoter (CMV-IE promoter), in particular the enhancer / promoter region from about-nucleotide -734 to about nucleotide +7 in M. Boshart et al Cell 1985, 4.1, 521-530 or the enhancer / promoter region from the pCI vector from Promega Corp. The CMV-IE promoter is advantageously of murine or human origin. The promoter of the elongation factor 1α can also be used. A muscle specific promoter can also be used (X. Li et al Nat. Biotechnol. 1999, 17, 241-245). Strong promoters are also discussed herein in relation to plasmid vectors. In one embodiment, a splicing sequence can be located downstream of the enhancer / promoter region. For example, the intron 1 isolated from the CMV-IE gene (R. Stenberg et al J. Virol. 1984, 49, 190), the intron isolated from the rabbit or human β-globin gene, in particular the intron 2 from the b-globin gene, the intron isolated from the immunoglobulin gene, a splicing sequence from the SV40 early gene or the chimeric intron sequence isolated from the pCI vector from Promege Corp. comprising the human β-globin gene donor sequence fused to the mouse immunoglobulin acceptor sequence (from about nucleotide 890 to about nucleotide 1022 in Genbank under the accession number CVU47120). A poly(A) sequence and terminator sequence can be inserted downstream the polynucleotide to be expressed, e.g. a bovine growth hormone gene, in particular from about nucleotide 2339 to about nucleotide 2550 in Genbank under the accession number BOVGHRH, a rabbit β-globin gene or a SV40 late gene polyadenylation signal.

In another embodiment the viral vector is a canine adenovirus, in particular a CAV-2 (see, e.g. L. Fischer et al. Vaccine, 2002, 20, 3485-3497; U.S. Patent No. 5,529,780; U.S. Patent No. 5,688,920; PCT Application No. WO95/14102). For CAV, the insertion sites can be in the E3 region and /or in the region located between the E4 region and the right ITR region (see U.S. Patent No. 6,090,393; U.S. Patent No. 6,156,567). In one embodiment the insert is under the control of a promoter, such as a cytomegalovirus immediate-early gene promoter (CMV-IB promoter) or a promoter already described for a human adenovirus vector. A poly(A) sequence and terminator sequence can be inserted downstream the polynucleotide to be expressed, e.g. a bovine growth hormone gene or a rabbit β-globin gene polyadenylation signal.

In another particular embodiment the viral vector is a herpesvirus such as a canine herpesvirus (CHV) or a feline herpesvirus (FHV). For CHV, the insertion sites may be in particular in the thymidine kinase gene, in the ORF3, or in the UL43 ORF (see U.S. Patent No. 6,159,477). In one embodiment the polynucleotide to be expressed is inserted under the control of a promoter functional in eukaryotic cells, advantageously a CMV-IB promoter (murine or human).. A poly(A) sequence and terminator sequence can be inserted downstream the polynucleotide to be expressed, e.g. bovine growth hormone or a rabbit β-globin gene polyadenylation signal.

According to a yet further embodiment of the description the expression vector is a plasmid vector or a DNA plasmid vector, in particular an *in vivo* expression vector. In a specific, non-limiting example, the pVR1020 or 1012 plasmid (VICAL Inc.; Luke C. et al., Journal of Infectious Diseases, 1997, 175, 91-97; Hartikka J. et al., Human Gene Therapy, 1996, 7, 1205-1217, see, e.g., U.S. Patent Nos. 5,846,946 and 6,451,769) can be utilized as a vector for the insertion of a polynucleotide sequence. The pVR1020 plasmid is derived from pYR1012 and contains the human tPA signal sequence. In one embodiment the human tPA signal comprises from amino acid M(1) to amino acid S(23) in Genbank under the accession number HUMTPA14. In another specific, non-limiting example, the plasmid utilized as a vector for the insertion of a polynucleotide sequence can contain the signal peptide sequence of equine IGF1 from amino acid M(24) to amino acid A(48) in Genbank under the accession number U28070. Additional information on DNA plasmids which may be consulted or employed in the practice are found, for example, in U.S. Patent Nos. 6,852,705; 6,818,628; 6,586,412; 6,576,243; 6,558,674; 6,464,984; 6,451,770; 6,376,473 and 6,221,362.

The term plasmid covers any DNA transcription unit comprising a polynucleotide according to the description and the elements necessary for its *in vivo* expression in a cell or cells of the desired host or target; and, in this regard, it is noted that a supercoiled or non-supercoiled, circular plasmid, as well as a linear form, are intended to be within the scope of the description.

Each plasmid comprises or contains or consists essentially of, in addition to the polynucleotide encoding an influenza antigen, epitope or immunogen, optionally fused with a heterologous peptide sequence, variant, analog or fragment, operably linked to a promoter or under the control of a promoter or dependent upon a promoter. In general, it is advantageous to employ a strong promoter functional in eukaryotic cells. The preferred strong promoter is the immediate early cytomegalovirus promoter (CMV-IE) of human or murine origin, or optionally having another origin such as the rat or guinea pig. The CMV-IE promoter can comprise the actual promoter part, which may or may not be associated with the enhancer part. Reference can be made to EP-A-260 148, EP-A-323 597, U.S. Patents Nos. 5,168,062, 5,385,839, and 4,968,615, as well as to PCT Application No WO87/03905. The CMV-IE promoter is advantageously a human CMV-IE (Bosbart M. et al., Cell., 1985, 41, 521-530) or murine CMV-IE.

In more general terms, the promoter has either a viral or a cellular origin. A strong viral promoter other than CMV-IE that may be usefully employed in the practice of the reception is the early/late promoter of the SV40 virus or the LTR promoter of the Rous sarcoma virus. A strong cellular promoter that may be usefully employed in the practice of the description is the promoter of a gene of the cytoskeleton, such as e. g. the desmin promoter (Kwissa M. et al., Vaccine, 2000, 18, 2337-2344), or the actin promoters (Miyazaki J. et al., Gene, 1989, 79, 269-277).

Functional sub fragments of these promoters, i.e., portions of these promoters that maintain an adequate promoting activity, are included within the present description, e.g. truncated CMV-IE promoters according to PCT Application No. WO98/00166 or U. S. Patent No. 6,156,567 can be used in the practice of the description. A promoter in the practice of the description consequently includes derivatives and sub fragments of a full-length promoter that maintain an adequate promoting activity and hence function as a promoter, preferably promoting activity substantially similar to that of the actual or full-length promoter from which the derivative or sub fragment is derived, e.g., akin to the activity of the truncated CMV-IE promoters of U.S. Patent No. 6,156,567 to the activity of full-length CMV-IE promoters. Thus, a CMV-IE promoter in the practice of the description can comprise or consist essentially of or consist of the promoter portion of the full-length promoter and/or the enhancer portion of the full-length promoter, as well as derivatives and sub fragments.

Preferably, the plasmids comprise or consist essentially of other expression control elements. It is particularly advantageous to incorporate stabilizing sequence(s), e.g., intron sequence(s), preferably the first intron of the hCMV-IE (PCT Application No. WO89/01036) the intron II of the rabbit β-globin gene (van Ooyen et al., Science, 1979, 206, 337-344).

As to the polyadenylation signal (polyA) for the plasmids and viral vectors other than poxviruses, use can more be made of the poly(A) signal of the bovine growth hormone (bGH) gene (*see* U.S. Patent No. 5,122,458), or the poly(A) signal of the rabbit β-globin gene or the poly(A) signal of the SV40 virus.

According to another embodiment of the description the expression vectors are expression vectors used for the *in vitro* expression of proteins in an appropriate cell system. The expressed proteins can be harvested in or from the culture supernatant after, or not after secretion (if there is no secretion a cell lysis typically occurs or is performed), optionally concentrated by concentration methods such as ultrafiltration and/or purified by purification means, such as affinity, ion exchange or gel filtration-type chromatography methods.

A "host cell" denotes a prokaryotic or eukaryotic cell that has been genetically altered, or is capable of being genetically altered by administration of an exogenous polynucleotide, such as a recombinant plasmid or vector. When referring to genetically altered cells, the term refers both to the originally altered cell and to the progeny thereof. Advantageous host cells include, but are not limited to, baby hamster kidney (BHK) cells, colon carcinoma (Caco-2) cells, COS7 cells, MCF-7 cells, MCF-10A cells, Madin-Darby canine kidney (MDCK) lines, mink lung (Mv1Lu) cells, MRC-5 cells, U937 cells and VERO cells. Polynucleotides comprising a desired sequence can be inserted into a suitable cloning or expression vector, and the vector in turn can be introduced into a suitable host cell for replication and amplification. Polynucleotides can be introduced into host cells by any means known in the art. The vectors containing the polynucleotides of interest can be introduced into the host cell by any of a number of appropriate means, including direct uptake, endocytosis, transfection, f-mating, electroporation, transfection employing calcium chloride, rubidium chloride, calcium phosphate, DEAE-dextran, or other substances; microprojectile bombardment; lipofection; and infection (where the vector is infectious, for instance, a retroviral vector). The choice of introducing vectors or polynucleotides will often depend on features of the host cell.

In an advantageous embodiment, the description provides for the administration of a therapeutically effective amount of a formulation for the delivery and expression of an influenza antigen, epitope or immunogen in a target cell. Determination of the therapeutically effective amount is routine experimentation for one of ordinary skill in the art. In one embodiment, the formulation comprises an expression vector comprising a polynucleotide that expresses an influenza antigen, epitope or immunogen and a pharmaceutically or veterinarily acceptable carrier, vehicle or excipient. In an advantageous embodiment, the pharmaceutically or veterinarily acceptable carrier, vehicle or excipient facilitates transfection and/or improves preservation of the vector or protein.

The pharmaceutically or veterinarily acceptable carriers or vehicles or excipients are well known to the one skilled in the art. For example, a pharmaceutically or veterinarily acceptable carrier or vehicle or excipient can be a 0.9% NaCl (e.g., saline) solution or a phosphate buffer. Other pharmaceutically or veterinarily acceptable carrier or vehicle or excipients that can be used for methods of this description include, but are not limited to, poly-(L-glutamate) or polyvinylpyrrolidone. The pharmaceutically or veterinarily acceptable carrier or vehicle or excipients may be any compound or combination of compounds facilitating the administration of the vector (or protein expressed from a vector *in vitro*); advantageously, the carrier, vehicle or excipient may facilitate transfection and/or improve preservation of the vector (or protein). Doses and dose volumes are herein discussed in the general description and can also be determined by the skilled artisan from this disclosure read in conjunction with the knowledge in the art, without any undue experimentation.

The cationic lipids containing a quaternary ammonium salt which are advantageously but not exclusively suitable for plasmids, are advantageously those having the following formula: 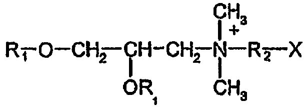 in which R₁ is a saturated or unsaturated straight-chain aliphatic radical having 12 to 18 carbon atoms, R₂ is another aliphatic radical containing 2 or 3 carbon atoms and X is an amine or hydroxyl group, e.g. the DMRIE. In another embodiment the cationic lipid can be associated with a neutral lipid, e.g. the DOPE.

Among these cationic lipids, preference is given to DMRIE (N-(2-hydroxyethyl)-N,N-dimethyl-2,3-bis(tetradecyloxy)-1-propane ammonium; WO96/34109), advantageously associated with a neutral lipid, advantageously DOPE (dioleoyl-phosphatidyl-ethanol amine; Behr J. P., 1994, Bioconjugate Chemistry, 5, 382-389), to form DMRIE-DOPE.

Advantageously, the plasmid mixture with the adjuvant is formed extemporaneously and advantageously contemporaneously with administration of the preparation or shortly before administration of the preparation; for instance, shortly before or prior to administration, the plasmid-adjuvant mixture is formed, advantageously so as to give enough time prior to administration for the mixture to form a complex, e.g. between about 10 and about 60 minutes prior to administration, such as approximately 30 minutes prior to administration.

When DOPE is present, the DMRIE:DOPE molar ratio is advantageously about 95: about 5 to about 5:about 95, more advantageously about 1: about 1, e.g.,1:1.

The DMRIE or DMRIE-DOPE adjuvant-plasmid weight ratio can be between about 50: about 1 and about 1: about 10, such as about 10: about 1 and about 1:about 5, and advantageously about 1: about 1 and about 1: about 2, e.g., 1:1 and 1:2.

The description also provides for inactivated canine influenza vaccines. As used herein, the term "inactivated vaccine" means a vaccine composition containing an infectious organism or pathogen that is no longer capable of replication or growth. Inactivation may be accomplished by a variety of methods sufficient to prevent replication or growth of the organism while maintaining its immunogenicity.

The inactivated vaccine may be an inactivated form of an isolate of an influenza virus from an affected dog. The virus may be isolated from the alveoli or lung of an affected dog. In another embodiment, the inactivated vaccine may be an inactivated equine influenza. In an advantageous embodiment, the equine influenza is a Kentucky or Newmarket equine influenza. The inactivated vaccine may be an inactivated version of any one of the influenza strains described above.

An inactivated vaccine may be prepared as well from the harvested culture fluid. The virus may be produced either by inoculation of 10-11-day embryonated eggs (J. Violay et al US6,048,537) or by inoculation of BHK-21 cell culture (C. Ross et al Archiv. Für die gesamte Virusforschung 1970, 30, 82-88; T. Tolstova et al Acta Virol. 1966,10, 315-321; Ho. Merten et al Adv. Exp. Med. Biol. 1996, 397,141-151), of MDCK cell culture (J. Tree et al Vaccine 2001, 19, 3444-3450; Y. Ghendon et al Vaccine 2005, 23, 4678-4684; R. Brands et al Dev. Biol. Stand.1999, 98, 93-100; R Youil et al J Virol. Methods 2004,120,23-31), of Vero cell culture (O. Kistner et al Vaccine 1998, 16, 960-968; E. Govorkova et al J. Virol. 1996, 70, 5519-5524). The allantoic fluid or the cell culture supernatant can be clarified by low centrifugation and/or filtration. The virus can be concentrated by ultrafiltration and can be purified by zonal centrifugation on sucrose gradient (J. Violay et al US6,048,537; O. Kistner et al idem), by gel filtration (D. Nayak et al J. Chromatogr. B Analyt. Technol. Biomed. Life Sci. 2005, 823, 75-81; S. Tomita et al Kitasato Arch. Exp. Med. 1971, 44, 185-196).

Inactivation may be achieved by treating the viruses by any of the methods commonly employed to make inactivated vaccines. These methods include but are not limited to formaldehyde treatment (O. Kistner et al idem; A. Grarcia et al Avian Diseases 1998, 42, 248-256), betapropriolactone treatment (B. Bdowsky et al Vaccine 1991, 9, 398-402 and Vaccine 1993, 11, 343-348; N. Keverin et al Arch. ViroL 2000, 145, 1059-1066), ethylene-imine treatment (D. Swayne et al Avian Diseases 2001, 45, 355-365), treatment with organic solvents, treatment with detergents, treatment with Tween-ether or treatment with Triton X-100 (J. Vilay et al idem) for allantoic fluid . For the inactivation the concentration can be about 0.01-0.2 % w/v for the formaldehyde; about 0.03-0.2 % w/v for the betapropiolactone; about 0.5-20 mM for ethyleneimine. The methods recited herein serve as art-known examples for inactivating virus. Inactivated virus vaccines are usually administered mixed with an adjuvant. The inactivated vaccine can be administered to the animal by any of a plurality of methods which include but are not limited to inoculation intramuscularly or subcutaneously, spraying, ocularly, nasally, orally, or in ovo.

The immunogenic compositions and vaccines according to the description may comprise or consist essentially of one or more adjuvants. Suitable adjuvants for use in the practice of the present invention are (1) polymers of acrylic or methacrylic acid, maleic anhydride and alkenyl derivative polymers, (2) immunostimulating sequences (ISS), such as oligodeoxyribonucleotide sequences having one ore more non-methytated CpG units (Klinman et al., Proc. Natl. Acad. Sci., USA, 1996, 93, 2879-2883; WO98/16247), (3) an oil in water emulsion, such as the SPT emulsion described on p 147 of "Vaccine Design, The Subunit and Adjuvant Approach" published by M. Powell, M. Newman, Plenum Press 1995, and the emulsion MF59 described on p 183 of the same work, (4) cation lipids containing a quaternary ammonium salt, e.g., DDA (5) cytokines, (6) aluminum hydroxide or aluminum phosphate, (7) sapoilin or (8) other adjuvants discussed in any document cited in the instant application, or (9) any combinations or mixtures thereof.

The oil in water emulsion (3), which is especially appropriate for viral vectors, can be based on: light liquid paraffin oil (European pharmacopoeia type), isoprenoid oil such as squalane, squalene, oil resulting from the oligomerization of alkenes, e.g. isobutene or decene, esters of acids or alcohols having a straight-chain alkyl group, such as vegetable oils, ethyl oleate, propylene glycol, di(caprylate/caprate), glycerol tri(caprylate/caprate) and propylene glycol dioleate, or esters of branched, fatty alcohols or acids, especially isostearic acid esters.

The oil is used in combination with emulsifiers to form an emulsion. The emulsifiers may be nonionic surfactants, such as: esters of on the one hand sorbitan, mannide (e.g. anhydromannitol oleate), glycerol, polyglycerol or propylene glycol and on the other hand oleic, isostearic, ricinoleic or hydroxystearic acids, said esters being optionally ethoxylated, or polyoxypropylene-polyoxyethylene copolymer blocks, such as Pluronic, e.g., L121.

Among the type (1) adjuvant polymers, preference is given to polymers of crosslinked acrylic or methacrylic acid, especially crosslinked by polyalkenyl ethers of sugars or polyalcohols. These compounds are known under the name carbomer (Pharmeuropa, vol. 8, no. 2, June 1996). One skilled in the art can also refer to U.S. Patent No. 2,909,462, which provides such acrylic polymers crosslinked by a polyhydroxyl compound having at least three hydroxyl groups, preferably no more than eight such groups, the hydrogen atoms of at least three hydroxyl groups being replaced by unsaturated, aliphatic radicals having at least two carbon atoms. The preferred radicals are those containing 2 to 4 carbon atoms, e.g. vinyls, allyls and other ethylenically unsaturated groups. The unsaturated radicals can also contain other substituents, such as methyl. Products sold under the name Carbopol (BF Goodrich, Ohio, USA) are especially suitable. They are crosslinked by allyl saccharose or by allyl pentaerythritol: Among them, reference is made to Carbopol 974P, 934P and 971P.

As to the maleic anhydride-alkenyl derivative copolymers, preference is given to EMA (Monsanto), which are straight-chain or crosslinked ethylene-maleic anhydride copolymers and they are, for example, crosslinked by divinyl ether. Reference is also made to J. Fields et al., Nature 186: 778-780, June 4, 1960.

With regard to structure, the acrylic or methacrylic acid polymers and EMA are preferably formed by basic units having the following formula: 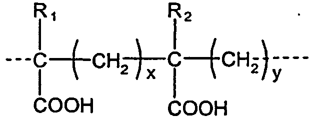 in which:
- R₁ and R₂, which can be the same or different, represent H or CH₃
- x = 0 or 1, preferably x = 1
- y = 1 or 2, with x+y=2.

For EMA, x = 0 and y = 2 and for carbomers x = y = 1.

These polymers are soluble in water or physiological salt solution (20 g/l NaCl) and the pH can be adjusted to 7.3 to 7.4, e.g., by soda (NaOH), to provide the adjuvant solution in which the expression vector(s) can be incorporated. The polymer concentration in the final vaccine composition can range between 0.01 and 1.5% w/v, advantageously 0.05 to 1% w/v and preferably 0.1 to 0.4% w/v.

The cytokine or cytokines (5) can be in protein form in the immunogenic or vaccine composition, or can be co-expressed in the host with the immunogen or immunogens or epitope(s) thereof. Preference is given to the co-expression of the cytokine or cytokines, either by the same vector as that expressing the immunogen or immunogens or epitope(s) thereof, or by a separate vector therefor.

The description comprehends preparing such combination compositions; for instance by admixing the active components, advantageously together and with an adjuvant, carrier, cytokine, and/or diluent

Cytokines that may be used in the present description include, but are not limited to, granulocyte colony stimulating factor (G-CSF), granulocyte/macrophage colony stimulating factor (GM-CSF), interferon α (IFN α), interferon β (IFN β), interferon γ, (IFN γ), interleukin-1α (IL-1α), interleukin-1 β (IL-1β), interleukin-2 (IL-2), interleukin-3 (IL-3), interleukin-4 (IL-4), interleukin-5 (IL-5), interleukin-6 (IL-6), interleukin-7 (IL-7), interleukin-8 (IL-8), interleukin-9 (IL-9), interleukin-10 (IL-10), interleukin-11 (IL-11), interleukin-12 (IL-12), tumor necrosis factor α (TNF α), tumor necrosis factor β (TNF β), and transforming growth factor β (TGF β). It is understood that cytokines can be co-administered and/or sequentially administered with the immunogenic or vaccine composition of the present description. Thus, for instance, the vaccine of the instant description can also contain an exogenous nucleic acid molecule that expresses in vivo a suitable cytokine, e.g., a cytokine matched to this host to be vaccinated or in which an immunological response is to be elicited (for instance, a canine cytokine for preparations to be administered to dogs).

Advantageously, the pharmaceutical and/or therapeutic compositions and/or formulations according to the description comprise or consist essentially of or consist of an effective quantity to elicit a therapeutic response of one or more expression vectors and/or polypeptides as discussed herein; and, an effective quantity can be determined from this disclosure, and the knowledge in the art, without undue experimentation.

In the case of therapeutic and/or pharmaceutical compositions based on a plasmid vector, a dose can comprise, consist essentially of or consist of, in general terms, about in 1 µg to about 2000 µg, advantageously about 50 µg to about 1000 µg and more advantageously from about 100 µg to about 800 µg of plasmid expressing the influenza antigen, epitope or immunogen. When the therapeutic and/or pharmaceutical compositions based on a plasmid vector is administered with electroporation the dose of plasmid is generally between about 0.1 µg and 1mg, advantageously between about 1 µg and 100 µg, advantageously between about 2 µg and 50 µg. The dose volumes can be between about 0.1 and about 2 ml, advantageously between about 0.2 and about 1 ml. These doses and dose volumes are suitable for the treatment of canines and other mammalian target species such as equines and felines.

The therapeutic and/or pharmaceutical composition contains per dose from about 10⁴ to about 10¹¹, advantageously from about 10⁵ to about 10¹⁰ and more advantageously from about 10⁶ to about 10⁹ viral particles of recombinant adenovirus expressing an influenza antigen, epitope or immunogen. In the case of therapeutic and/or pharmaceutical compositions based on a poxvirus, a dose can be between about 10² pfu and about 10⁹ pfu. The pharmaceutical composition contains per dose from about 10⁵ to 10⁹, advantageously from about 10⁶ to 10⁸ pfu of poxvirus or herpesvirus recombinant expressing the influenza antigen, epitope or immunogen.

The dose volume of compositions for target species that are mammals, e.g., the dose volume of canine compositions, based on viral vectors, e.g., non-poxvirus-viral-vector-based compositions, is generally between about 0.1 to about 2.0 ml, preferably between about 0.1 to about 1.0 ml, and more preferably between about 0.5 ml to about 1.0 ml.

With inactivated compositions of the virus or organism or pathogen produced on the new cell culture, the animal may be administered approximately 10⁴-10⁹ equivalent CCID₅₀ (titer before inactivation), advantageously approximately 10⁵-10⁸ equivalent CCID₅₀ in a single dosage unit. The volume of one single dosage unit can be between 0.2 ml and 5.0 ml and advantageously between 0.5 ml and 2.0 ml and more advantageously about 2.0 ml. One or more administrations can be done; e.g. with two injections at 2-4 weeks interval, and advantageously with a boost about 3 weeks after the first injection.

In an advantageous embodiment, an animal, advantageously a dog, is vaccinated with two doses of inactivated vaccine at about 3 to 4 week intervals via the subcutaneous route, although an intramuscular route is also contemplated. Blood samples may be collected on the day of the first and/or second vaccination and about 2 to 4 weeks after the second vaccination to determine the levels of anti-influenza virus specific antibodies by methods known to one of skill in the art, for example, virus neutralization, hemagglutination inhibition, ELISA or single radial heamolysis (SRH) tests.

The efficacy of the inactivated vaccines may be tested about 2 to 4 weeks after the second immunization by challenging animals, advantageously dogs, with a virulent strain of influenza, advantageously the influenza H3N8 strain. The animal may be challenged by spray, intra-nasally, intra-tracheally and/or orally. The challenge viral may be about 10⁵⁻⁸ EID50 in a volume depending upon the route of administration. For example, if the administration is by spray, a virus suspension is aerosolized to generate about 1 to 100 µm droplets, if the administration is intra-nasal, intra-tracheal or oral, the volume of the challenge virus is about 0.5 ml, 1-2 ml and 5-10 ml, respectively. Animals may be observed daily for 14 days following challenge for clinical signs, for example, fever, cough, nasal, ocular discharge, respiratory distress, anorexia and lethargy. In addition, groups of animals may be euthanized and evaluated for pathological findings of pulmonary and pleural hemorrhage, tracheitis, bronchitis, broncholilitis, and bronchopneumonia. Tracheal swabs may be collected from all animals post challenge days 1-14 for virus isolation. The presence or absence of viral antigens in respiratory tissues may be evaluated by immunohistochemistry, for example, on days 3, 7 and 10 post-challenge. Blood samples may be collected post-challenge (e.g., on days 7 and 14 post-challenge) and may be analyzed for the presence of anti-influenza H3N8 virus specific antibody.

It should be understood by one of skill in the art that the disclosure herein is provided by way of example and the present invention is not limited thereto. From the disclosure herein and the knowledge in the art, the skilled artisan can determine the number of administrations, the administration route, and the doses to be used for each injection protocol, without any undue experimentation.

The present description contemplates at least one administration to an animal of an efficient amount of the therapeutic composition made according to the description. The animal may be male, female, pregnant female and newborn. This administration may be via various routes including, but not limited to, intramuscular (IM), intradermal (ID) or subcutaneous (SC) injection or via intranasal or oral administration. The therapeutic composition according to the description can also be administered by a needleless apparatus (as, for example with a Pigjet, Biojector or Vitajet apparatus (Bioject, Oregon, USA)). Another approach to administer plasmid compositions is to use electroporation (see, e.g. S. Tollefsen et al. Vaccine, 2002, 20, 3370-3378; S. Tollefsen et al. Scand. J. Immunol., 2003, 57, 229-238; S. Babiuk et al., Vaccine, 2002, 20, 3399-3408; PCT Application No. WO99/01158). In another embodiment, the therapeutic composition is delivered to the animal by gene gun or gold particle bombardment. In an advantageous embodiment, the animal is a vertebrate. In a more advantageous embodiment, the vertebrate is a dog.

One embodiment of the description is a method of eliciting an immune response against influenza in an animal, comprising administering a formulation for delivery and expression of a recombinant poxvirus influenza vaccine or inactivated influenza vaccine in an effective amount for eliciting an immune response. Still another embodiment of the description is a method of inducing an immunological or protective response against influenza in an animal, comprising administering to the animal an effective amount of a formulation for delivery and expression of an influenza antigen, epitope or immunogen wherein the formulation comprises recombinant poxvirus influenza vaccine or inactivated influenza vaccine and a pharmaceutically or veterinarily acceptable carrier, vehicle or excipient.

The description relates to a method to elicit, induce or stimulate the immune response of an animal, advantageously a vertebrate. In one embodiment, the vertebrate is a dog but may also be a cat.

Another embodiment of the description is a kit for performing a method of inducing an immunological or protective response against influenza in an animal comprising a recombinant influenza poxvirus vaccine or an inactivated influenza vaccine and instructions for performing the method of delivery in an effective amount for eliciting an immune response in the animal.

The invention will now be further described by way of the following non-limiting examples.

### EXAMPLE 1: Vaccination Of Dogs With Canarypox Expressing H3 Genes

A study was conducted in which dogs were vaccinated dogs on days 0 and 21 with a canarypox expressing H3 genes from Kentucky (CP1529) and Newmarket (CP1533) equine influenza. The construction of CP1529 and CP1533 is described in Examples 1 (c5 locus), 4 and 5 of International Patent Publication WO99/44633.

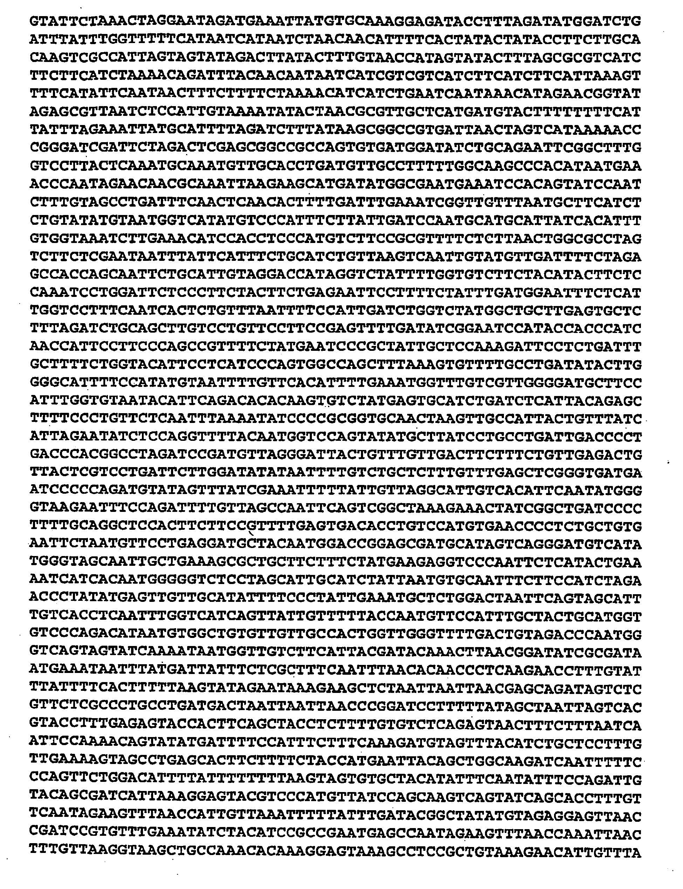

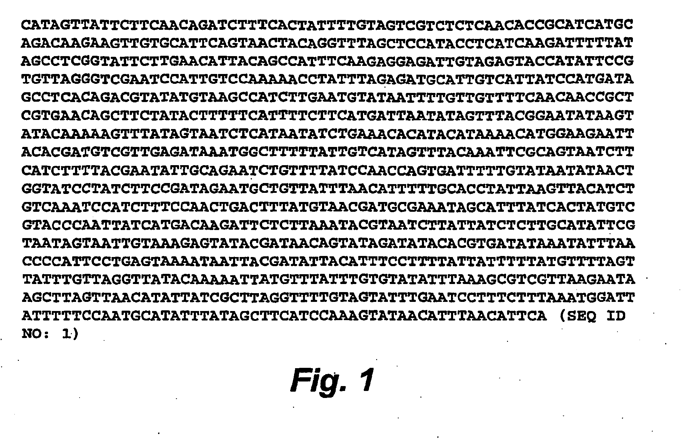

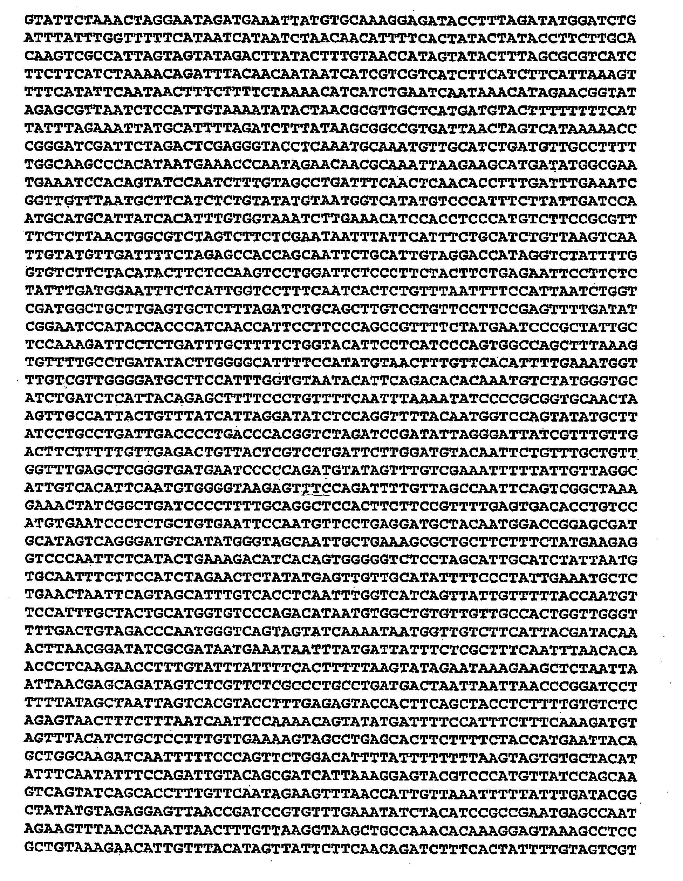

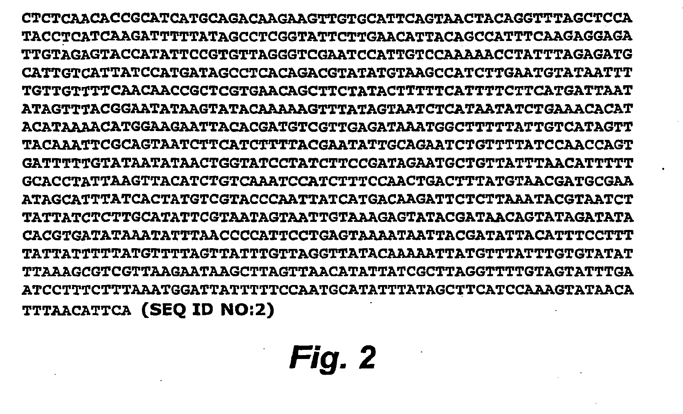

The nucleotide sequence of the donor plasmids pJT004 and pJT005 are presented in FIGS. 1 and 2.

Sera was collected and tested against H3N8 influenza viruses and the other viruses. As shown in Table 1, canarypox expressing hemagglutinin H3 genes induced a substantial amount of antibodies which specifically reacted with H3N8 strains but not with H1N1 or H7N7. Importantly antibodies were detectable within two weeks after the first immunization.

Accordingly, a canarypox expressing an influenza HA gene is immunogenic in dogs.

**Table 1: Vaccination of dogs with canarypox expressing H3 hemaglutinin genes**

| **Antigen** | **Bleed** | **VACCINATED GROUP** | | | | | **CONTROL GROUP** | | | | |
|---|---|---|---|---|---|---|---|---|---|---|---|
| N/1/93 (H3N8) | D0 | 0 | 0 | 0 | 0 | 0 | 0 | 0 | 0 | 0 | 0 |
| | D14 | 0 | 63.8 | 69.9 | 31.4 | 96.7 | n.s. | n.s. | n.s. | n.s. | n.s. |
| | D21 | 104.1 | 91.4 | 121.5 | 74.6 | 121.5 | n.s. | n.s. | n.s. | n.s. | n.s. |
| | D36 | 106 | 96.7 | 111.7 | 69.9 | 123.6 | n.s. | n.s. | n.s. | n.s. | n.s. |
| | D51 | 76.2 | 55.1 | 74.6 | 38.3 | 69.9 | n.s. | n.s. | n.s. | n.s. | n.s. |
| N/2/93 (H3N8) | D0 | 0 | 0 | 0 | 0 | 0 | 0 | 0 | 0 | 0 | 0 |
| | D14 | 0 | 25.1 | 59.4 | 29.2 | 60.8 | n.s. | n.s. | n.s. | n.s. | n.s. |
| | D21 | 86.2 | 71.4 | 102.2 | 65.3 | 406 | n.s. | n.s. | n.s. | n.s. | n.s. |
| | D36 | 82.8 | 74.6 | 96.7 | 62.3 | 100.4 | n.s. | n.s. | n.s. | n.s. | n.s. |
| | D51 | 52.3 | 37.1 | 66.8 | 26.1 | 57.9 | n.s. | n.s. | n.s. | n.s. | n.s. |
| Pr/56 (H7N7) | D0 | 0 | 0 | 0 | 0 | 0 | 0 | 0 | 0 | 0 | 0 |
| | D14 | 0 | 19.3 | 0 | 24.1 | 0 | n.s. | n.s. | n.s. | n.s. | n.s. |
| | D21 | 0 | 0 | 0 | 0 | 0 | n.s. | n.s. | n.s. | n.s. | n.s. |
| | D36 | 0 | 0 | 0 | 0 | 0 | n.s. | n.s. | n.s. | n.s. | n.s. |
| | D51 | 0 | 0 | 0 | 0 | 0 | n.s. | n.s. | n.s. | n.s. | n.s. |
| PR8 (H1N1) | D0 | 0 | 0 | 0 | 0 | 0 | 0 | 0 | 0 | 0 | 0 |
| | D14 | 0 | 16.7 | 0 | 19.3 | 0 | n.s. | n.s. | n.s. | n.s. | n.s. |
| | D21 | 0 | 0 | 0 | 0 | 0 | n.s. | n.s. | n.s. | n.s. | n.s. |
| | D36 | 0 | 0 | 0 | 0 | 0 | n.s. | n.s. | n.s. | n.s. | n.s. |
| | D51 | 0 | 0 | 0 | 0 | 0 | 0 | 0 | 0 | 0 | 0 |

| | | | | | | | | | | | |
|---|---|---|---|---|---|---|---|---|---|---|---|
| n.s. = no sample | | | | | | | | | | | |

### EXAMPLE 2: Contruction of the donor plasmid pALVAC C5 H6p-synthetic EIV H3 HA, pJY1571.1)

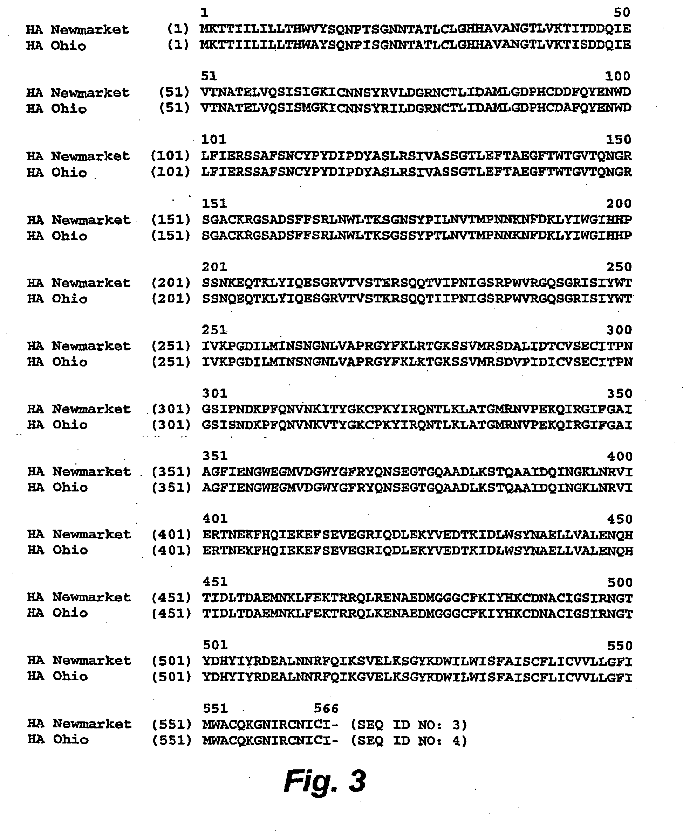

The HA gene was derived from equine influenza virus (EIV) H3N8 Ohio 03 strain isolated from a horse in 2003. The HA gene was synthetic with codon optimization for expression in mammlian cells. The amino acid sequence of EIV Ohio 03 strain HA was compared to that of New Market strain H3 HA and is presented in FIG. 3.

The purpose was the construction of an ALVAC donor plasmid for generation of an ALVAC recombinant expressing codon optimized EIV H3 HA (Ohio 03). The plasmid name was pALVAC C5 H6p-synthetic EIV H3 HA, pJY1571.1. The plasmid backbone is pALVAC C5 H6p. The promoter was a H6 promoter.

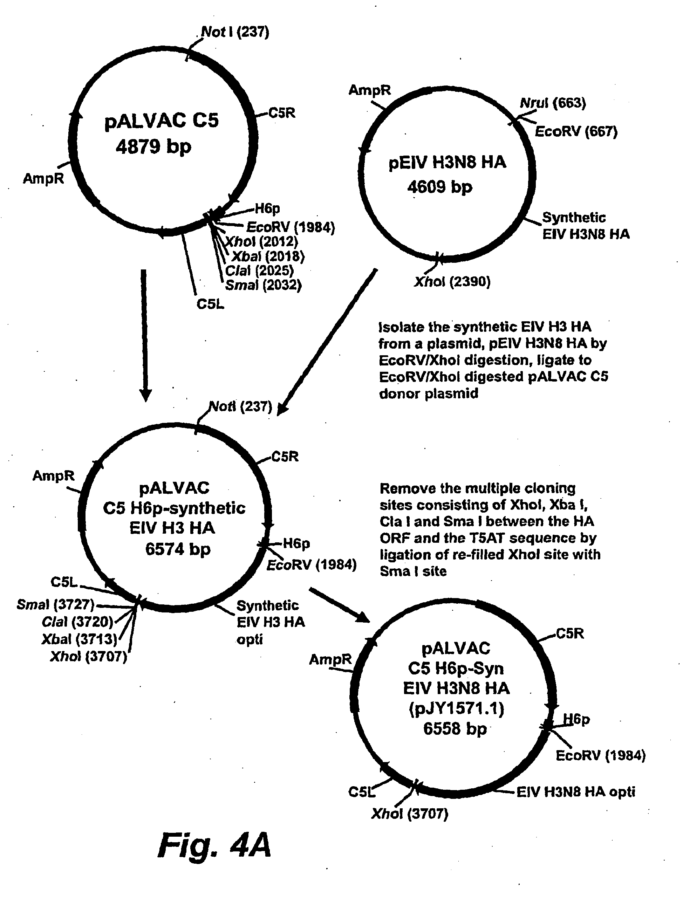

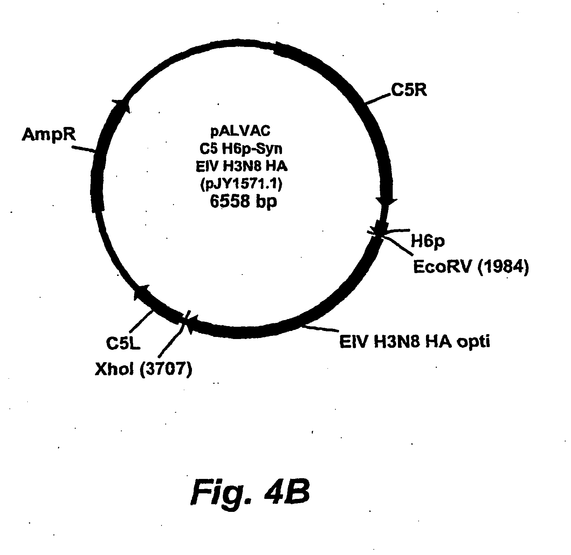

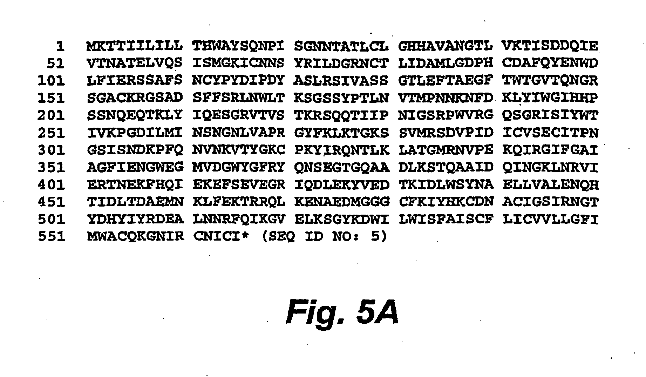

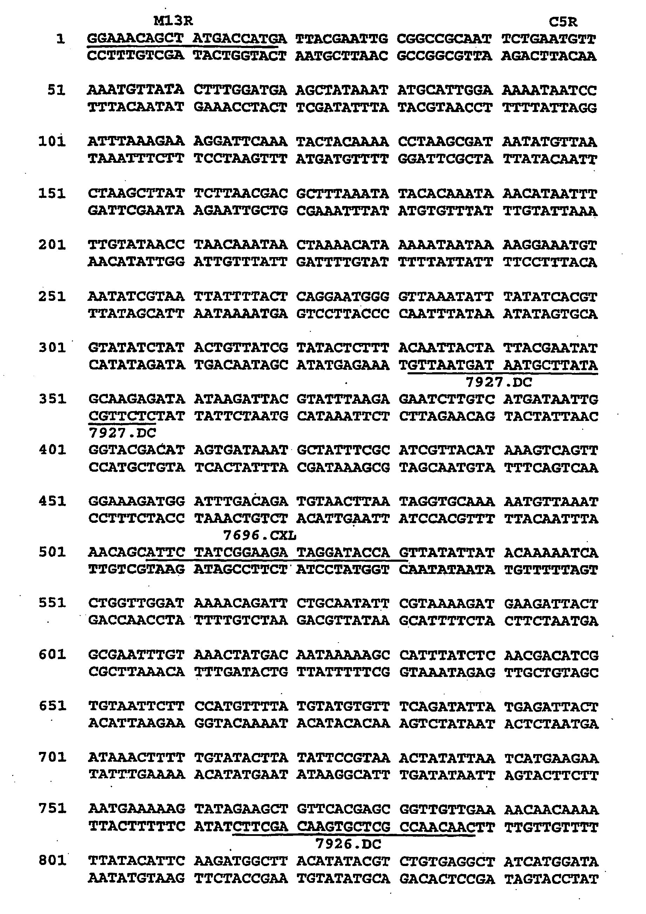

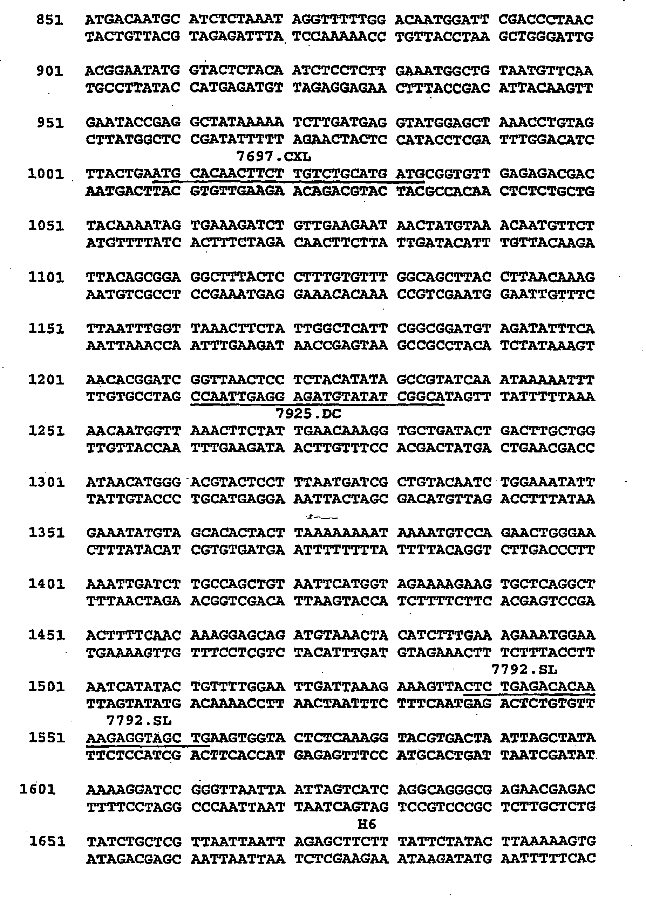

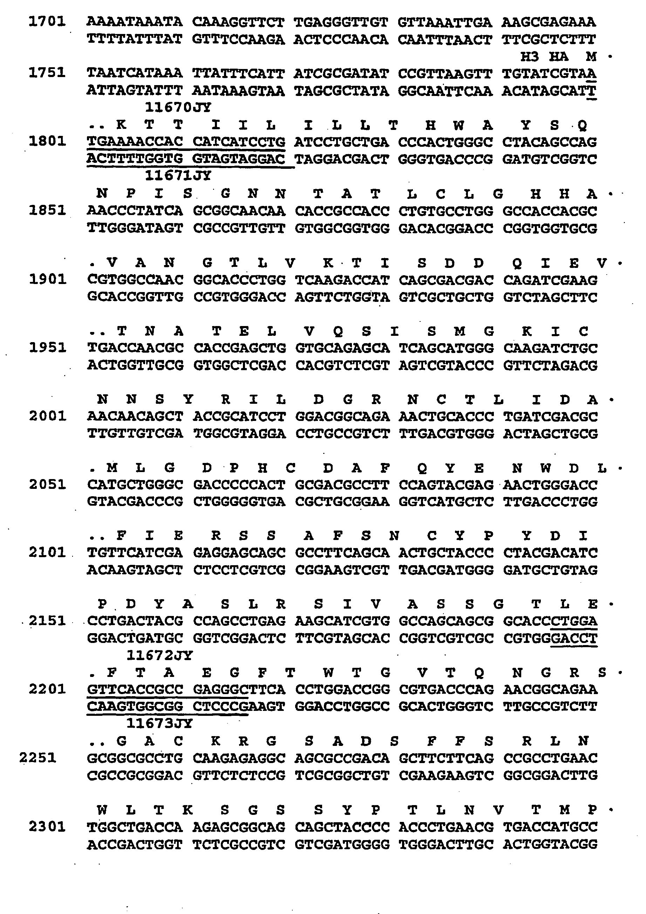

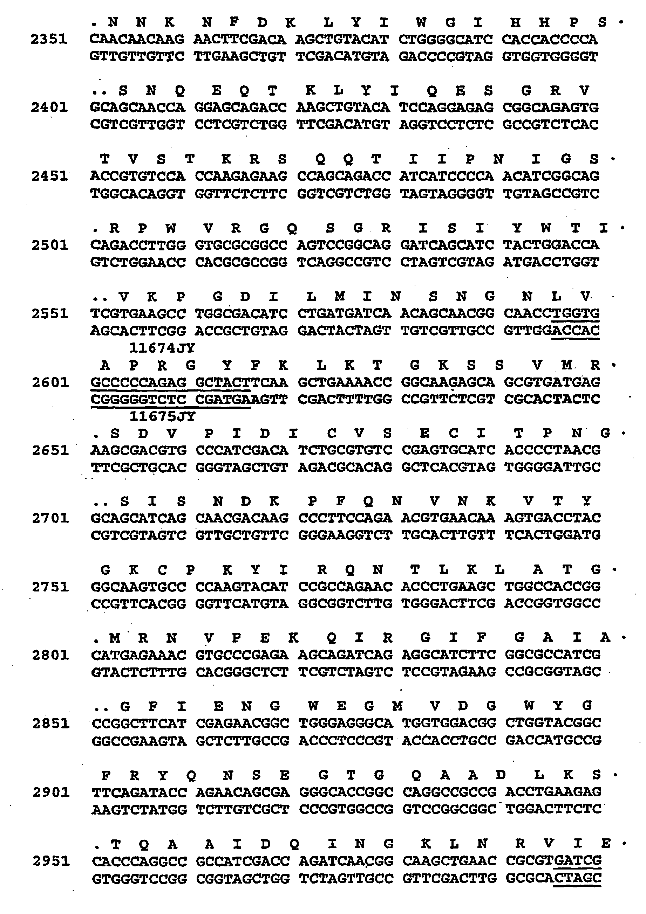

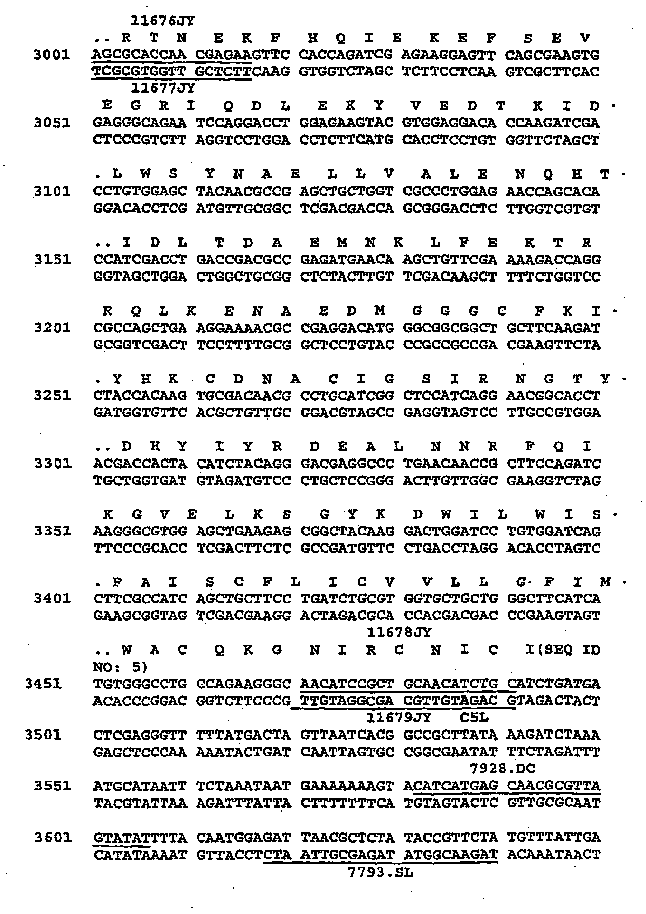

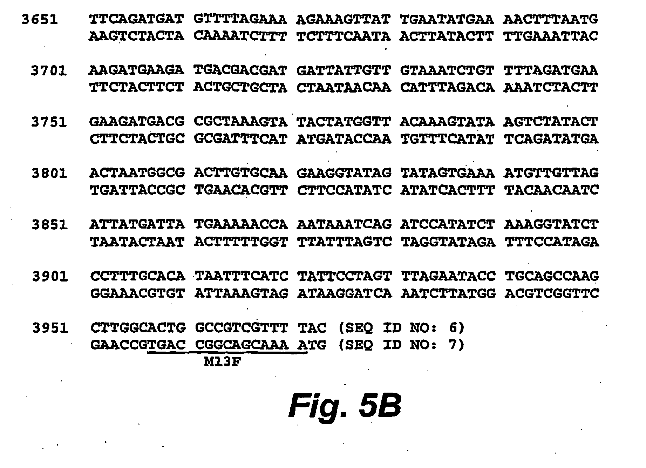

The description of plasmid construction was as follows and presented schematically in FIG. 4A. The synthetic EIV H3 HA (Ohio 03) was isolated from a plasmid pEIV H3N8 HA by EcoRV/Xhol digestion, and ligated to EcoRV/Xhol digested donor plasmid pALVAC C5 H6p to create pALVAC C5 H6p-Synthetic EIV H3 HA. In the resulting plasmid, there are multiple cloning sites consisting of Xhol, Xba I, Cla I and Sma I between the HA ORF and the T5AT sequence which serves as the transcription termination signal. To bring the HA ORF and the T5AT sequence close together, those cloning sites were then subsequently removed by ligation of re-filled Xhol site with Sma 1 site. The resulting plasmid pJY1571.1 was then sequenced and confirmed to contain the correct sequence. A diagram of the resulting plasmid is presented in FIG. 4B. The predicted amino acid sequence of EIV H3 HA is shown in FIG. 5A and the nucleotide sequence of arms and insert with translation is shown in FIG. 5B.

### EXAMPLE 3: Construction of the recombinant canarypox vCP2242

The purpose of this example was the generation and characterization of ALVAC recombinant containing EIV H3N8 codon optimized HA inserted at C5 loci of ALVAC (vCP2242). The parental virus was ALVAC, the donor plasmid was pJY1571.1, the insertion site was a C5 Locus, the promoter was a H6 promoter and cells for in vitro recombination were primary chicken embryo fibroblast cells (1°CEF).

The in vitro recombination (IVR) was performed by transfection of 1°CEF cells with 15 µg of Not I-linearized donor plasmid pJY1571.1. The transfected cells were subsequently infected with ALVAC as rescue virus at MOI of 10. After 24 hr, the transfected-infected cells were harvested, sonicated and used for recombinant virus screening.

Recombinant plaques were screened based on the plaque lift hybridization method using a 821 bp EIV syn HA specific probe labeled with horse radish peroxidase (HRP) according to the manufacturer's protocol. After four sequential rounds of plaque purification, the recombinant designated as vCP2242 was generated and confirmed by hybridization as 100% positive for the EIV syn HA insert and 100% negative for the C5 ORF.

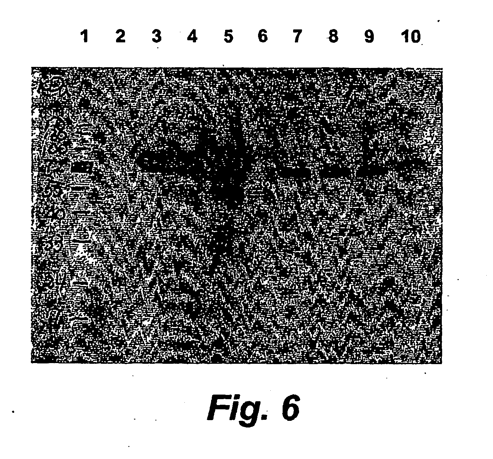

Expression analysis and sequence analysis were performed. Expression analysis was performed by Western blot. Primary CEF cells were infected with vCP2242 stock at MOI of 10 and incubated at 37 C for 26.5 hrs. The cells and culture supernatant were then harvested. Sample proteins were separated on a 10% SDS-PAGE gel, transferred to Immobilon nylon membrane, and probed with a pool of monoclonal mouse anti-EIV HA antibodies (anti Eq/AK/91: 124-1D9-1, 124-3E3-3, 124-4F3-2 and H3N8 A Eq/miami/63 pool at 1/1,000 dilution). Peroxidase-conjugated goat anti-mouse antiserum was used as a secondary antibody and the bands were visualized using luminol reagents. vCP2242 showed a protein expression profile with a 80 kDa protein expressed in cell pellet, but not in the culture medium as presented in FIG. 6.

Results of the sequence analysis demonstrated that the sequences of the EIV syn HA and C5L and C5R of ALVAC were correct.

### EXAMPLE 4: Kentucky Equine Influenza Nucleotide Sequences

DEFINITION Influenza A virus (A/equine/Kentucky/5/02(H3N8)) nonstructural protein gene, complete cds.

ACCESSION AY855345; SEQ ID NO: 8

1 agcaaaagca gggtgacaaa aacataatgg attccaacac tgtgtcaagc tttcaggtag

61 actgttttct ttggcatgtc cgcaaacgat tcgcagacca agaactgggt gatgccccat
121 tccttgaccg gcttcgccga gaccagaagt ccctaagggg aagaggtatc actcttggtc
181 tggacatcga aacagccact catgcaggaa agcagatagt ggagcagatt ctggaaaagg
241 aatcagatga ggcacttaaa atgaccattg cctctgttcc tacttcacgc tacttaactg
301 acatgactct tgatgagatg tcaagagact ggttcatgct catgcccaag caaaaagtaa
361 caggctccct atgtataaga atggaccagg caatcatgga taagaacatc atacttaaag
421 caaactttag tgtgattttc gaaaggctgg aaacactaat actacttaga gccttcaccg
481 aagaaggagc agtcgttggc gaaatttcac cattaccttc tcttccagga catactaatg
541 aggatgtcaa aaatgcaatt ggggtcctca tcggaggact taaatggaat gataatacgg
601 ttagaatctc tgaaactcta cagagattcg cttggagaag cagtcatgag aatgggagac
661 cttcattccc ttcaaagcag aaatgaaaaa tggagagaac aattaagcca gaaatttgaa
721 gaaataagat ggttgattga agaagtgcga catagattga aaaatacaga aaatagtttt
781 gaacaaataa catttatgca agccttacaa ctattgcttg aagtagaaca agagataaga
841 actttctcgt ttcagcttat ttaatgataa aaaacaccct tgtttctact

DEFINITION Influenza A virus (A/equine/Kentucky/5/02(H3N8)) matrix protein gene, complete cds.
ACCESSION AY855344; SEQ ID NO: 9

1 agcaaaagca ggtagatatt taaagatgag tcttctgacc gaggtcgaaa cgtacgttct
61 ctctatcgta ccatcaggcc ccctcaaagc cgagatcgcg cagagacttg aagatgtctt
121 tgcagggaag aacaccgatc ttgaggcact catggaatgg ctaaagacaa gaccaatcct
181 gtcacctttg actaaaggga ttttaggatt tgtattcacg ctcaccgtgc ccagtgagcg
241 aggactgcag cgtagacgct ttgtccaaaa tgcccttagt ggaaacggag atccaaacaa
301 catggacaga gcagtaaaac tgtacaggaa gcttaaaaga gaaataacat tccatggggc
361 aaaagaggtg gcactaagct attccactgg tgcactagcc agctgcatgg gactcatata
421 caacagaatg ggaactgtga caaccgaagt ggcatttggc ctggtatgcg ccacatgtga
481 acagattgct gattcccagc atcgatctca caggcagatg gtgacaacaa ccaacccatt
541 aatcagacat gaaaacagaa tggtattagc cagtaccacg gctaaagcca tggaacagat
601 ggcaggatca agtgagcagg cagcagaggc catggaggtt gctagtaagg ctaggcagat
661 ggtacaggca atgagaacca ttgggaccca ccctagctcc agtgccggtt tgaaagatga
721 tctccttgaa aatttacagg cctaccagaa acggatggga gtgcaaatgc agcgattcaa
781 gtgatcctct cgttattgca gcaagtatca ttgggatctt gcacttgata ttgtggattc
841 ttgatcgtct tttcttcaaa ttcatttatc gtcgccttaa atacgggttg aaaagagggc
901 cttctacgga aggagtacct gagtctatga gggaagaata tcggcaggaa cagcagaatg
961 ctgtggatgt tgacgatggt cattttgtca acatagagct ggagtaaaaa actaccttgt
1021 ttctact

DEFINITION Influenza A virus (A/equine/Kentucky/5/02(H3N8)) neuraminidase gene, complete cds.
ACCESSION AY855343; SEQ ID NO: 10

1 agcaaaagca ggagtttaaa atgaatccaa atcaaaagat aatagcaatt ggatttgcat
61 cattggggat attaatcatt aacgtcattc tccatgtagt cagcattata gtaacagtac
121 tggtcctcaa taacaatgga acaggtctga actgcaaagg gacgatcata agagagtaca
181 atgaaacagt aagagtagaa aaaattactc aatggtataa taccagtgca attaagtaca
241 tagagagacc tccaaatgaa tactacatga acaacaccga accactttgt gaggcccaag
301 gctttgcacc attttccaaa gataatggaa tacgaattgg gtcgagaggc catgtttttg
361 tgataagaga accttttgta tcatgttcgc cctcagaatg tagaaccttt ttcctcacac
421 agggctcatt actcaatgac aaacattcta acggcacagt aaaggaccga agtccatata
481 ggactttgat gagtgtcaaa atagggcaat cacctaatgt gtatcaagct aggtttgaat
541 cggtggcatg gtcagcaaca gcatgccatg atggaaaaaa atggatgaca gttggagtca
601 cagggcccga caatcaagca attgcagtag tgaactatgg aggtgttccg gttgatatta
661 ttaattcatg ggcaggggat atcttaagaa cccaagaatc atcatgcacc tgcattaaag
721 gagactgtta ttgggtaatg actgatggac cggcaaatag gcaagctaaa tataggatat
781 tcaaagcaaa agatggaaga gtaattggac agactgatat aagtttcaat gggggacaca
841 tagaggagtg ttcttgttac cccaatgaag ggaaggtgga atgcatatgc agggacaatt
901 ggactggaac aaatagacca attctggtaa tatcttctga tctatcgtac acagttggat

961 atttgtgtgc tggcattccc actgacactc ctaggggaga ggatagtcaa ttcacaggct
1021 catgtacaag acctttggga aataaaggat acggtgtaaa aggtttcggg tttcgacaag
1081 gaactgacgt atgggccgga aggacaatta gtaggacttc aagatcagga ttcgaaataa
1141 taaaaatcag gaatggttgg acacagaaca gtaaagacca aatcaggagg caagtgatta
1201 tcgatgaccc aaattggtca ggatatagcg gttctttcac attgccggtt gaactaacaa
1261 aaaagggatg tttggtcccc tgtttctggg ttgaaatgat tagaggtaaa cctgaagaaa
1321 caacaatatg gacctctagc agctccattg tgatgtgtgg agtagatcat aaaattgcca
1381 gttggtcatg gcacgatgga gctattcttc cctttgacat cgataagatg taatttacga
1441 aaaaactcct tgtttctact

DEFINITION Influenza A virus (A/equine/Kentucky/5/02(H3N8)) nucleoprotein gene, complete cds.
ACCESSION AY855342; SEQ ID NO: 11

1 agcaaaagca gggtagataa tcactcactg agtgacatca aagtcatggc gtctcaaggc
61 accaaacgat cctatgaaca gatggaaact gatggggaac gccagaatgc aactgaaatc
121 agagcatctg tcggaaggat ggtgggagga atcggccggt tttatgttca gatgtgtact
181 gagcttaaac taaacgacca tgaagggcgg ctgattcaga acagcataac aatagaaagg
241 atggtacttt cggcattcga cgaaagaaga aacaagtatc tcgaggagca tcccagtgct
301 ggaaaagacc ctaagaaaac gggaggcccg atatacagaa ggaaagatgg gaaatggatg
361 agggaactca tcctccatga taaagaagaa atcatgagaa tctggcgtca ggccaacaat
421 ggtgaagacg ctactgctgg tcttactcat atgatgatct ggcactccaa tctcaatgac
481 accacatacc aaagaacaag ggctcttgtt cggactggga tggatcccag aatgtgctct
541 ctgatgcaag gctcaaccct cccacggaga tctggagccg ctggtgctgc agtaaaaggt
601 gttggaacaa tggtaatgga actcatcaga atgatcaaac gcggaataaa tgatcggaat
661 ttctggagag gtgaaaatgg tcgaaggacc agaattgctt atgaaagaat gtgcaatatc
721 ctcaaaggga aatttcagac agcagcacaa cgggctatga tggaccaggt gagggaaggc
781 cgcaatcctg gaaacgctga gattgaggat ctcattttct tagcacgatc agcacttatt
841 ttgagaggat cagtagccca taaatcatgc ctacctgcct gtgtttatgg ccttgcagta
901 accagtgggt atgactttga gaaggaagga tactctctgg ttggaattga tcctttcaaa
961 ctactccaga acagtcaaat tttcagtcta atcagaccaa aagaaaaccc agcacacaag
1021 agccagttgg tgtggatggc atgccattct gcagcatttg aggacctgag agttttgaat
1081 ttcattagag gaaccaaagt aatcccaaga ggacagttaa caaccagagg agttcaaatt
1141 gcttcaaatg aaaacatgga gacaatagat tctagcacac ttgaactgag aagcaaatat
1201 tgggcaataa ggaccagaag tggaggaaac accagtcaac agagagcatc tgcaggacag
1261 ataagtgtgc aacctacttt ctcagtacag agaaatcttc cctttgagag agcaaccatt
1321 atggctgcat tcactggtaa cactgaaggg aggacttccg acatgagaac ggaaatcata
1381 aggatgatgg aaagtgccaa atcagaagat gtgtctttcc aggggcgggg agtcttcgag
1441 ctctcggacg aaaaggcaac gaacccgatc gtgccttcct ttgacatgag caatgaaggg
1501 tcttatttct tcggagacaa tgctgaggaa tttgacagtt aaagaaaaat acccttgttt
1561 ctact

DEFINITION Influenza A virus (A/equine/Kentucky/5/02(H3N8)) hemagglutinin precursor, gene, complete cds.
ACCESSION AY855341; SEQ ID NO: 12

1 agcaaaagca ggggatattt ctgtcaatca tgaagacaac cattattttg atactactga
61 cccattgggc ctacagtcaa aacccaatca gtggcaacaa cacagccaca ttgtgtctgg
121 gacaccatgc agtagcaaat ggaacattgg taaaaacaat aagtgatgat caaattgagg
181 tgacaaatgc tacagaatta gttcagagca tttcaatggg gaaaatatgc aacaactcat
241 atagaattct agatggaaga aattgcacat taatagatgc aatgctagga gacccccact
301 gtgacgtctt tcagtatgag aattgggacc tctttataga aagaagcagc gctttcagca
361 attgctaccc atatgacatc cctgactatg catcgctccg atccattgta gcatcctcag
421 gaacattgga attcacagca gagggattca catggacagg tgtcactcaa aacggaagaa
481 gtggagcctg caaaagggga tcagccgata gtttctttag ccgactgaat tggctaacaa
541 aatctggaaa ctcttacccc acattgaatg tgacaatgcc taacaataaa aatttcgaca
601 agctatacat ctgggggatt catcacccga gctcaatcaa agagcagaca aaattgtaca
661 tccaagaatc aggacgagta acagtctcaa caaaaagaag tcaacaaaca ataatcccta
721 acatcggatc tagaccgtgg gtcagaggtc aatcaggcag gataagcata tactggacca
781 ttgtaaaacc tggagatatc ctaatgataa acagtaatgg caacttagtt gcaccgcggg

841 gatattttaa attgaaaaca gggaaaagct ctgtaatgag atcagatgca cccatagaca
901 tttgtgtgtc tgaatgtatt acaccaaatg gaagcatctc caacgacaag ccattccaaa
961 atgtgaacaa agttacatat ggaaaatgcc ccaagtatat caggcaaaac actttaaagt
1021 tggccactgg gatgaggaat gtaccagaaa agcaaatcag aggaatcttt ggagcaatag
1081 cgggattcat cgaaaacggc tgggaaggaa tggttgatgg gtggtatggg ttccgatatc
1141 aaaactctga aggaacaggg caagctgcag atctaaagag cactcaagca gccatcgacc
1201 agattaatgg aaagttaaac agagtgattg aaagaaccaa tgagaaattc catcaaatag
1261 agaaggaatt ctcagaagta gaaggaagaa ttcaggactt ggagaaatat gtagaagaca
1321 ccaaaataga cctatggtcc tacaatgcag aattgctggt ggctctagaa aatcaacata
1381 caattgactt aacagatgca gaaatgaata aattattcga gaagactaga cgccagttaa
1441 gagaaaacgc agaagacatg ggaggtggat gtttcaagat ttaccacaaa tgtgataatg
1501 catgcattgg atcaataaga aatgggacat atgaccatta catatacaga gatgaagcat
1561 taaacaaccg atttcaaatc aaaggtgttg agttgaaatc aggctacaaa gattggatac
1621 tgtggatttc attcgccata tcatgcttct taatttgcgt tgttctattg ggtttcatta
1681 tgtgggcttg ccaaaaaggc aacatcagat gcaacatttg catttgagta aactgatagt
1741 taaaaacacc cttgtttcta ct

DEFINITION Influenza A virus (A/equine/Kentucky/5/02(H3N8)) PA polymerase gene, complete cds.
ACCESSION AY855340; SEQ ID NO: 13

1 agcgaaagca ggtactgatc caaaatggaa gactttgtgc gacagtgctt caatccaatg
61 atcgtcgagc ttgcggaaaa ggcaatgaaa gaatatggag aggacccgaa aatcgaaaca
121 aacaaatttg cagcaatatg cactcacttg gaagtctgct tcatgtactc ggatttccac
181 tttattaatg aactgggtga gtcagtggtc atagagtctg gtgacccaaa tgctcttttg
241 aaacacagat ttgaaatcat tgagggagaa gatcgaacaa tggcatggac agtggtaaac
301 agcatctgca acaccacaag agctgaaaaa cctaaatttc ttccagattt atacgactat
361 aaggagaaca gatttgttga aattggtgtg acaaggagag aagttcacat atactacctg
421 gagaaggcca acaaaataaa gtctgagaaa acacatatcc acattttctc atttacagga
481 gaggaaatgg ctacaaaagc ggactatact cttgatgaag agagtagagc caggatcaag
541 accagactat tcactataag acaagaaatg gccagtagag gcctctggga ttcctttcgt
601 cagtccgaga gaggcgaaga gacaattgaa gaaagatttg aaatcacagg gacgatgcgc
661 aagcttgcca attacagtct cccaccgaac ttctccagcc ttgaaaattt tagagtctat
721 gtggatggat tcgaaccgaa cggctgcatt gagagtaagc tttctcaaat gtccaaagaa
781 gtaaatgcca gaatcgaacc attttcaaag acaacacccc gaccactcaa aatgccaggt
841 ggtccaccct gccatcagcg atctaaattc ttgctaatgg atgctctgaa actgagcatt
901 gaggacccaa gtcacgaggg agagggaata ccactatatg atgcaatcaa atgcatgaaa
961 actttctttg gatggaaaga gcccagtatt gttaaaccac atgaaaaggg tataaacccg
1021 aactatctcc aaacttggaa gcaagtatta gaagaaatac aagaccttga gaacgaagaa
1081 aggaccccca agaccaagaa tatgaaaaaa acaagccaat tgaaatgggc actaggtgaa
1141 aatatggcac cagagaaagt ggattttgag gattgtaaag acatcagtga tttaaaacag
1201 tatgacagcg atgagccaga aacaaggtct cttgcaagtt ggattcaaag tgagttcaac
1261 aaagcttgtg agctgacaga ttcaagctgg atagagctcg atgaaattgg ggaggatgtc
1321 gccccaatag aatacattgc gagcatgagg agaaattatt ttactgctga gatttcccat
1381 tgtagagcaa cagaatatat aatgaaagga gtgtacatca acactgctct actcaatgca
1441 tcctgtgctg cgatggatga atttcaatta attccgatga taagtaaatg caggaccaaa
1501 gaagggagaa ggaaaacaaa tttatatgga ttcataataa agggaaggtc ccatttaaga
1561 aatgatactg acgtggtgaa ctttgtaagt atggaatttt ctctcactga tccaagattt
1621 gagccacaca aatgggaaaa atactgcgtt ctagaaattg gagacatgct tctaaggact
1681 gctgtaggtc aagtgtcaag acccatgttt ttgtatgtaa ggacaaatgg aacctctaaa
1741 attaaaatga aatggggaat ggaaatgagg cgctgcctcc ttcagtctct gcaacagatt
1801 gaaagcatga tcgaagctga gtcctcagtc aaagaaaagg acatgaccaa agaatttttt
1861 gagaacaaat cagagacatg gcctatagga gagtccccca aaggagtgga agaggctca
1921 atcgggaagg tttgcaggac cttattagca aaatctgtgt ttaacagttt atatgcatct
1981 ccacaactgg aagggttttc agctgaatct aggaaattac ttctcattgt tcaggctctt
2041 agggataacc tggaacctgg aacctttgat attggggggt tatatgaatc aattgaggag
2101 tgcctgatta atgatccctg ggttttgctt aatgcatctt ggttcaactc cttccttaca
2161 catgcactga agtagttgtg gcaatgctac tatttgctat ccatactgtc caaaaaagta
2221 ccttgtttct act

DEFINITION Influenza A virus (A/equine/Kentucky/5/02(H3N8)) PB1 polymerase 1 gene, complete cds.
ACCESSION AY855339; SEQ ID NO: 14

1 agcgaaagca ggcaaaccat ttgaatggat gtcaatccga ctctactttt cttaaaggtg
61 ccagcgcaaa atgctataag cacaacattc ccttatactg gagatcctcc ctacagtcat
121 ggaacaggga caggatacac catggatact gtcaacagaa cacaccaata ttcagaaaag
181 gggaaatgga caacaaacac tgagattgga gcaccacaac ttaatccaat cgatggacca
241 cttcctgaag acaatgaacc aagtgggtac gcccaaacag attgtgtatt ggaagcaatg
301 gctttccttg aagaatccca tcccggaatc tttgaaaatt cgtgtcttga aacgatggag
361 gtgattcagc agacaagagt ggacaaacta acacaaggcc gacaaactta tgattggacc
421 ttgaatagga atcaacctgc cgcaacagca cttgctaata caattgaagt gttcagatca
481 aatggtctga cttccaatga atcagggagg ttgatggact tcctcaaaga tgtcatggag
541 tccatgaaca aggaagaaat ggaaataaca acacacttcc aacgaaagag aagagtaaga
601 gacaacatga caaagagaat ggtaacacag agaaccatag ggaagaaaaa acaacgatta
661 aacagaaaga gttatctaat cagaacatta accctaaaca caatgaccaa ggacgctgag
721 agagggaaat tgaaacgacg agcaatcgca accccaggga tgcagataag aggatttgta
781 tattttgttg aaacactagc ccgaagaata tgtgaaaagc ttgaacaatc aggattgcca
841 gttggcggta atgagaaaaa ggccaaactg gctaatgtcg tcagaaaaat gatgactaat
901 tcccaagaca ctgaactctc cttcaccatc actggggaca ataccaaatg gaatgaaaat
961 cagaacccac gcatattcct ggcaatgatc acatacataa ctagaaacca gccagaatgg
1021 ttcagaaatg ttctaagcat tgcaccgatt atgttctcaa ataaaatggc aagactgggg
1081 aaaggatata tgtttgaaag caaaagtatg aaactgagag ctcaaatacc agcagaaatg
1141 ctagcaagca ttgacctgaa atatttcaat gattcaacaa aaaagaaaat taaaaagata
1201 cgaccacttc tggttgacgg gactgcttca ctgagtcctg gcatgatgat gggaatgttc
1261 aacatgttga gcactgtgct gggtgtatcc atattaaacc tgggccagag gaaatacaca
1321 aagaccacat actggtggga tggtctgcaa tcatccgatg actttgcttt gatagtgaat
1381 gcgcctaatc atgaaggaat acaagctgga gtagacagat tctatagaac ttgcaaactg
1441 gtcgggatca acatgagcaa aaagaagtcc tacataaata gaaccggaac attcgaattc
1501 acaagctttt tctaccggta tggttttgta gccaatttca gcatggaact acccagtttt
1561 ggggtttccg gaataaatga atctgcagac atgagcattg gagtgacagt catcaaaaac
1621 aacatgataa ataatgatct cggtcctgcc acggcacaaa tggcactcca actcttcatt
1681 aaggattacc ggtacacata ccggtgccat agaggtgata cccagataca aaccagaaga
1741 tcttttgagt tgaagaaact gtgggaacag actcgatcaa agactggtct actggtatca
1801 gatgggggtc caaacctata taacatcaga aacctacaca tcccggaagt ttgtttaaaa
1861 tgggagctaa tggatgaaga ttataagggg aggctatgca atccattaaa tcctttcgtt
1921 agtcacaaag aaattgaatc agtcaacagt gcagtagtaa tgcctgcgca tggccctgcc
1981 aaaagcatgg agtatgatgc tgttgcaaca acacattctt ggatccccaa gaggaaccgg
2041 tccatattga acacaagcca aaggggaata ctcgaagatg agcagatgta tcagaaatgc
2101 tgcaacctgt ttgaaaaatt cttccccagc agctcataca gaagaccagt cggaatttct
2161 agtatggttg aggccatggt gtccagggcc cgcattgatg cacgaattga cttcgaatct
2221 ggacggataa agaaggatga gttcgctgag atcatgaaga tctgttccac cattgaagag
2281 ctcagacggc aaaaatagtg aatttagctt gatcttcatg aaaaaatgcc ttgtttctac
2341 t

DEFINITION Influenza A virus (A/equine/Kentucky/5/02(H3N8)) PB2 polymerase 2 gene, complete cds.
ACCESSION AY855338; SEQ ID NO: 15

  1 agcgaaagca ggtcaaatat attcaatatg gagagaataa aagaactgag agatctgatg
 61 ttacaatccc gcacccgcga gatactaaca aaaactactg tggaccacat ggccataatc
121 aagaaataca catcaggaag acaagagaag aaccctgcac ttaggatgaa atggatgatg
181 gcaatgaaat acccaattac agcagataag aggataatgg agatgattcc tgagagaaat
241 gaacagggac aaaccctttg gagcaaaacg aacgatgctg gctcagaccg cgtaatggta
301 tcacctctgg cagtgacatg gtggaatagg aatggaccaa caacgagcac aattcattat
361 ccaaaagtct acaaaactta ttttgaaaag gttgaaagat tgaaacacgg aacctttggc
421 cccgttcatt ttaggaatca agtcaagata agacgaagag ttgatgtaaa ccctggtcac
481 gcggacctca gtgccaaaga agcacaagat gtgatcatgg aagttgtttt cccaaatgaa

541 gtgggagcca gaattctaac atcggaatca caactaacaa taaccaaaga gaaaaaggaa
601 gaacttcagg actgcaaaat tgctcccttg atggtagcat acatgctaga aagagagttg
661 gtccgaaaaa caaggttcct cccagtggca ggcggaacaa gcagtgtata cattgaagtg
721 ttgcatctga ctcagggaac atgctgggag caaatgtaca ccccaggagg agaagttaga
781 aacgatgata ttgatcaaag tttaattatt gcagcccgga acatagtgag aagagcgaca
841 gtatcagcag atccactagc atccctactg gaaatgtgcc acagtacaca gattggtgga
901 ataaggatgg tagacatcct taagcagaat ccaacagagg aacaagctgt ggatatatgc
961 aaagcagcaa tgggattgag aattagctca tcattcagct ttggtggatt caccttcaaa
1021 agaacaagtg gatcatcagt caagagagaa gaagaaatgc ttacgggcaa ccttcaaaca
1081 ttgaaaataa gagtgcatga gggctatgaa gaattcacaa tggtcggaag aagagcaaca
1141 gccattctca gaaaggcaac cagaagattg attcaattga tagtaagtgg gagagatgaa
1201 caatcaattg ctgaagcaat aattgtagcc atggtgtttt cgcaagaaga ttgcatgata
1261 aaagcagttc gaggcgattt gaactttgtt aatagagcaa atcagcgttt gaaccccatg
1321 catcaactct tgaggcattt ccaaaaagat gcaaaagtgc ttttccaaaa ttggggaatt
1381 gaacccatcg acaatgtaat gggaatgatt ggaatattgc ctgacatgac cccaagcacc
1441 gagatgtcat tgagaggagt gagagtcagc aaaatgggag tggatgagta ctccagcact
1501 gagagagtgg tggtgagcat tgaccgtttt ttaagagttc gggatcaaag gggaaacata
1561 ctactgtccc ctgaagaagt cagtgaaaca caaggaacgg aaaagctgac aataatttat
1621 tcgtcatcaa tgatgtggga gattaatggt cccgaatcag tgttggtcaa tacttatcaa
1681 tggatcatca ggaactggga aattgtaaaa attcagtggt cacaggaccc cacaatgtta
1741 tacaataaga tagaatttga gccattccag tccctggtcc ctaggccacc cagaagccaa
1801 tacagcggtt tcgtaagaac cctgtttcag caaatgcgag atgtacttgg aacatttgat
1861 actgctcaaa taataaaact cctccctttt gccgctgctc ctccggaaca gagtaggatg
1921 cagttctctt ctttgactgt taatgtaaga ggatcgggaa tgaggatact tgtaagaggc
1981 aattccccag tgttcaacta caataaagcc actaaaaggc tcacagtcct cggaaaggat
2041 gcaggtgcgc ttactgagga cccagatgaa ggtacggctg gagtagaatc tgctgttcta
2101 agagggtttc tcattttagg taaagaaaac aagagatatg gcccagcact aagcatcaat
2161 gaactaagca aacttgcaaa aggggagaaa gccaatgtac taattgggca aggggacgta
2221 gtgttggtaa tgaaacggaa acgtgactct agcatactta ctgacagcca gacagcgacc
2281 aaaaggattc ggatggccat caattagtgt tgaattgttt aaaaacgacc ttgtttctac
2341 t

DEFINITION Influenza A virus (A/equine/Kentucky/1/91 (H3N8)) hemagglutinin precursor (HA) gene, complete cds.
ACCESSION L39918; SEQ ID NO: 16

1 agcaaaagca ggggatattt ctgtcaatca tgaagacaac cattattttg atactactga
61 cccattgggt ctacagtcaa aacccaacca gtggcaacaa cacagccaca ttatgtctgg
121 gacaccatgc agtagcaaat ggaacattgg taaaaacaat aactgatgac caaattgagg
181 tgacaaatgc tactgaatta gttcagagca tttcaatagg gaaaatatgc aacaactcat
241 atagagttct agatggaaga aattgcacat taatagatgc aatgctagga gacccccact
301 gtgatgtctt tcagtatgag aattgggacc tcttcataga aagaagcagc gctttcagca
361 gttgctaccc atatgacatc cctgactatg catcgctccg gtccattgta gcatcctcag
421 gaacattgga attcacagca gagggattca catggacagg tgtcactcaa aacggaagaa
481 gtggatcctg caaaagggga tcagccgata gtttctttag ccgactgaat tggctaacaa
541 aatctggaaa ctcttacccc acattgaatg tgacaatgcc taacaataaa aatttcgaca
601 aactatacat ctgggggatt catcacccga gctcaaacga agagcagaca aaattgtaca
661 tccaagaatc aggacgagta acagtctcaa caaaaagaag tcaacaaaca ataatcccta
721 acatcggatc tagaccgtgg gtcaggggtc aatcaggcag gataagcata tactggacca
781 ttgtaaaacc tggagatatc ctaatgataa acagtaatgg caacttagtt gcaccgcggg
841 gatattttaa attgaaaaca gggaaaagct ctgtaatgag atcagatgca cccatagaca
901 tttgtgtgtc tgaatgtatt acaccaaatg gaagcatccc caacgacaaa ccatttcaaa
961 atgtgaacaa agttacatat ggaaaatgcc ccaagtatat caggcaaaac actttaaagc
1021 tggccactgg gatgaggaat gtaccagaaa agcaaatcag aggaatcttt ggagcaatag
1081 cgggattcat agaaaacggc tgggaaggaa tggttgatgg gtggtatgga ttccgatatc
1141 aaaactcgga aggaacagga caagctgcag atctaaagag cactcaagca gccatcgacc
1201 agatcaatgg aaaattaaac agagtgattg aaaggaccaa tgagaaattc catcaaatag
1261 agaaggaatt ctcagaagta gaagggagaa tccaggattt ggagaagtat gtagaagaca
1321 ccaaaataga cctatggtcc tacaatgcag aattgctggt ggctctagaa aatcaacata
1381 caattgactt aacagatgca gaaatgaata aattattcga gaagactaga cgccagttaa

1441 gagaaaacgc ggaagacatg ggaggtggat gtttcaagat ataccacaaa tgtgataatg
1501 catgcattgg atcaataaga aatgggacat atgaccatta catatacaga gatgaagcat
1561 taaacaaccg gtttcaaatc aaaggtgttg agttgaaatc aggctacaaa gattggatac
1621 tgtggatttc attcgccata tcatgcttct taatttgcgt tgttctattg ggtttcatta
1681 tgtgggcttg ccaaaaaggc aacatcagat gcaacatttg catttgagta aactgatagt
1741 taaaaacacc cttgtttcta ct

DEFINITION Influenza A virus (A/equine/Kentucky/1/92(H3N8)) hemagglutinin precursor (HA) gene, complete cds.
ACCESSION L39917; SEQ ID NO: 17

1 agcaaaagca ggggatattt ctgtcaatca tgaagacaac cattattttg atactactga
61 cccattgggt ctacagtcaa aacccaacca gtggcaacaa cacagccaca ttatgtctgg
121 gacaccatgc agtagcaaat ggaacattgg taaaaacaat aactgatgac caaattgagg
181 tgacaaatgc tactgaatta gttcagagca tttcaatagg gaaaatatgc aacaactcat
241 atagggttct agatggaaga aattgcacat taatagatgc aatgctagga gacccccact
301 gtgatgtctt tcagtatgag aattgggacc tcttcataga aagaagcagc gctttcagca
361 attgctaccc atatgacatc cctgactatg catcgctccg gtccattgta gcatcctcag
421 gaacattaga attcacagca gagggattca catggacagg tgtcactcaa aacggaagaa
481 gtggagcctg caaaagggga tcagccgata gtttctttag ccgactgaat tggctaacaa
541 aatctggaaa ctcttacccc acattgaatg tgacaatgcc taacaataaa aatttcgaca
601 aactatacat ctgggggatt catcacccga gctcaaacaa tgagcagaca aaattgtata
661 tccaagaaac aggacgagta acagtctcaa caaaaagaag tcaacaaaca ataatcccta
721 acatcggatc tagaccgtgg gtcaggggtc aatcaggcag gataagcata tactggacca
781 ttgtaaaacc tggagatatc ctaatgataa acagcaatgg caacttagtt gcaccgcggg
841 gatattttaa attgagaaca gggagaagct ctgtaatgag atcagatgca cccatagaca
901 tttgtgtgtc tgaatgtatt acaccaaatg gaagcatccc caacgacaaa ccatttcaaa
961 atgtgaacaa agttacatat ggaaaatgcc ccaaatatat caggcaaaac actttaaagc
1021 tggccactgg gatgaggaat gtaccagaaa agcaaatcag aggaatcttt ggagcaatag
1081 cgggattcat agaaaacggc tgggaaggaa tggttgatgg gtggtatgga ttccgatatc
1141 aaaactcgga aggaacagga caagctgcag atctaaagag cactcaagca gccatcgacc
1201 agatcaatgg aaaattaaac agagtgattg aaaggaccaa tgagaaattc catcaaatag
1261 agaaggaatt ctcagaagta gaagggagaa tccaggattt ggagaagtat gtagaagaca
1321 ccaaaataga cctatggtcc tacaatgcag aattgctagt ggctctagaa aatcaacata
1381 caattgactt aacagacgca gaaatgaata aattattcga gaagactaga cgccagttaa
1441 gagaaaacgc ggaagacatg ggaggtggat gtttcaagat ttaccacaaa tgtgataatg
1501 catgcattgg atcaataaga aatgggacat atgaccatta catatacaga gatgaagcat
1561 taaacaaccg atttcaaatc aaaggtgttg aattaaaatc aggctacaaa gattggatac
1621 tgtggatttc attcgccata tcatgcttct taatttgtgt tgttctattg ggtttcatta
1681 tgtgggcttg ccaaaaaggc aacatcagat gcaacatttg catttgagta aactgatagt
1741 taaaaacacc cttgtttcta ct

DEFINITION Influenza A virus (A/equine/Kentticky/1/90(H3N8)) hemagglutinin precursor (HA) gene, complete cds.
ACCESSION L39915; SEQ ID NO: 18

1 agcaaaagca ggggatattt ctgtcaatca tgaaaacaac cattattttg atactactga
61 cccattgggt ctacagtcaa aacccaacca gtggcaacaa cacagccaca ttatgtctgg
121 gacaccatgc agtagcaaat ggaacattgg taaaaacaat aactgatgac caaattgagg
181 tgacaaatgc tactgaatta gttcagagca tttcaatagg aaaaatatgc aacaactcat
241 atagggttct agatggaaga aattgcacat taatagatgc aatgctagga gaccctcact
301 gtgatgtctt tcagtatgag aattgggacc tcttcataga aagaagcagc gctttcagca
361 attgctaccc atatgacatc cctgactatg catcgctccg gtccattgta gcatcctcag
421 gaacattaga attcacagca gagggattca catggacagg tgtcactcaa aacggaggaa
481 gtggagcctg caaaagagga tcagccgata gtttctttag ccgactgaat tggctaacaa
541 aatctggaaa ctcttacccc acattgaatg tgacaatgcc taacaataaa aatttcgaca
601 aactatacat ctgggggatt catcacccga gctcaaacaa tgagcagaca aaattgtata
661 tccaagaatc aggacgagta acagtctcaa caaaaagaag tcaacaaaca ataatcccta

721 acatcggatc tagaccgtgg gtcaggggtc aatcaggcag gataagcata tactggacca
781 ttgtaaaacc tggagatatc ctaatgataa acagtaatgg caacttagtt gcaccgcggg
841 gatattttaa attgagaaca gggaaaagct ctgtaatgag atcagatgca cccatagaca
901 cttgtgtgtc tgaatgtatt acaccaaatg gaagcatccc caacgacaaa ccatttcaaa
961 atgtgaacaa agttacatat ggaaaatgcc ccaagtatat caggcaaaac actttaaagc
1021 tggccactgg gatgaggaat gtaccagaaa agcaaatcag aggaatcttt ggagcaatag
1081 cgggattcat agaaaacggc tgggaaggaa tggttgatgg gtggtatgga ttccgatatc
1141 aaaactcgga aggaacagga caagctgcag atctaaagag cactcaagca gccatcgacc
1201 agatcaatgg aaaattaaac agagtgattg aaaggaccaa tgagaaattc catcaaatag
1261 agaaggaatt ctcagaagta gaagggagaa tcaaggactt ggagaagtat gtagaagaca
1321 ccaaaataga cctatggtcc tacaatgcag aattgctggt ggctctagaa aatcaacata
1381 caattgactt aacagatgca gaaatgaata aattattcga gaagactaga cgccagttaa
1441 gagaaaacgc ggaagacatg ggaggtggat gtttcaagat ctaccacaaa tgtgataatg
1501 catgcattgg atcaataaga aatgggacat atgaccatta catatacaaa gatgaagcat
1561 taaacaaccg atttcaaatc aaaggtgttg agttgaaatc aggctacaaa gattggatac
1621 tgtggatttc attcgccata tcatgcttct taatttgcgt tgttctattg ggtttcatta
1681 tgtgggcttg ccaaaaaggc aacatcagat gcaacatttg catttgagta aactgatagt
1741 taaaaacacc cttgtttcta ct

DEFINITION Influenza A virus (A/equine/Kentucky/1/94(H3N8)) hemagglutinin precursor (HA) gene, complete cds.
ACCESSION L39914; SEQ ID NO: 19

1 agcaaaagca ggggatattt ctgtcaatca tgaagacaac cattattttg atactactga
61 cccattgggt ctacagtcaa aacccaacca gtggcaacaa cacagccaca ttatgtctgg
121 gacaccatgc agtagcaaat ggaacattgg taaaaacaat aactgatgac caaattgagg
181 tgacaaatgc tactgaatta gttcagagca tttcaatagg gaaaatatgc aacaactcat
241 atagagttct agatggaaga aattgcacat taatagatgc aatgctagga gacccccact
301 gtgatgtctt tcagtatgag aattgggacc tcttcataga aagaagcagc gctttcagca
361 attgctaccc atatgacatc cctgactatg catcgctccg gtccattgta gcatcctcag
421 gaacattgga attcacagca gagggattca catggacagg tgtcactcaa aacggaagaa
481 gtggagcctg caaaagggga tcagccgata gtttctttag ccgactgaat tggctaacaa
541 aatctggaaa ctcttacccc acattgaatg tgacaatgcc taacaataaa aatttcgaca
601 aactatacat ctgggggatt catcacccga gctcaaccaa acagcaaaca gaattgtaca
661 tccaagaatc aggacgagta acagtctcaa caaaaagaag tcaacaaacg ataatcccta
721 atatcggatc tagaccgtgg gtcaggggtc aatcaggcag gataagcata tactggacca
781 ttgtaaaacc tggagatatc ctaatgataa acagtaatgg caacttagtt gcaccgcggg
841 gatattttaa attgaaaaca gggaaaagct ctgtaatgag atcagatgca cccatagaca
901 tttgtgtgtc tgaatgtatt acaccaaatg gaagcatccc caacgacaaa ccatttcaaa
961 atgtgaacaa agttacatat ggaaaatgcc ccaagtatat caggcaaaac actttaaagc
1021 tggccactgg gatgaggaat gtaccagaaa agcaaatcag aggaatcttt ggagcaatag
1081 cgggattcat agaaaacggc tgggaaggaa tggttgatgg gtggtatgga ttccgatatc
1141 aaaactcgga aggaacagga caagctgcag atctaaagag cactcaagca gccatcgacc
1201 agattaatgg aaaattaaac agagtgattg aaaggaccaa tgagaaattc catcaaatag
1261 agaaggaatt ctcagaagta gaagggagaa tccaggactt ggagaagtat gtagaagaca
1321 ccaaaataga cctatggtcc tacaatgcag aattgctggt ggctctagaa aatcaacata
1381 caattgactt aacagatgca gaaatgaata aattattcga gaagactaga cgccagttaa
1441 gagaaaacgc ggaagacatg ggaggtggat gtttcaagat ttaccacaaa tgtgataatg
1501 catgcattgg atcaataaga aatgggacat atgaccatta catatacaga gatgaagcat
1561 taaacaaccg atttcaaatc aaaggtgttg agttgaaatc aggctacaaa gattggatac
1621 tgtggatttc attcgccata tcatgcttct taatttgcgt tgttctattg ggtttcatta
1681 tgtgggcttg ccaaaaaggc aacatcagat gcaacatttg catttgagta aactgatagt
1741 taaaaacacc cttgtttcta ct

DEFINITION Influenza A virus (A/equine/Kentucky/1/81(H3N8)) nucleoprotein (NP) gene, complete cds.
ACCESSION AY291288; SEQ ID NO: 20

1 agcaaaagca gggtagataa tcactcactg agtgacatca aagtcatggc gtctcaaggc
61 accaaacgat cttatgagca gatggaaact ggtggggaac gccagaatgc aactgaaatc
121 agagcatctg ttggaaggat ggtgggagga atcggccggt tctatgttca aatgtgtact
181 gagcttaaac tcaacgacca tgaagggcgg ctgattcaga acagcataac aatagaaagg
241 atggtacttt cggcattcga cgaaagaaga aacaagtacc tcgaggagca tcccagtgct
301 gggaaagacc ccaagaaaac gggaggcccg atatacagaa ggagagatgg gaaatggatg
361 agagaactca tcctccatga taaagaagaa atcatgagga tctggcgtca ggccaacaat
421 ggtgaagacg ctactgctgg tcttactcat atgatgatct ggcactccaa tctcaatgac
481 accacctacc aaagaacaag ggctcttgtt cgggctggga tggatcccag aatgtgctct
541 ctgatgcaag gatcaactct cccacggaga tctggagctg ccggtgctgc agtgaagggt
601 gttggaacaa tggtaatgga actcatcagg atgatcaaac gcgggataaa tgatcgaaac
661 ttctggagag gtgaaaatgg tcgaagaacc agaattgctt atgaaagaat gtgcaacatc
721 ctcaagggga aattccaaac agcagcacaa cgagcaatga tggaccaagt gagggagggc
781 cgcaatcctg gaaatgctga gattgaggat ctcattttct tggcacgatc agcactcatt
841 ctgagaggat cagtagccca taaatcatgc ctacctgcct gtgtttatgg ccttgcagta
901 gccagtgggt atgactttga gaaagaggga tactctctgg ttggaattga tcctttcaaa
961 ctactccaga acagccaaat tttcagtcta atcagaccga aagaaaatcc agcacacaag
1021 agccagctgg tgtggatggc atgccattct gcagcatttg aggacctgag agtttcgaat
1081 ttcattagag gaaccaaagt aatcccaaga ggacagttag caaccagggg agtgcaaatt
1141 gcttcaaatg aaaacatgga gacaatagat tctagcacac tcgaactgag gagcagatat
1201 tgggcaataa ggaccaggag tgggggaaac accagtcaac agagagcatc tgcaggacag
1261 ataagtgtgc aacccacttt ctcagtgcag agaaatcttc cctttgaaag agcaaccatt
1321 atggctgcat tcactggaaa cactgagggg aggacttccg acatgagaac ggaaatcata
1381 aggatgatgg aaaatgccag atcagaagat gtgtctttcc aggggcgggg agtcttcgag
1441 ctctcggacg aaaaggcaac gaacccgatc gtgccttcct ttgacatgag caatgaaggg
1501 tcttatttct tcggagacaa tgctgaggag tttgacagtt aaagaaaaat acccttgttt
1561 ctact

DEFINITION Influenza A virus (A/Equine/Kentucky/1/92(H3N8)) gene for hemagglutinin precursor, partial cds.
ACCESSION D30683; SEQ ID NO: 21

1 gtcaatcatg aagacaacca ttattttgat actactgacc cattgggtct acagtcaaaa
61 cccaaccagt ggcaacaaca cagccacatt atgtctggga caccatgcag tagcaaatgg
121 aacattggta aaaacaataa ctgatgacca aattgaggtg acaaatgcta ctgaattagt
181 tcagagcatt tcaataggga aaatatgcaa caactcatat agggttctag atggaagaaa
241 ttgcacatta atagatgcaa tgctaggaga cccccactgt gatgtctttc agtatgagaa
301 ttgggacctc ttcatagaaa gaagcagcgc tttcagcaat tgctacccat atgacatccc
361 tgactatgca tcgctccggt ccattgtagc atcctcagga acattagaat tcacagcaga
421 gggattcaca tggacaggtg tcactcaaaa cggaggaagt ggagcctgca aaggggatc
481 agccgatagt ttctttagcc gactgaattg gctaacaaaa tctggaaact cttaccccac
541 attgaatgtg acaatgccta acaataaaaa tttcgacaaa ctatacatct gggggattca
601 tcacccgagc tcaaacaatg agcagacaaa attgtatatc caagaaacag gacgagtaac
661 agtctcaaca aaaagaagtc aacaaacaat aatccctaac atcggatcta gaccgtgggt
721 caggggtcaa tcaggcagga taagcatata ctggaccatt gtaaaacctg gagatatcct
781 aatgataaac agcaatggca acttagttgc accgcgggga tattttaaat tgagaacagg
841 gagaagctct gtaatgagat cagatgcacc catagacatt tgtgtgtctg aatgtattac
901 accaaatgga agcatcccca acgacaaacc atttcaaaat gtgaacaaag ttacatatgg
961 aaaatgcccc aaatatatca ggcaaaacac tttaaagctg gccactggga tgaggaatgt
1021 accagaaaag caaatcagag gaatctttgg agcaatagcg ggattcatag aaaacggctg
1081 ggaaggaatg gttgat

DEFINITION Influenza A virus (A/equine/Kentucky/1/97(H3N8)) hemagglutinin precursor (HA1) mRNA, partial cds.
ACCESSION AF197249; SEQ ID NO: 22

1 agcaaaagca ggggatattt ctgtcaatca tgaagacaac cattattttg atactactga
61 cccattgggc ctacagtcaa aacccaatca gtggcaacaa cacagccaca ttgtgtctgg

121 gacaccatgc agtagcaaat ggaacattgg taaaaacaat aactgatgat caaattgagg
181 tgacaaatgc tactgaatta gttcagagca tttcaatggg gaaaatatgc aacaactcat
241 atagagttct agatggaaga aattgcacat taatagatgc aatgctagga gacccccact
301 gtgatgtctt tcagtatgag aattgggacc tctttataga aagaagcagc gctttcagca
361 attgctaccc atatgacatc cctgactatg catcgctccg gtccattgta gcatcctcag
421 gaacattgga attcacagca gagggattca catggacagg tgtcactcaa aacggaagaa
481 gtggagcctg caaaagggga tcagccgata gtttctttag ccgactgaat tggctaacaa
541 aatctggaaa ctcttacccc acattgaatg tgacaatgcc taacaataaa aatttcgaca
601 aactatacat ctgggggatt catcacccga gctcaaacca agagcagaca aaattgtaca
661 tccaagaatc aggacgagta acagtctcaa caaaaagaag tcaacaaaca ataatcccta
721 acatcggatc tagaccgtgg gtcaggggtc aatcaggcag gataagcata tactggacca
781 ttgtaaaacc tggagatatc ctaatgataa acagtaatgg caacttagtt gcaccgcggg
841 gatattttaa attgaaaaca gggaaaagct ctgtaatgag atcagatgca cccatagaca
901 tttgtgtgtc tgaatgtatt acaccaaatg gaagcatctc caacgacaaa ccattccaaa
961 atgtgaacaa agttacatat ggaaaatgcc ccaagtatat caggcaaaac actttaaagc
1021 tggccactgg gatgaggaat gaaccagaaa agcaaatcag a

DEFINITION Influenza A virus (A/equine/Kentucky/1/96(H3N8)) hemagglutinin precursor (HA1) mRNA, partial cds.
ACCESSION AF197248; SEQ ID NO: 23

1 agcaaaagca ggggatattt ctgtcaatca tgaagacaac cattattttg atactactga
61 cccattgggt ctacagtcaa aacccaacca gtggcaacaa cacagccaca ttatgtctgg
121 gacaccatgc agtagcaaat ggaacattgg taaaaacaat aactgatgac caaattgagg
181 tgacaaatgc tactgaatta gttcagagca tttcaatagg gaaaatatgc aacaactcat
241 atagagttct agatggaaga aattgcacat taatagatgc aatgctagga gacccccact
301 gtgatgtctt ccagtatgag aattgggacc tcttcataga aagaagcagc gctttcagca
361 attgctaccc atatgacatc cctgactatg catcgctccg gtccattgta gcatcctcag
421 gaacattaga attcacagca gagggattca catggacagg tgtcactcaa aacggaagaa
481 gtggagcctg caaaagggga tcagccgata gtttctttag ccgactgaat tggctaacaa
541 aatctggaaa ctcttacccc acattgaatg tgacaatgcc taacaataaa aatttcgaca
601 aactatacat ctgggggatt catcacccga gctcaaacca aaagcagaca gaattgtaca
661 tccaagaatc aggacgagta acagtctcaa caaaaagaag tcaacaaacg ataatcccta
721 atatcggatc tagaccgtgg gttaggggtc aatcaggcag gataagcata tactggacca
781 ttgtaaaacc tggagatatc ctaatgataa acagtaatgg caacttagtt gcaccgcggg
841 gatattttaa attgaaaaca gggaaaagct ctgtaatgag atcagatgca cccatagaca
901 tttgtgtgtc tgaatgtatt acaccaaatg gaagcatccc caacgacaaa ccatttcaaa
961 atgtgaacaa agttacatat ggaaaatgcc ccaagtatat caggcaaaac actttaaagc
1021 tggccactgg gatgaggaat gtaccagaaa agcaaatcag a

DEFINITION Influenza A virus (A/equine/Kentucky/9/95(H3N8)) hemagglutinin precursor (HA1) mRNA, partial cds.
ACCESSION AF197247; SEQ ID NO: 24

1 agcaaaagca ggggatattt ctgtcaatca tgaagacaac cattattttg atactactga
61 cccattgggt ctacagtcaa aacccaacca gtggaaacaa cacagccaca ttatgtctgg
121 gacaccatgc agtagcaaat ggaacattgg taaaaacaat aactgatgac caaattgagg
181 tgacaaatgc tactgaatta gttcagagca tttcaatagg gaaaatatgc aacaactcat
241 atagagttct agatggaaga aattgcacat taatagatgc aatgctagga gacccccact
301 gtgatgtctt ccagtatgag aattgggacc tcttcataga aagaagcagc gctttcagca
361 attgctaccc atatgacatc cctgactatg catcgctccg gtccattgta gcatcctcag
421 gaacattgga attcacagca gagggattca catggacagg tgtcactcaa aacggaagaa
481 gtggagcctg caaaagggga tcagccgata gtttctttag ccgactgaat tggctaacaa
541 aatctggaaa ctcttacccc acattgaatg tgacaatgcc taacaataaa aatttcgaca
601 aactatacat ctgggggatt catcacccga gctcaaacca aaagcagaca gaattgtaca
661 tccaagaatc aggacgagta acagtctcaa caaaaagaag tcaacaaacg ataatcccta
721 atatcggatc tagaccgtgg gtcaggggtc aatcaggcag gataagcata tactggacca
781 ttgtaaaacc tggagatatc ctaatgataa acagtaatgg caacttagtt gcaccgcggg

841 gatattttaa attgaaaaca gggaaaagct ctgtaatgag atcagatgca cccatagaca
901 tttgtgtgtc tgaatgtatt acaccaaatg gaagcatccc caacgacaaa ccatttcaaa
961 atgtgaacaa agttacatat ggaaaatgcc ccaagtatat caggcaaaac actttaaagc
1021 tggccactgg gatgaggaat gtaccagaaa agcaaatcag a

DEFINITION Influenza A virus (A/equine/Kentucky/1/98(H3N8)) hemagglutinin precursor (HA1) mRNA, partial cds.
ACCESSION AF197241; SEQ ID NO: 25

   1 agcaaaagca ggggatattt ctgtcaatca tgaagacaac cattattttg atactactga
  61 cccattgggt ctacagtcaa aacccaacca gtggaacaac cacagccaca ttatgtctgg
 121 gacaccatgc agtagcaaat ggaacattgg taaaaacaat aactgatgac caaattgagg
 181 tgacaaatgc tactgaatta gttcagagca tttcaatagg gaaaatatgc aacaactcat
 241 ataaagttct agatggaaga aattgcacat taatagatgc aatgctagga gacccccact
 301 gtgatgtctt ccagtatgag aattgggacc tcttcataga aagaagcagc gctttcagca
 361 attgctaccc atatgacatc cctgactatg catcgctccg gtccattgta gcatcctcag
 421 gaacattaga attcacagca gagggattca catggacagg tgtcactcaa aacggaagaa
 481 gtggagcctg caaaagggga tcagccgata gtttctttag ccgactgaat tggctaacaa
 541 aatctggaaa ctcttacccc acattgaatg tgacaatgcc taacaataaa aatttcgaca
 601 aactatacat ctgggggatt catcacccga gctcaaccaa acagcagaca gaattgtaca
 661 tccaagaatc aggacgagta acagtctcaa caaaaagaag tcaacaaacg atagtcccta
 721 atatcggatc tagaccgtgg gttaggggtc aatcaggcag gataagcata tactggacca
 781 ttgtaaaacc tggagatatc ctaatgataa acagtaatgg caacttagtt gcaccgcggg
 841 gatattttaa attgaaaaca gggaaaagct ctgtaatgag atcagatgca cccatagaca
 901 tttgtgtgtc tgaatgtatt acaccaaatg gaagcatccc caacgacaaa ccatttcaaa
 961 atgtgaacaa agttacatat ggaaaatgcc ccaagtatat caggcaaaac actttaaagc
1021 tggccactgg gatgaggaat ataccagaaa agcaaatcag a

DEFINITION Influenza A virus (A/eq/Kentucky/81(H3N8)) hemagglutinin mRNA, complete cds.
ACCESSION U58195; SEQ ID NO: 26

1 agcaaaagca ggggatactt tctgtcaatc atgaagacaa ccattatttt gatactactg
61 acccattggg tctacagtca aaacccaacc agtggcaaca acacagccac actatgtctg
121 ggacaccatg cagtagcaaa tggaacattg gtaaaaacaa taactgatga ccaaattgag
181 gtgacaaatg ctactgaatt agttcagagc acttcaatag ggaaaatatg caacaaccca
241 tatagggttc tagatggaag aaactgcaca ttaatagatg caatgctagg agatccccac
301 tgtgatgttt ttcagtatga gaattgggac ctcttcatag aaagaagcag cgctttcagc
361 aattgctacc catatgacat ccctgactat gcatcgctcc ggtctattgt ggcatcttca
421 ggaacattag aattcacagc agagggattc acatggacag gtgtcactca aaacggagga
481 agtggagcct gcagaagggg gtcagccgat agtttcttta gccgactgaa ttggctaaca
541 aaatctggaa attcttaccc cacattgaat gtaacaatgc ctaacaataa caatttcgat
601 aaactataca tctgggggat ccatcacccg agcacaaaca atgagcagac aaaattgtat
661 atccaagaat cagggcgagt aacagtctca acaaaaagaa gtcaacaaac aataatcccc
721 aacatcggat ctagaccgtg ggtcaggggt caatcaggca ggataagcat atattggacc
781 attgtgaaac ctggagatat cctaatgata aacagtaatg gcaacttagt tgcaccgcgg
841 ggatatttta aaatgcgaac agggaaaagc tctgtaatga gatcagatgc acccatagac
901 acttgtgtgt ccgagtgtat tacaccaaat ggaagcatcc ccaacgacaa accatttcaa
961 aatgtgaaca aagttacata tggaaaatgc cccaagtata tcaagcagaa tactttgaag
1021 ctggccactg ggatgaggaa tgtaccagaa aagcaaatca gaggaatctt tggagcaata
1081 gcgggattca tagaaaacgg ctgggaagga atggttgatg ggtggtatgg attccgatat
1141 cagaattcgg aaggaacagg acaagctgca gatctaaaga gcactcaagc agccatcgac
1201 cagatcaatg gaaaattgaa cagagtgatt gaaaggacca atgagaaatt ccatcaaata
1261 gagaaggaat tctcagaagt agaagggaga atccaggact tggagaagta tgtagaagac
1321 accaaaatag acctatggtc ctacaatgca gagttactgg tggctctaga aaatcaacat
1381 acgattgact taacagatgc agaaatgaat aaattattcg agaagactag gcgccagtta
1441 agagaaaacg cggaagacat ggggggtgga tgtttcaaga tttatcacaa atgtgataat
1501 gcatgcattg gatcaataag aaatgggaca tatgaccatt acatatacag agatgaagca

1561 ttaaacaacc gatttcaaat taaaggtgtt gaattgaaat caggctacaa agattggata
1621 ctgtggattt cattcgccat atcatgcttc ttaatttgcg ttgttctatt gggtttcatc
1681 atgtgggctt gccaaaaagg caacatcaga tgcaacattt gcatttgagt aaactgataa
1741 ttaaaaacac ccttgtttct act

DEFINITION Influenza A virus (A/equine/Kentucky/2/86 (H3N8)) membrane protein M1 and membrane protein M2 genes, complete cds.
ACCESSION M63540; SEQ ID NO: 27

1 agcaaaagca ggtagatatt taaagatgag tcttctaacc gaggtcgaaa cgtacgttct
61 ctctattgta ccatcaggcc ccctcaaagc cgagatcgcg cagagacttg aagatgtctt
121 tgcagggaag aacaccgatc ttgaggcact catggaatgg ctaaagacaa gaccaatcct
181 gtcacctctg actaaaggga ttttaggatt tgtgttcacg ctcaccgtgc ccagtgagcg
241 aggactgcaa cgtagacgct ttgtccaaaa tgcccttagt ggaaacggag atccaaataa
301 catggacaga gcagtaaaac tgtacaagaa gcttaaaaga gaaataacat tccatggggc
361 aaaagaggtg gcactcagct attccactgg tgcactagcc agctgcatgg gactcatata
421 caacagaatg gggactgtga caaccgaagt ggcatttggc ctggtatgcg ccacatgtga
481 acagattgct gattcccagc atcgatctca caggcagatg gtgacaacaa ccaacccact
541 aatcagacat gaaaacagaa tggtactagc cagtaccaca gctaaaacca tggagcaggt
601 ggcagggtcg agtgagcagg cagcagaggc catggaggtt gctagtaagg ccaggcagat
661 ggtgcaggca atgaggacca ttgggaccca ccctagctcc agtgccggtt tgaaagatga
721 tcttcttgaa aatttgcagg cctaccagaa acggatggga gtgcaaatgc agcggttcaa
781 gtgatcctct cgttattgca gcaagtatca ttgggatctt gcacttgata ttgtggattc
841 ttgatcgcct tttcttcaaa ttcatttatc gtctccttaa atacggtttg aaaagagggc
901 cttctacgga aggagtacct gagtctatga gggaagaata tcggcaggaa cagcagaatg
961 ctgtggatgt tgacgatggt cattttgtca acatagagct ggagtaaaaa actaccttgt
1021 ttctact

DEFINITION Influenza A virus isolate A/equine/Kentucky/76 nonstructural protein, complete cds.
ACCESSION M80971; SEQ ID NO: 28

1 agcaaaagca gggtgacaaa aacataatgg attccaacac tgtgtcaagc tttcaggtag
61 actgttttct ttggcatgtc cgcaaacgat ttgcagacca agaactgggt gatgccccat
121 tccttgaccg gcttcgccga gaccagaagt ccctaaaagg aagaggcagc actcttggtc
181 tggacatcga aacagccact cgtgcaggaa agcagatagt ggagcggatt ctggaagagg
241 agtcagatga ggcacttaaa atgaccattg cctctgttcc tgcttcacgc tacttaactg
301 acatgactct tgatgagatg tcaagagact ggttcatgct catgcccaag cagaaagtaa
361 caggctccct atgtataagg atggaccagg caatcatgga taagaacatc atactaaaag
421 caaactttag tgtgattttc gaaaggctgg agacactaat actacttaga gctttcaccg
481 aagaaggagc agtcgttggc gaaatttcac cattgccttc tcttccagga catactaatg
541 aggatgtcaa aaatgcaatt ggggtcctca tcggaggact taaatggaat gataacacag
601 ttagaatctc tgaaactcta cagagattcg cttggagaag cagtcatgag aatgggagac
661 cttcattccc tccaaagcag aaacgaaaaa tggcgagaac aattgagtca gaagtttgaa
721 gaaataaggt ggttgattga agaagtgcga catagattga aaaatacaga aaatagtttt
781 gaacaaataa catttatgca agccttacaa ctattgcttg aagtagaaca agagataaga
841 actttctcgt ttcagcttat ttaatgataa aaaacaccct tgtttctact

DEFINITION Influenza A virus (A/eq/Kentucky/92(H3N8)) matrix proteins M1 and M2 (M) gene, complete cds.
ACCESSION AF001683; SEQ ID NO: 29

1 atgagtcttc tgaccgaggt cgaaacgtac gttctctcta tcgtaccatc aggcccctc
61 aaagccgaga tcgcgcagag acttgaagat gtctttgcag ggaagaacac cgatcttgag
121 gcactcatgg aatggctaaa gacaagacca atcctgtcac ctctgactaa agggatttta
181 ggattcgtat tcacgctcac cgtgcccagt gagcgaggac tgcagcgtag acgctttgtc
241 caaaatgccc ttagtggaaa cggagatcca aacaacatgg acagagcagt aaaactgtac

301 aggaaactta aaagagaaat aacattccat ggggcaaaag aggtggcact cagctattcc
361 actggtgcac tagccagctg catgggactc atatacaaca gaatgggaac tgtgacaacc
421 gaagtggcat ttggcctagt atgcgccaca tgtgaacaga ttgctgattc ccagcatcga
481 tctcacaggc agatggtgac aacaaccaac ccattaatca gacatgaaaa cagaatggta
541 ttagccagta ccacggctaa agccatggag cagatggcag ggtcgagtga gcaggcagca
601 gaggccatgg aggttgctag taaggctagg cagatggtac aggcaatgag gaccattggg
661 acccacccta gctccagtgc cggtttgaaa aatgatctcc ttgaaaattt gcaggcctac
721 cagaaacgga tgggagtgca aatgcagcga ttcaagtgat cctctcgtta ttgcagcaag
781 tatcattggg atcttgcact tgatattgtg gattcttgat cgccttttct tcaaattcat
841 ttatcgtcgc cttaaatacg ggttgaaaag agggccttct acggaaggag tacctgagtc
901 tatgagggaa gaatatcggc aggaacagca gaatgctgtg gatgttgacg atggtcattt
961 tgtcaacata gagctggagt aa

DEFINITION Influenza A virus (A/eq/Kentucky/81(H3N8)) matrix proteins M1 and M2 (M) gene, complete cds.
ACCESSION AF001676; SEQ ID NO: 30

1 atgagtcttc taaccgaggt cgaaacgtac gttctctcta tcgtaccatc aggcccctc
61 aaagccgaga tcgcgcagag acttgaagat gtctttgcag ggaagaacac cgatcttgag
121 gcactcatgg aatggctaaa gacaagacca atcctgtcac ctctgactaa agggatttta
181 ggatttgtgt tcacgctcac cgtgcccagt gagcgaggac tgcagcgtag acgctttgtc
241 caaaatgccc ttagtggaaa cggagatcca aacaacatgg acagagcagt aaaactgtac
301 aggaagctta aaagagaaat aacattccat ggggcaaaag aggtggcact cagctattcc
361 actggtgcac tagccagctg catgggactc atatacaaca gaatggggac tgtgacaacc
421 gaagtggcat ttggcctggt atgcgccaca tgtgaacaga ttgctgattc ccagcatcga
481 tctcacaggc agatggtgac aacaaccaac ccactaatca gacatgaaaa cagaatggta
541 ctagccagta ccacagctaa agccatggaa cagatggcag ggtcgagtga gcaggcagca
601 gaggccatgg aggttgctag taaggccagg cagatggtac aggcaatgag gaccattggg
661 acccacccta gctccagtgc cggtttgaaa gatgatcttc ttgaaaattt gcaggcctac
721 cagaaacgga tgggagtgca aatgcagcga ttcaagtgac cctctcgtta ttgcagcaag
781 tatcattggg atcttgcact tgatattgtg gattcttgat cgccttttct tcaaattcat
841 ttatcgtcgc cttaaatacg gtttgaaaag agggccttct acggaaggag tacctgagtc
901 tatgagggaa gaatatcggc aggaacagca gaatgctgtg gatgttgacg atggtcattt
961 tgtcaacata gagctggagt aa

DEFINITION Influenza A virus (A/eq/Kentucky/92(H3N8)) nonstructural proteins NS1 and NS2 (NS) gene, complete cds.
ACCESSION AF001671; SEQ ID NO: 31

1 atggattcca acactgtgtc aagctttcag gtagactgtt ttctttggca tgtccgcaaa
61 cgattcgcag accaagaact gggtgatgcc ccattccttg accggcttcg ccgagaccag
121 aagtccctaa aaggaagagg tagcactctt ggtctggaca tcgaaacagc cactcgtgca
181 ggaaagcaga tagtggagca gattctggaa gaggaatcag atgaggcact taaaatgacc
241 attgcctctg ttcctgcttc acgctactta actgacatga ctcttgatga gatgtcaaga
301 gactggttca tgctcatgcc caagcagaaa gtaacaggct ccctatgtat aagaatggac
361 caggcaatca tggataagaa catcatactt aaagcaaact ttagtgtgat tttcgaaagg
421 ctggaaacac taatactact tagagccttc accgaagaag gagcagtcgt tggcgaaatt
481 tcaccattgc cttctcttcc aggacatact aatgaggatg tcaaaaatgc aattggggtc
541 ctcatcggag gacttaaatg gaatgataat acggttagaa tctctgaaac tctacagaga
601 ttcgcttgga gaagcagtca tgagaatggg agaccttcat tccctccaaa gcagaaacga
661 aaaatggaga gaacaattga gccagaagtt tgaagaaata agatggttga ttgaagaagt
721 gcgacataga ttgaaaaata cagaaaatag ttttgaacaa ataacattta tgcaagcctt
781 acaactattg cttgaagtag aacaagagat aagaactttc tcgtttcagc ttatttaa

DEFINITION Influenza A virus (A/eq/Kentucky/1/88(H3N8)) nonstructural proteins NS 1 and NS2 (NS) gene, complete cds.
ACCESSION AF001664; ; SEQ ID NO: 32

1 atggattcca acactgtgtc aagctttcag gtagactgtt ttctttggca tgtccgcaaa
61 cgatttgcag accaagaact gggtgatgcc ccattccttg accggcttcg ccgagaccag
121 aagtccctaa aaggaagagg cagcactctt ggtctggaca tcgaaacagc cactcgtgca
181 ggaaagcaga tagtggagca gattctggaa gaggaatcag atgaggcact taaaatgacc
241 attgcctctg ttcctgcttc acgctactta actgacatga ctcttgatga gatgtcaaga
301 gactggttca tgctcatgcc caagcagaaa gtaacaggct ccctatgtat aaggatggac
361 caggcaatca tggataagaa catcatacta aaagcaaact ttagtgtgat tttcgaaagg
421 ctggagacac taatactact tagagccttc accgaagaag gagcagtcgt tggcgaaatt
481 tcaccattgc cttctcttcc aggacatact aatgaggatg tcaaaaatgc aattggggtc
541 ctcatcggag gacttaaatg gaatgataat acagttagag tctctgaaac tctacagaga
601 ttcgcttgga gaagcagtca tgagaatggg agaccttcat tccctccaaa gcagaaacga
661 aaaatggcga gaacaattga gccagaagtt tgaagaaata agatggttga ttgaagaagt
721 gcgacataga ttgaaaaata cagaaaatag ttttgaacaa ataacattta tgcaagcctt
781 acaactattg cttgaagtag aacaagagat aagaactttc tcgtttcagc ttatttaa

DEFINITION Influenza A/equine/Kentucky/2/86 (H3N8) nucleoprotein (seg 5) mRNA, complete cds.
ACCESSION M30751; SEQ ID NO: 33

1 agcaaaagca gggtagataa tcactcactg agtgacatca aagtcatggc gtctcaaggc
61 accaaacgat cttatgagca gatggaaact ggtggggaac gccagaatgc aactgaaatc
121 agagcatctg tcggaaggat ggtgggagga atcggccggt tctatgttca gatgtgtact
181 gagcttaaac tcaacgacca tgaagggcgg ctgattcaga acagcataac aatagaaagg
241 atggtacttt cggcattcga cgaaagaaga aacaagtacc tcgaggagca tcccagtgct
301 gggaaagacc ccaagaaaac gggaggcccg atatacagaa ggaaagatgg gaaatggatg
361 agagaactca tcctccatga taaagaagaa atcatgagga tctggcgtca ggccaacaat
421 ggtgaagacg ctactgctgg tcttactcat atgatgatct ggcactccaa tctcaatgac
481 accacatacc aaagaacaag ggctcttgtt cgggctggga tggatcccag aatgtgctct
541 ctgatgcaag gatcaaccct cccacggaga tctggagctg ccggtgctgc agtaaaaggt
601 gttggaacaa tggtaatgga actcatcagg atgatcaaac gcgggataaa tgatcgaaat
661 ttctggagag gtgaaaatgg tcgaagaacc agaattgctt atgaaagaat gtgcaatatc
721 ctcaaaggga aattccaaac agcagcacaa cgggcaatga tggaccaagt gagggagggc
781 cgcaatcctg gaaatgctga gattgaggat ctcattttct tggcacgatc agcactcatt
841 ttgagaggat cagtagccca taaatcatgc ctacctgcct gtgtttatgg ccttgcagta
901 gccagtgggt atgactttga gaaggaagga tactctctgg ttggaattga tcctttcaaa
961 ctactccaga acagccaaat tttcagtcta atcagaccga aagaaaatcc agcacacaag
1021 agccagttgg tgtggatggc atgccattct gcagcatttg aggacctgag agttttgaat
1081 ttcattagag gaaccaaagt aatcccaaga ggacagttag caaccagagg agtgcaaatt
1141 gcttcaaatg aaaacatgga gacaatagat tctagcacac tcgaactgag gagcagatat
1201 tgggcaataa ggaccaggag tggaggaaac accagtcaac agagagcatc tgcaggacag
1261 ataagtgtgc aacccacttt ctcagtgcag agaaatcttc cctttgaaag agcaaccatt
1321 atggctgcat tcactgggaa cactgagcgg aggacttccg acatgagaac ggaaatcata
1381 aggatgatgg aaaatgccag atcagaagat gtgtctttcc aggggcgggg agtcttcgag
1441 ctctcggacg aaaaggcaac gaacccgatc gtgccttcct ttgacatgag caatgaaggg
1501 tcttatttct tcggagacaa tgctgaggag tttgacagtt aaagaaaaat acccttgttt
1561 ctact

DEFINITION Influenza A/Equine/Kentucky/2/86 (H3N8), PB2 polymerase, complete cds.
ACCESSION M73526 M36049; SEQ ID NO: 34

1 agcaaaagca ggtcaaatat attcaatatg gagagaataa aagaactgag agatctaatg
61 tcacagtccc gcacccgcga gatactaaca aaaactactg tggaccatat ggccataatc
121 aagaaataca catcaggaag acaagagaag aaccccgcac ttaggatgaa gtggatgatg
181 gcaatgaaat acccaattac agcagataag aggataatgg aaatgattcc tgagagaaat
241 gaacaggggc aaaccctttg gagcaaaacg aacgatgctg gctcagaccg cgtaatggta
301 tcacctctgg cagtgacatg gtggaatagg aatggaccaa caacgagcac aattcattat
361 ccaaaagtct acaaaactta ttttgaaaaa gttgaaaggt taaaacacgg aacctttggc

421 cccgttcatt ttaggaatca agtcaagata agacggagag ttgacgtaaa ccctggtcac
481 gcggacctca gtgccaaaga agcacaagat gtgatcatgg aagttgtttt cccaaatgaa
541 gtgggagcca gaattctaac atcggaatca caactaacaa taaccaaaga gaaaaaagaa
601 gaacttcagg actgcaaaat tgcccccttg atggtagcat acatgctaga aagagagttg
661 gtccgaaaaa caaggttcct cccagtggct ggcggaacaa gcagtgtata cattgaggtg
721 ttgcatctga ctcagggaac gtgctgggaa caaatgtaca ccccaggagg agaagttaga
781 aacgatgaca ttgatcaaag tttaattatt gctgcccgga acatagtgaa aagagcgaca
841 gtatcagcag atccactagc atccctgctg gagatgtgcc acagtacaca gattggtgga
901 ataaggatgg tagacatcct taagcagaat ccaacagagg aacaagctgt ggatatatgc
961 aaagcagcaa tggggttaag aattagctca tcattcagct ttggtggatt cacctttaag
1021 agaacaagtg gatcatcagt caagagagaa gaagaaatgc ttacgggcaa ccttcaaaca
1081 ttgaaaataa gagtgcatga gggctatgaa gaattcacaa tggtcggaag aagagcaaca
1141 gccattctca gaaagacaac cagaagattg attcaattga tagtaagtgg gagagatgaa
1201 cagtcaattg ctgaagcaat aattgtagcc atggtgtttt cgcaagaaga ttgcatgata
1261 aaagcagttc gaggcgattt gaacttcgtt aatagagcaa atcagcgctt gaaccccatg
1321 catcaactct tgaggcattt ccaaaaggat gcaaaagtgc ttttccagaa ttgggggatt
1381 gaacccatcg acaatgtgat gggaatgatc ggaatattgc ccgacatgac cccaagcacc
1441 gagatgtcat tgagaggagt gagagtcagc aaaatgggag tggatgagta ctccagcact
1501 gagagagtgg tggtgagcat tgaccgtttt ttaagagttc gggatcaaag gggaaacata
1561 ctactgtccc ctgaagaggt cagtgaaaca caaggaacgg aaaagctgac aataatttat
1621 tcatcatcaa tgatgtggga gattaatggt cccgagtcag tgttggtcaa tacttatcaa
1681 tggatcatca gaaactggga aattgtgaaa attcaatggt cacaggatcc cacaatgtta
1741 tacaataaga tagaatttga gccattccag tccctggtcc ctagggccac cagaagccaa
1801 tacagcggtt tcgtaaggac cctgtttcag caaatgcgag atgtacttgg aacatttgac
1861 actgctcaaa taataaaact cctccctttt gccgctgctc ctccggaaca gagtagaatg
1921 cagttctctt ctttgactgt taatgtaaga ggatcgggaa tgaggatact tgtaagaggc
1981 aattccccag tgttcaacta caacaaagcc actaagaggc tcacagtcct cggaaaggat
2041 gcaggtgcgc ttactgaaga cccagatgaa ggtacggctg gagtagaatc tgctgttctg
2101 agagggtttc tcatcttagg taaagaaaac aagagatatg gcccagcact aagcatcaat
2161 gaactgagca aacttacaaa aggggagaaa gctaatgtgc taattgggca aggggacgtg
2221 gtgttggtaa tgaaacggaa acgtgactct agcatactta ctgacagtca gacagcgacc
2281 aaaaggattc ggatggccat caattagtgt tgaattgttt aaaaacgacc ttgtttctac
2341 t

DEFINITION Influenza A/equine/Kentucky/1/87 (H3N8) hemagglutinin (HA) RNA (seg. 4), complete cds.
ACCESSION M24728 J04336; SEQ ID NO: 35

1 agcaaaagca ggggatattt ctgtcaatca tgaagacaac cattgttttg atactactga
61 cccattgggt ctacagtcaa aacccaacca gtggcaacaa cacagccaca ttatgtctgg
121 gacaccatgc agtagcaaat ggaacactgg taaaaacaat aactgatgac cagattgagg
181 tgacaaatgc tactgaatta gttcagagca tttcaatagg gaaaatatgc aacaactcat
241 atagggttct agatggaaga aattgcacat taatagatgc aatgctagga gacccccact
301 gtgatgtttt tcngtatgag aattgggacc tcttcataga aagaagcagc gctttcagca
361 attgctaccc atatgacatc cctgactatg catcgctccg gtctattgtg gcatcctcag
421 gaacattaga attcacagca gagggattca catggacagg tgtcactcaa aacggaagaa
481 gtggagcctg caaaagggga tcagccgata gtttctttag ccgactgaat tggctaacaa
541 aatctggaaa ttcttacccc acattgaatg tgacaatgcc taacaataac aatttcgata
601 aactatacat ctgggggatt catcacccga gctcaaacaa tgagcagaca aaattgtata
661 tccaagaatc aggacgagta acagtctcaa caaaaagaag tcaacaaaca ataatcccca
721 acatcggatc tagaccgtgg gtcaggggtc aatcaggcag gataagcata tattggacca
781 ttgtgaaacc tggagatatc ctaatgataa acagtaatgg caacttagtt gcaccgcggg
841 gatatttcaa attgagaaca gggaaaagct ctgtaatgag atcagatgca cccatagaca
901 cttgtgtgtc tgaatgtatt acaccaaatg gaagcatccc caacgacaaa ccattccaaa
961 atgtgaacaa agttacatat ggaaaatgcc ccaagtatat caggcaaaac actttgaagc
1021 tggccactgg gatgaggaat gtaccagaaa agcaaatcag aggaatcttt ggagcaatag
1081 cgggattcat agaaaacggc tgggaaggaa tggttgatgg gtggtatgga ttccgatatc
1141 aaaattcgga aggaacagga caagctggag atctaaagag cactcaagca gccatcgacc
1201 agatcaatgg aaaattaaac agagtgattg aaaggaccaa tgagaaattc catcaaatag
1261 agaaggaatt ctcagaagta gaagggagaa tccaggactt ggagaagtat gtagaagaca

1321 ccaaaataga cctatggtcc tacaatgcag aattgctggt ggctctagaa aatcaacata
1381 caattgactt aacagatgca gaaatgaata aattattcga gaagactagg cgccagttaa
1441 gagaaaacgc ggaagacatg ggaggtggat gtttcaggat ttaccacaaa tgtgataatg
1501 catgcattgg atcaataaga aatgggacat atgaccatta catatacaga gatgaagcat
1561 taaacaaccg atttcaaatt aaaggtgttg agttgaaatc aggctacaaa gattggatac
1621 tgtggatttc attcgccata tcatgcttct taatttgcgt tgttctattg ggtttcatta
1681 tgtgggcttg ccaaaaaggc aacatcagat gcaacatttg catttgagta aactgatagt
1741 taaaaacacc cttgtttcta ct

DEFINITION Influenza A/equine/Kentucky/2/86 (H3N8) hemagglutinin (HA) RNA (seg. 4), complete cds.
ACCESSION M24727 J04336; SEQ ID NO: 36

1 agcaaaagca ggggatattt ctgtcaatca tgaagacaac cattattttg atactactga
61 cccattgggt ctacagtcaa aacccaacca gtggcaacaa cacagccaca ttatgtctgg
121 gacaccatgc agtagcaaat ggaacattgg taaaaacaat aactgatgac caaattgagg
181 tgacaaatgc tactgaatta gttcagagca tttcaatagg gaaaatatgc aacaactcat
241 atagggttct agatggaaga aattgcacat taatagatgc aatgctagga gacccccact
301 gtgatgtttt tcngtatgag aattgggacc tcttcataga aagaagcagc gcttccagca
361 attgctaccc atatgacatc cctgactatg catcgctccg gtctattgtg gcatcctcag
421 gaacattaga attcacagca gagggattca catggacagg tgtcactcaa aacggaagaa
481 gtggagcctg caaaagggga tcagccgata gtttctttag ccgactgaat tggctaacaa
541 aatctggaaa ttcttacccc acattgaatg tgacaatgcc taacaataac aatttcgata
601 agctatacat ctgggggatc catcacccga gctcaaacaa tgagcagaca aaattgtata
661 tccaagaatc aggacgagta acagtctcaa caaaaagaag tcaacaaaca ataatcccca
721 acatcggatc tagaccgtgg gtcaggggtc aatcaggcag gataagcata tattggacca
781 ttgtgaaacc tggagatatc ctaataataa acagtaatgg caacttagtt gcaccgcggg
841 gatatttcaa attgcgaaca gggaaaagct ctgtaatgag atcagatgca cccatagaca
901 cttgtgtgtc tgaatgtatt acaccaaatg gaagcatccc caacgacaaa ccatttcaaa
961 atgtgaacaa agttacatat ggaaaatgcc ccaagtatat caggcaaaac actttgaagc
1021 tggccactgg gatgaggaat gtaccagaaa agcaaatcag aggaatcttt ggagcaatag
1081 cgggattcat agaaaacggc tgggaaggaa tggttgatgg gtggtatgga ttccgatatc
1141 aaaactcgga aggaacagga caagctggag atctaaagag cactcaagca gccatcgacc
1201 agatcaatgg aaaattgaac agagtgattg aaaggaccaa tgagaaattc catcaaatag
1261 agaaggaatt ctcagaagta gaagggagaa tccaggactt ggagaagtat gtagaagaca
1321 ccaaaataga cctatggtcc tacaatgcag agttgctggt ggctctagaa aatcaacata
1381 caattgactt aacagatgca gaaatgaata aactattcga gaagactagg cgccagttaa
1441 gagaaaacgc ggaagacatg ggaggtggat gtttcaagat ttatcacaaa tgtgataatg
1501 catgcattgg atcaataaga aatgggacat atgaccatta catatacaga gatgaagcat
1561 taaacaaccg atttcaaatt aaaggtgtag agctgaaatc aggctacaaa gattggatac
1621 tgtggatttc attcgccata tcatgcttct taatttgcgt tgttctattg ggtttcatta
1681 tgtgggcttg ccaaaaaggc aacatcagat gcaacatttg catttgagta aactgatagt
1741 taaaaacacc cttgtttcta ct

### EXAMPLE 5: Newmarket Equine Influenza Nucleotide Sequences

DEFINITION Influenza A virus (A/equine 2/Suffolk/89(H3N8)) NS1 gene.
ACCESSION X80060; SEQ ID NO: 37

1 atggattcca acactgtgtc aagctttcag gtagactgtt ttctttggca tgtccgcaaa
61 cgatttgcag accaagaact gggtgatgcc ccattccttg accggcttcg ccgagaccag
121 aagtccctaa aaggaagagg tagcactctt ggtctggaca tcgaaacagc cactcgtgca
181 ggaaagcaga tagtggagca gattctggaa gaggaatcag atgaggcatt taaaatgacc
241 attgcctctg ttcctgcttc acgctactta actgacatga ctcttgatga gatgtcaaga
301 gactggttca tgctcatgcc caagcagaaa gtaacaggct ccctatgtat aagaatggac
361 caggcaatca tggataagaa catcatactt aaagcaaact ttagtgtgat tttcgaaagg
421 ctggagacac taatactact cagggccttc accgaagaag gagcagtcgt tggcgaaatt
481 tcaccattgc cttctcttcc aggacatact aatgaggatg tcaaaaatgc aattggggtc
541 ctcatcggag gacttaaatg gaatgataat acggttagag tctctgaaac tctacagaga

601 ttcgcttgga gaagcagtca tgagaatggg agaccttcat tccctccaaa gcagaaacga
661 aaaatggaga gaacaattga gtcagaagtt tga

DEFINITION Influenza A virus (A/eq/Newmarket/93/(H3N8)) HAI gene for HAY subunit of haemagglutinin, genomic RNA.
ACCESSION X85089; SEQ ID NO: 38

1 atgaagacaa ccattatttt gatactactg acccattggg tctacagtca aaacccaacc
61 agtggcaaca acacagccac attatgtctg ggacaccatg cagtagcaaa tggaacattg
121 gtaaaaacaa taactgatga ccaaattgag gtgacaaatg ctactgaatt agtccagagc
181 atttcaatag ggaaaatatg caacaactca tatagggttc tagatggaag aaattgcaca
241 ttaatagatg caatgctagg agacccccat tgtgatgatt ttcagtatga gaattgggac
301 ctcttcatag aaagaagcag cgctttcagc aattgctacc catatgacat ccctgactat
361 gcatcgctcc ggtccattgt agcatcctca ggaacattag aattcacagc agaggggttc
421 acatggacag gtgtcactca aaacggagga agtggagcct gcaaaagggg atcagccgat
481 agtttcttta gccgactgaa ttggctaaca aaatctggaa attcttaccc catattgaat
541 gtgacaatgc ctaacaataa aaatttcgat aaactataca tctgggggat tcatcacccg
601 agctcaaaca aagagcagac aaaattatat atccaagaat caggacgagt aacagtctca
661 acagaaagaa gtcaacaaac agtaatccct aacatcggat ctaggccgtg ggtcaggggt
721 caatcaggca ggataagcat atactggacc attgtaaaac ctggagatat tctaatgata
781 aacagtaatg gcaacttagt tgcaccgcgg ggatatttta aattgagaac agggaaaagc
841 tctgtaatga gatcagatgc actcatagac acttgtgtgt ctgaatgtat tacaccaaat
901 ggaagcatcc ccaacgacaa accatttcaa aatgtgaaca aaattacata tggaaaatgc
961 cccaagtata tcaggcaaaa cactttaaag ctggccactg ggatgaggaa tgtaccagaa
1021 aagcaaatca ga

DEFINITION Influenza A virus (A/eq/Newmarket/93/(H3N8)) HAI gene for HAY subunit of haemagglutinin, genomic RNA.
ACCESSION X85088; SEQ ID NO: 39

1 atgaagacaa ccattatttt gatactactg acccattggg tctacagtca aaacccaacc
61 agtggcaaca acacagccac attatgtctg ggacaccatg cagtagcaaa tggaacattg
121 gtaaaaacaa taactgatga ccaaattgag gtgacaaatg ctactgaatt agttcagagc
181 atttcaatag ggaaaatatg caacaactca tatagagttc tagatggaag aaattgcaca
241 ttaatagatg caatgctagg agacccccac tgtgatgtct ttcagtatga gaattgggac
301 ctcttcatag aaagaagcag cgctttcagc aattgctacc catatgacat ccctgactat
361 gcatcgctcc ggtccattgt agcatcctca ggaacattgg aattcacagc agagggattc
421 acatggacag gtgtcactca aaacggaaga agtggagcct gcaaaagggg atcagccgat
481 agtttcttta gccgactgaa ttggctaaca aaatctggaa actcttaccc cacattgaat
541 gtgacaatgc ctaacaataa aaatttcgac aaactataca tctgggggat tcatcacccg
601 agctcaaacc aacagcagac agaattgtac atccaagaat caggacgagt aacagtctca
661 acaaaaagaa gtcaacaaac gataatccct aatatcggat ctagaccatg ggtcaggggt
721 caatcaggca ggataagcat atactggacc attgtaaaac ctggagatat cctaatgata
781 aacagtaatg gcaacttagt tgcaccgcgg ggatatttta aattgaaaac agggaaaagc
841 tctgtaatga gatcagatgc acccatagac atttgtgtgt ctgaatgtat tacaccaaat
901 ggaagcatcc ccaacgacaa accatttcaa aatgtgaaca aagttacata tggaaaatgc
961 cccaagtata tcaggcaaaa cactttaaag ctggccactg ggatgaggaa tgtaccagaa
1021 aagcaaatca ga

DEFINITION Influenza A virus (A/eq/Sussex/89/(H3N8)) HAI gene for HAY subunit of haemagglutinin, genomic RNA.
ACCESSION X85090; SEQ ID NO: 40

  1 atgaagacaa ccattatttt gatactactg acccattggg tctacagtca aaacccaacc
 61 agtggcaaca acacagccac attatgtctg ggacaccatg cagtagcaaa tggaacattg
121 gtaaaaacaa taactgatga ccaaattgag gtgacaaatg ctactgaatt agttcagagc
181 atttcaatag ggaaaatatg caacaactca tatagggttc tagatggaag aaattgcaca

241 ttaatagatg caatgctagg agacccccac tgtgatgttt ttcagtatga gaattgggac
301 ctcttcatag aaagaagcag cgctttcagc aattgctacc catatgacat ccctgactat
361 gcatcgctcc ggtccattgt agcatcctca ggaacattag aattcacagc agagggattc
421 acatggacag gtgtcactca aaacggaaga agtggagcct gcaaaagggg atcagccgat
481 agtttcttta gccgactgaa ttggctaaca aaatctggaa attcttaccc catattgaat
541 gtgacaatgc ctaacaataa aaatttcgat aaactataca tctgggggat tcatcacccg
601 agctcaaaca aagagcagac aaaattgtat atccaagaat caggacgagt aacagtctca
661 acagaaagaa gtcaacaaac agtaatccct aacatcggat ctagaccgtg ggtcaggggt
721 caatcaggca ggataagcat atactggacc attgtaaaac ctggagatat tctaatgata
781 aacagtaatg gcaacttagt tgcaccgcgg ggatatttta aattgagaac agggaaaagc
841 tctgtaatga gatcagatgc actcataggc acttgtgtgt ctgaatgtat tacaccaaat
901 ggaagcatcc ccaacgacaa accatttcaa aatgtgaaca aagttacata tggaaaatgc
961 cccaagtata tcaggcaaaa cactttaaag ctggccactg ggatgaggaa tgtaccagaa
1021 aagcaaatca ga

DEFINITION Influenza A virus (A/eq/Lambourn/92/(H3N8)) HAI gene for HAY subunit of haemagglutinin, genomic RNA.
ACCESSION X85087; SEQ ID NO: 41

1 atgaagacaa ccattatttt gatactactg acccattggg tctacagtca aaacccaacc
61 agtggcaaca acacagccac attatgtctg ggacaccatg cagtagcaaa tggaacattg
121 gtaaaaacaa taactgatga ccaaattgag gtgacaaatg ctactgaatt agttcagagc
181 atttcaatag ggaaaatatg caacaactca tatagggttc tagatggaag aaattgcaca
241 ttaatagatg caatgctagg agacccccat tgtgatgatt ttcagtatga gaattgggac
301 ctcttcatag aaagaagcag tgctttcagc aattgctacc catatggcat ccctgactat
361 gcatcgctcc ggtccattgt agcatcctca ggaacattag aattcacagc agaggggttc
421 acatggacag gtgtcactca aaacggaaga agtggagcct gcaaaagggg atcagccgat
481 agtttcttta gccgactcaa ttggctaaca aaatctggaa attcttaccc catattgaat
541 gtgacaatgc ctaacaataa aaatttcgat aaactataca tctgggggat tcatcacccg
601 agctcaaaca aagagcagac aaaattatat atccaagaat caggacgagt aacagtctca
661 acagaaagaa gtcaacaaac agtaatccct aacatcggat ctaggccgtg ggtcaggggt
721 caatcaggca ggataagcat atactggacc attgtaaaac ctggagatat tctaatgata
781 aacaataatg gcaacttagt tgcaccgcgg ggatatttta aattgagaac agggaaaagc
841 tctgtaatga gatcagatgc actcatagac acttgtgtgt ctgaatgtat tacaccaaat
901 ggaagcatcc ccaacgacaa accatttcaa aatgtgaaca aagttacata tggaaaatgc
961 cccaagtata tcaggcaaaa cactttaaag ctggccactg ggatgaggaa tataccagaa
1021 aagcaaatca ga

DEFINITION Influenza A virus (A/eq/E11a/89/(H3N8)) HAI gene for HAY subunit of haemagglutinin, genomic RNA.
ACCESSION X85086; SEQ ID NO: 42

1 atgaagacaa ccattatttt gatactactg acccattggg tctacagtca aaacccaacc
61 agtggcaaca acacagccac attatgtctg ggacaccatg cagtagcaaa tggaacattg
121 gtaaaaacaa taactgatga ccaaattgag gtgacaaatg ctactgaatt agttcagagc
181 atttcaatag ggaaaatatg caacaactca tatagggttc tagatggaag aaattgcaca
241 ttaatagatg caatgctagg agacccccac tgtgatgttt ttcagtatga gaattgggac
301 ctcttcatag aaagaagcag cgctttcagc aattgctacc catatgacat ctctgactat
361 gcatcgctcc ggtccattgt agcatcctca ggaacattag aattcacagc agagggattc
421 acatggacag gtgtcactca aaacggaaga agtggagcct gcaaaagggg atcagccgat
481 agtttcttta gccgactgaa ttggctaaca aaatctggaa attcttaccc catattgaat
541 gtgacaatgc ctaacaataa aaatttcgat aaactataca tctgggggat tcatcacccg
601 agctcaaaca aagagcagac aaaattgtat atccaagaat caggacgagt aacagtctca
661 acagaaagaa gtcaacaaac agtaatccct aacatcggat ctagaccgtg ggtcaggggt
721 caatcaggca ggataagcat atactggacc attgtaaaac ctggagatat tctaatgata
781 aacagtaatg gcaacttagt tgcaccgcgg ggatatttta aattgagaac agggaaaagc
841 tctgtaatga gatcagatgc acccataggc acttgtgtgt ctgaatgtat tacaccaaat
901 ggaagcatcc ccaacgacaa accatttcaa aatgtgaaca aagttacata tggaaaatgc

961 cccaagtata tcaggcaaaa cactttaaag ctggccactg ggatgaggaa tgtaccagaa
1021 aagcaaatca ga

DEFINITION Influenza A virus (A/eq/Arundel/91/(H3N8)) HAI gene for HAY subunit of haemagglutinin, genomic RNA.
ACCESSION X85085; SEQ ID NO: 43

1 atgaagacaa ccattatttt gatactactg acccattggg tctacagtca aaacccaacc
61 agtggcaaca acacagccac attatgtctg ggacaccatg cagtagcaaa tggaacattg
121 gtaaaaacaa taactgatga ccaaattgag gtgacaaatg ctactgaatt agttcagagc
181 atttcaatag ggaaaatatg caacaactca tatagagttc tagatggaag aaattgcaca
241 ttaatagatg caatgctagg agacccccac tgtgatgtct ttcagtatga gaattgggac
301 ctcttcatag aaagaagcag cgctttcagc aattgctacc catatgacat ccctgactat
361 gcatcgctcc ggtccattgt agcatcctca ggaacattag aattcacagc agagggattc
421 acatggacag gtgtcactca aaacggaaga agtggagcct gcaaaagggg atcagccgat
481 agtttcttta gccgactgaa ttggctaaca aaatctggaa attcttaccc catattgaat
541 gtgacaatgc ctaacaataa aaatttcgat aaactataca tctgggggat tcatcacccg
601 agctcaaaca aagagcagac aaaattgtac atccaagaat caggacgagt aacagtctca
661 acaaaaagaa gtcaacaaac aataatccct aacatcggat ctagaccgtg ggtcaggggt
721 caatcaggca ggataagcat atactggacc attgtaaaac ctggagatat cctaatgata
781 aacagtaatg gcaacttagt tgcaccgcgg ggatatttta aattgagaac agggaaaagc
841 tctgtaatga gatcagatgc acccatagac atttgtgtgt ctgaatgtat tacaccaaat
901 ggaagcatcc ccagcgacaa accatttcaa aatgtaaaca aagttacata tggaaaatgc
961 cccaagtata tcaggcaaaa cactttaaag ctggccactg ggatgaggaa tgtaccagaa
1021 aagcaaatca ga

DEFINITION Influenza A virus (A/equine/Suffolk/89(H3N8)) HA gene for haemagglutinin.
ACCESSION X68437; SEQ ID NO: 44

1 ctgtcaatca tgaagacaac cattattttg atactactga cccattgggt ctacagtcaa
61 aacccaacca gtggcaacaa cacagccaca ttatgtctgg gacaccatgc agtagcaaat
121 ggaacattgg taaaaacaat aactgatgac caaattgagg tgacaaatgc tactgaatta
181 gttcagagca tttcaatagg gaaaatatgc aacaactcat atagggttct agatggaaga
241 aattgcacat taatagatgc aatgctagga gacccccact gtgatgtttt tcagtatgag
301 aattgggacc tcttcataga aagaagcagc gctttcagca attgctaccc atatgacatc
361 cctgactatg catcgctccg gtccattgta gcatcctcag gaacattaga attcacagca
421 gagggattca catggacagg tgtcactcaa aacggaagaa gtggagcctg caaaagggga
481 tcagccgata gtttctttag ccgactgaat tggctaacaa aatctggaaa ttcttacccc
541 atattgaatg tgacaatgcc taacaataaa aatttcgata aactatacat ctgggggatt
601 catcacccga gctcaaacaa agagcagaca aaattgtata tccaagaatc aggacgagta
661 acagtctcaa cagaaagaag tcaacaaaca gtaatcccta acatcggatc tagaccgtgg
721 gtcaggggtc aatcaggcag gataagcata tactggacca ttgtaaaacc tggagatatt
781 ctaacgataa acagtaatgg caacttagtt gcaccgcggg gatattttaa attgagaaca
841 gggaaaagct ctgtaatgag atcagatgca cccatagaca cttgtgtgtc tgaatgtatt
901 acaccaaatg gaagcatccc caacgacaaa ccatttcaaa atgtgaacaa agttacatat
961 ggaaaatgcc ccaagtatat caggcaaaac actttaaagc tggccaccgg gatgaggaat
1021 gtaccagaaa agcaaatcag aggaatcttt ggagcaatag cgggattcat agaaaacggc
1081 tgggaaggaa tggttgatgg gtggtatgga ttccgatatc aaaactcgga aggaacagga
1141 caagctgcag atctaaagag cactcaagca gccatcgacc agatcaatgg aaaattaaac
1201 agagtgattg aaaggaccaa tgagaaattc catcaaatag agaaggaatt ctcagaagta
1261 gaagggagaa tccaggattt ggagaagtat gtagaagaca ccaaaataga cctatggtcc
1321 tacaatgcag aattgctggt ggctctagaa aatcaacata caattgactt aacagatgca
1381 gaaatgaata aattattcga gaagactagg cgccagttaa gagaaaacgc ggaagacatg
1441 ggaggtggat gtttcaagat ttaccacaaa tgtgataatg catgcattgg atcaataaga
1501 aatgggacat atgaccatta catatacaga gatgaagcat taaacaaccg atttcaaatc
1561 aaaggtgttg agttgaaatc aggctacaaa gattggatac tgtggatttc attcgccata
1621 tcatgcttct taatttgcgt tgttctattg ggtttcatta tgtgggcttg ccaaaaaggc
1681 aacatcagat gcaacatttg catttgagta aactgatagt taaaaacacc cttgtttcta
1741 ct

DEFINITION Influenza A virus (A/equine/Newmarket/1/77(H7N7)) gene for haemagglutinin.
ACCESSION X62554; SEQ ID NO: 45

1 nnnnnnnnnn nnnnnnnnna aatgaacact cagattctaa tattagccat ttcggcattc
61 ctctgtgtac gtgcagataa aatctgccta ggacatcatg ctgtgtctaa tggaaccaaa
121 gtagacaccc ttactgaaaa gggaatagaa gtcgtcaatg caacagaaac agttgaacaa
181 aaaaacatcc ccaagatctg ctcaaaaggg aaacagacta ttgaccttgg tcaatgtgga
241 ttactaggga ccactattgg tcccccccaa tgcgaccaat ttcttgaatt ctctgctaat
301 ttaataattg agagaagaga aggtgatgat atttgttatc caggcaaatt tgacaatgaa
361 gaaacattga gacaaatact cagaaaatcc ggaggaatta aaaaggagaa tatgggattc
421 acatataccg gagtgagaac caatggagag actagcgcct gtagaaggtc aagatcttcc
481 ttttatgcag aaatgaaatg gctcctatct aacacagaca atggggtatt cccacaaatg
541 acaaaatcct acaagaacac taagaaggag ccagctctga taatctgggg aatccaccac
601 tcaggatcaa ctgctgaaca gactagattg tatggaagtg gaaacaagtt gataacagtt
661 tggagttcca aataccaaca atcttttgcc ccaaaccctg gaccaaggcc gcaaatgaat
721 ggccaatcag gaagaattga cttttactgg ctgatgttag atcccaatga tactgttaat
781 ttcagtttta atggggcctt tatagcacct gaccgcgcca gttttctaag aggtaaatct
841 ctaggaattc agagtgacgc acaacttgac aacaattgtg aaggtgaatg ttatcatatt
901 ggaggtacca taattagcaa cttgcccttt caaaacatta atagcagagc aattgggaaa
961 tgccccagat acgtaaagca aaaaagctta atgctagcaa ccggaatgaa aaatgttcct
1021 gaaaattcta cacacaaaca gttaactcat cacatgcgca aaaaaagagg tttatttggt
1081 gcaatagcag gatttattga aaatggatgg gaaggattaa tagatggatg gtatggatac
1141 agacatcaga atgcacaagg agaaggaact gctgcagact acaaaagtac acaatctgct
1201 gtcaatcaaa taaccgggaa attaaacaga ctaatagaaa aaaccaacca gcaatttgaa
1261 ctaatagata atgaattcaa tgaaatagaa aagcaaattg gcaatgttat taactggact
1321 agagattcta tcatcgaaat atggtcatat aatgcagaat tcctcgtggc agtggagaat
1381 caacacacta ttgatttaac tgattcagag atgaacaaat tatatgaaaa ggtaagaaga
1441 cagctgagag aaaatgctga ggaagatggt aatggctgtt ttgaaatatt tcaccaatgt
1501 gacaatgatt gcatggccag cattagaaac aatacatatg atcataaaaa atacagaaag
1561 gaggcaatac aaaacagaat tcagattgat gcagtaaagt tgagcagcgg ttacaaagaa
1621 ataatacttt ggtttagctt cggggcatca tgtttcttat ttcttgccat tgcaatggtt
1681 cttgctttca tatgcataaa aaatggaaac atgcggtgca ctatttgtat ataagtttga
1741 aaaaacaccc ttgtttctan n

DEFINITION Influenza A virus HA partial gene for haemagglutinin, genomic RNA, strain A/equine/Newmarket-Bob Champion/89(H3N8).
ACCESSION AJ223193; SEQ ID NO: 46

1 agtcaaaacc caaccagtgg caacaacaca gccacattat gtctgggaca ccatgcagta
61 gcaaatggaa cattggtaaa aacaataact gatgaccaaa ttgaggtgac aaatgctact
121 gaattagttc agagcatttc aataggaaaa atatgcaaca actcatatag ggttctagat
181 ggaagaaatt gcacattaat agatgcaatg ctaggagacc cccactgtga tgtttttcag
241 tatgagaatt gggacctctt catagaaaga agcagcgctt tcagcaattg ctacccatat
301 gacatccctg actatgcatc gctccggtcc attgtagcat cctcaggaac attagaattc
361 acagcagagg gattcacatg gacaggtgtc actcaaaacg gaagaagtgg agcctgcaaa
421 aggggatcag ccgatagttt ctttagccga ctgaattggc taacaaaatc tggaaattct
481 taccccatat tgaatgtgac aatgcctaac aataaaaatt tcgataaact atacatctgg
541 gggattcatc acccgagctc aaacaaagag cagacaaaat tgtatatcca agaatcagga
601 cgagtaacag tctcaacaga aagaagtcaa caaacagtaa tccctaacat cggatctaga
661 ccgtgggtca ggggtcaatc aggcaggata agcatatact ggaccattgt aaaacctgga
721 gatattctaa tgataaacag taatggcaac ttagttgctc cgcggggata ttttaaattg
781 agaacaggga aaagctctgt aatgagatca gatgcaccca tagacacttg tgtgtctgaa
841 tgtattacac caaatggaag catccccaac gacaaaccat ttcaaaatgt gaacaaagtt
901 acatatggaa aatgccccaa gtatatcagg caaaacactt taaagctggc cactgggatg
961 aggaatgtac cagaaaagca aatcagagga atctttggag caatagaggg attcatagaa
1021 aacggctggg aaggaatggt tgatgggtgg tatggattcc gatatcaaaa ctcggaagga

1081 acaggacaag ctgcag

DEFINITION Influenza A virus (A/Equine/NewMarket/D64/79(H3N8)) gene for hemagglutinin precursor, partial cds.
ACCESSION D30677; SEQ ID NO: 47

1 ctgtcaatca tgaagacaac cattattttg atactactga cccattgggt ctacagtcaa
61 aacccaacca gtggcaacaa cacagccaca ctatgtctgg gacaccatgc agtagcaaat
121 ggaacattgg taaaaacaat aactgatgac caaattgagg tgacaaatgc cactgaatta
181 gttcagagca cttcaatagg gaaaatatgc aacaacccat atagggttct agatggaaga
241 aactgcacat taatagatgc aatgctagga gatccccact gtgatgtttt tcagtatgag
301 aattgggacc tcttcataga aagaagcagc gctttcagca attgctaccc atatgacatc
361 cctgactatg catcgctccg gtctattgtg gcatcttcag gaacattaga attcacagca
421 gagggattca catggacagg tgtcactcaa aacggaagaa gtggcgcctg cagaagggga
481 tcagccgata gtttctttag ccgactgaat tggctaacaa aatctggaga ttcttacccc
541 acattgaatg tgacaatgcc taacaataac aatttcgata aactatacat ctgggggatc
601 catcacccga gcacaaacaa tgagcagaca aaattgtatg tccaagaatc agggcgagta
661 acagtctcaa caaaaagaag tcaacaaaca ataatcccca acatcggatc tagaccgtgg
721 gtcaggggtc aaccaggcag gataagcata tattggacca ttgtgaaacc tggagatatc
781 ctaatgataa acagtaatgg caacttagtt gcaccgcggg gatatttcaa aatgcgaaca
841 ggaaaaagct ctataatgag atcagatgca cccatagaca cttgtgtgtc cgagtgtatt
901 acaccaaatg gaagcatccc caacgacaaa ccatttcaaa atgtgaacaa agttacatat
961 gggaaatgcc ccaagtatat caagcagaat actttgaagc tggccactgg gatgaggaat
1021 gtaccagaaa agcaaatcag aggaatcttt ggagcaatag cgggattcat agaaaatggc
1081 tgggag

DEFINITION Influenza A virus (A/eq/Newmarket/1/77(H7N7)) matrix proteins M1 and M2 (M) gene, complete cds.
ACCESSION AF001686; SEQ ID NO: 48

1 atgagtcttc tgaccgaggt cgaaacgtac gttctctcta tcgtaccatc aggccccctc
61 aaagccgaga tcgcgcagag acttgaagat gtctttgcag gaaagaacac cgatcttgag
121 gcactcatgg aatggctaaa gacaagacca atcctgtcac ctctgactaa ggggatttta
181 ggatttgtgt tcacgctcac cgtgcccagt gagcgaggac tgcagcgtag acgctttgtc
241 caaaatgccc ttaatgggaa cggagatcca aacaacatgg acagagcagt aaaactgtac
301 aggaagctta aaagggaaat aacattccat ggggcaaaag aggtggcact cagctattcc
361 actggtgcac tagccagctg catgggactc atatacaaca gaatggggac tgtgacaacc
421 gaagtggcat ttggcctggt atgcgccaca tgtgaacaga ttgctgattc ccagcaccga
481 tctcacagac agatggtgac aacaaccaac ccactaatca gacacgagaa cagaatggta
541 ctagccagta ccacagctaa agccatggag cagatggcag ggtcgagtga gcaggcagca
601 gaggccatgg aggttgctag tcaggccagg cagatggtgc aggcaatgag aaccattggg
661 acccacccta gctccagtgc cggtttgaaa aatgatcttc ttgaaaattt gcaggcctac
721 cagaaacgga tgggagtgca aatgcagcga ttcaagtgat cctctcgtta ttgcagcaag
781 tatcattggg atcttgcact tgatattgtg gattcttgat cgtcttttct tcaaatgcat
841 ttatcgtcgt cttaaatacg gtttgaaaag agggccttct acggaaggag tacctgagtc
901 tatgagggaa gaatatcggc aggaacagca gagtgctgtg gatgttgacg atggtcattt
961 tgtcaacata gagctggagt aa

DEFINITION Influenza A virus (A/eq/Newmarket/79(H3N8)) matrix proteins M1 and M2 (M) gene, complete cds.
ACCESSION AF001675; SEQ ID NO: 49

  1 atgagtcttc taaccgaggt cgaaacgtac gttctctcta tcgtaccatc aggcccctc
 61 aaagccgaga tcgcgcagag acttgaagat gtctttgcag ggaagaacac cgatcttgag
121 gcactcatgg aatggctaaa gacaagacca atcctgtcac ctctgactaa agggatttta
181 ggatttgtgt tcacgctcac cgtgcccagt gagcgaggac tgcagcgtag acgctttgtc
241 caaaatgccc ttagtggaaa cggagatcca aacaacatgg acagagcagt aaaactgtac

301 aggaagctta aaagagaaat aacattccat ggggcaaaag aggtggcact cagctattcc
361 actggtgcac tagccagctg catgggactc atatacaaca gaatggggac tgtgacaacc
421 gaagtggcat ttggcctggt atgcgccaca tgtgaacaga ttgctgattc ccagcatcga
481 tctcacaggc agatggtgac aacaaccaac ccactaatca gacatgaaaa cagaatggta
541 ctagccagta ccacagctaa agccatggag cagatggcag ggtcgagtga gcaggcagca
601 gaggccatgg aggttgctag taaggccagg cagatggtac aggcaatgag gaccattggg
661 acccacccta gctccagtgc cggtttgaaa gatgatcttc ttgaaaattt gcaggcctac
721 cagaaacgga tgggagtgca aatgcagcga ttcaagtgat cctctcgtta ttgcagcaag
781 tatcattggg atcttgcact tgatattgtg gattcttgat cgccttttct tcaaattcat
841 ttatcgtcgc cttaaatacg gtttgaaaag agggccttct acggaaggag tacctgagtc
901 tatgagggaa gaatatcggc aggaacagca gaatgctgtg gatgttgacg atggtcattt
961 tgtcaacata gagctggagt aa

DEFINITION Influenza A virus (A/eq/Newmarket/1/77(H7N7)) nonstructural proteins NS1 and NS2 (NS) gene, complete cds.
ACCESSION AF001663; SEQ ID NO: 50

1 atggattcca acactgtgtc aagctttcag gtagactgtt ttctttggca tgtccgcaaa
61 cgatttgcag accaagaact gggtgatgcc ccattccttg accggcttcg ccgagaccag
121 aagtccctaa aaggaagagg cagcactctt ggtctggaca tcgaaacagc cactcatgca
181 ggaaagcaga tagtggagcg aattctggaa gaggaatcag atgaggcact taaaatgacc
241 atagcctctg ttcctacttc acgctactta actgacatga ctcttgatga gatgtcaaga
301 gactggttca tgctcatgcc caagcagaaa gtaacaggct ccctatgtat aaggatggac
361 caagcaatca tggataagaa catcatacta aaagcaaact ttagtgtgat tttcgaaagg
421 ctggagacac taatactact tagagctttc accgaagaag gagcagtcgt tggcgaaatt
481 tcaccattgc cttctcttcc aggacatact aatgaggatg tcaaaaatgc aattggggtc
541 ctcatcggag gacttaaatg gaatgataac acagttagag tctctgaaac tctacagaga
601 ttcgcttgga gaagcagtca tgagaatggg agaccttcat tccctccaaa gcagaaacga
661 aaaatggcga gaacaattga gtcagaagtt tgaagaaata aggtggttga ttgaagaagt
721 gagacataga ttgaaaaata cagaaaatag ttttgaacaa ataacattta tgcaagcctt
781 acaactattg cttgaagtag aacaagagat aagaactttc tcgtttcagc ttatttaa

DEFINITION Influenza A virus (A/eq/Newmarket/D63/79(H3N8)) nonstructural proteins NS1 and NS2 (NS) gene, complete cds.
ACCESSION AF001662; SEQ ID NO: 51

1 atggattcca acactgtgtc aagctttcag gtagactgtt ttctttggca tgtccgcaaa
61 cgatttgcag accaagaact gggtgatgcc ccattccttg accggcttcg ccgagaccag
121 aagtccctaa aaggaagagg cagcactctt ggtctggaca tcgaaacagc cactcgtgca
181 ggaaagcaga tagtggagcg gattctggaa gaggagtcag atgaggcact taaaatgacc
241 attgcctctg ttcctgcttc acgctactta actgacatga ctcttgatga gatgtcaaga
301 gactggttca tgctcatgcc caagcagaaa gtaacaggct ccctatgtat aaggatggac
361 caggcaatca tggataagaa catcatacta aaagcaaact ttagtgtgat tttcgaaagg
421 ctggagacac taatactact tagagccttc accgaagaag gagcagtcgt tggcgaaatt
481 tcaccattgc cttctcttcc aggacatact aatgaggatg tcaaaaatgc aattggggtc
541 ctcatcggag gacttaaatg gaatgataat acagttagag tctctgaaac tctacagaga
601 ttcgcttgga gaagcagtca tgagaatggg agaccttcat tccctccaaa gcagaaacga
661 aaaatggcga gaacaattga gccagaagtt tgaagaaata agatggttga ttgaagaagt
721 gcgacataga ttgaaaaata cagaaaatag ttttgaacaa ataacattta tgcaagcctt
781 acaactattg cttgaagtag aacaagagat aagaactttc tcgtttcagc ttatttaa

### EXAMPLE 6: Efficacy of inactivated influenza virus vaccine in canines

Vaccine active ingredient (AI) is produced either in 9-11 day-old embryonated chicken eggs or in continuous cell cultures such as MDCK cells.

Egg Production, Harvest and Purification. Vaccine AI production in embryonating chicken eggs is prepared by inoculation 0.1 - 0.25 ml of virus suspension containing 100-1000 EID50 (egg infectious dose) into the allantoic cavity. Allantoic fluid is harvested after incubation at 33-37 °C for 2-6 days and pooled. The virus pool is clarified by centrifugation at 1500 - 4000g for 5-15 minutes and filtered through a 20 µm filter (1). The virus maybe concentrated by ultra-filtration using a 300 KDa membrane with a maximun concentration factor of 20 (2).

Further the concentrated virus is optionally purified by zonal ultra-centrifugation on a sucrose gradient followed by filtration of the virus containing fraction through a 0.5 µm membrane. This step can be repeated. The virus containing fraction is diluted in a suitable buffer such as a phosphate buffer (3).

Further purification consists of treatment with Tween/ether at 0.1 % final concentration (w/v)) and 50% final concentration (w/w), respectively. The resulting solution is centrifuged and the aqueous phase is collected. This step can be repeated. The aqueous phase is treated with nitrogen to remove residual ether (4).

Cell Culture Production, Harvest and Purification. Active ingredient is prepared in cells (e.g. MDCK, BHK, PerC6 cells) by inoculation at a rate of MOI of 0.001-1 TCID50. Culture is harvested after incubation at 35-37C for 48-96 hrs when cytopathic effect (CPE) is approximately 50-90%. Culture is sonicated and centrifuged at 1500-4000g for 5-15 minutes (1). The virus is concentrated by ultra-filtration using a 300 KD membrane with a max. concentration factor of 20 (2).

The virus is optionally purified by exclusion chromatography (Sepharose 6 fast flow)(3).

Inactivation. Virus (fraction 1, 2, 3 or 4) are inactivated by addition of formalin or beta-propiolactone to a final concentration of 0.05 % (w/v) or 0.01 % (w/v), respectively and the suspension is held respectively at 5 °C for 16-18 hours or at 20°C for 25-36 hours with continuous agitation.

Active ingredient inactivation is confirmed by inoculation into embryonated chicken eggs or onto MDCK cells.

Preparation of Final Vaccine. Vaccine consists of the inactivated influenza virus and an adjuvant such as aluminium hydroxide, aluminum phosphate, Carbomer or oil-in-water emulsion, which may contain other immunostimulating components such as CpG (1-100 ug/dose). Vaccine typically contains 15-50 µg of hemagglutinin measured by single radial-immunodiffusion (SRD) or >400 haemagglutination Units

The vaccine is administered either by subcutaneous or intramuscular route. Two doses are administered at an interval of 2-5 weeks to pups from 4 weeks of age. Duration of immunity: 6-12 months after a primary vaccination course.

Vaccination/Challenge Protocol. Dogs (less than 4 weeks old) are vaccinated with 2 doses of inactivated vaccine at 3-4 weeks interval via the subcutaneous route. Blood samples are collected on the day of the first and second vaccination and 2-4 weeks after second vaccination to determine the levels of anti-influenza virus specific antibodies using virus neutralization, hemagglutination inhibition, ELISA or Single Radial Heamolysis (SRH) tests.

Two to 4 weeks after 2^{nd} immunization dogs are challenged with a virulent strain of influenza H3N8 to determine efficacy of the vaccines.

Vaccinated and control dogs are challenged by the following route:

By spray: animals are placed into an enclosed container or a mask is placed on the nose of the dogs and the virus suspension is aerosolized to generate 1-100 µM droplets. Animals are kept in the aerosolization chambers for 30 minutes to allow inhalation of the aerosol.

Alternativelly thy can be challenged by one the following routes:
Intra-nasally: 0.5 ml containing 10⁵⁻⁸ EID50 of challenge virus is dropped to each nostril by the aid of dropper.
Intra-tracheally: 1-2 ml containing 10⁵⁻⁸ EID₅₀ virus is directly instilled into the trachea.
Orally: 5- 10 ml containing 10⁵⁻⁸ EID50 of challenge virus is orally administered.

Animals are observed daily for 14 days following challenge for clinical signs including fever, cough, nasal, ocular discharge, respiratory distress, anorexia and lethargy. In addition, groups of animals are euthanized and evaluated for pathological findings of pulmonary and pleural hemorrhage, tracheitis, bronchitis, broncholilitis, and bronchopneumonia.

Tracheal swabs are collected from all animals post challenge days 1-14 for virus isolation.

Presence or absence of viral antigens in respiratory tissues is evaluated by immunohistochemistry on days 3, 7 and 10 post-challenge.

Blood samples are collected on days 7 and 14 post-challenge and are analyzed for the presence of anti-influenza H3N8 virus specific antibody.

By way of reference, the description is further described by the following numbered paragraphs:

## Claims

1. Use of a formulation comprising an avipox expression vector comprising a polynucleotide encoding an equine influenza antigen and a pharmaceutically or veterinarily acceptable carrier, excipient or vehicle in an effective amount for eliciting a protective immune response, for the manufacture of a medicament for eliciting a protective immune response against influenza in a canine;
wherein the avipox expression vector is an attenuated avipox expression vector, wherein the avipox expression vector is a canarypox vector and wherein the canarypox vector is ALVAC; and
wherein the equine influenza antigen is a hemagglutinin wherein the hemagglutinin is H3.

2. A formulation comprising an avipox expression vector comprising a polynucleotide encoding an equine influenza antigen and a pharmaceutically or veterinarily acceptable carrier, excipient or vehicle in an effective amount for eliciting a protective immune response, for use in eliciting a protective immune response against influenza in a canine;
wherein the avipox expression vector is an attenuated avipox expression vector, wherein the avipox expression vector is a canarypox vector and wherein the canarypox vector is ALVAC; and
wherein the influenza antigen is a hemagglutinin wherein the hemagglutinin is H3.

3. Use according to claim 1 or the formulation according to claim 2, wherein the formulation further comprises an adjuvant.

4. Use according to claim 1 or 3 or the formulation according to claim 2 or 3, wherein the influenza antigen is a fragment of the hemagglutinin.

5. Use according to any one of claims 1, 3 or 4 or the formulation according to any one of claims 2 to 4, wherein the influenza antigen is isolated from a canine infected with influenza.

6. Use according to claim 5 or the formulation according to claim 5 wherein the influenza antigen is isolated from a broncho alveolar lavage or lung tissue of the infected canine.

7. Use according to any one of claims 1, 3 or 4 or the formulation according to any one of claims 2 to 4, wherein the influenza antigen is isolated from an equine influenza virus isolate.

8. Use according to claim 7 or the formulation according to claim 7, wherein the equine influenza virus is selected from the group consisting of an Ohio equine influenza virus isolate, a Kentucky equine influenza virus isolate, a Newmarket equine influenza virus isolate or mixtures thereof.

9. Use according to claim 1 or 3 or the formulation according to claim 2 or 3, wherein the canarypox vector is selected from the group consisting of CP1529 or CP1533.

10. Use of a formulation comprising an inactivated influenza vaccine and a pharmaceutically acceptable carrier in an effective amount for eliciting a protective immune response in a canine, for the manufacture of a medicament for eliciting a protective immune response against influenza in a canine; wherein said inactivated influenza vaccine is an inactivated equine influenza of serotype H3; wherein the inactivated influenza vaccine is an inactivated equine influenza isolate and wherein the equine influenza isolate is chosen from the group consisting of an Ohio equine influenza isolate, a Kentucky equine influenza isolate, a Newmarket equine influenza isolate or mixtures thereof.

11. A formulation comprising an inactivated influenza vaccine and a pharmaceutically acceptable carrier in an effective amount for eliciting a protective immune response in a canine, for use in eliciting a protective immune response against influenza in a canine; wherein said inactivated influenza vaccine is an inactivated equine influenza of serotype H3; wherein the inactivated influenza vaccine is an inactivated equine influenza isolate and wherein the equine influenza isolate is chosen from the group consisting of an Ohio equine influenza isolate, a Kentucky equine influenza isolate, a Newmarket equine influenza isolate or mixtures thereof.

12. Use according to claim 10 or the formulation according to claim 11, wherein the formulation further comprises an adjuvant.

13. Use according to claim 12 or the formulation according to claim 12, wherein the adjuvant is aluminum hydroxide, aluminum phosphate, a carbomer or an oil-water-emulsion and optionally further comprises CpG.

14. Use according to any one of claims 10, 12 or 13 or the formulation according to any one of claims 11 to 13, wherein the inactivated influenza vaccine is inactivated with formalin or beta-propiolactone.

15. Use according to any one of claims 10 or 12 to 14 or the formulation according to any one of claims 11 to 14 wherein the medicament or formulation is formulated for subcutaneous or intramuscular administration.

## Patentansprüche

1. Verwendung einer Formulierung, umfassend einen Vogelpocken-Expressionsvektor, der ein Polynucleotid umfasst, das ein Pferdeinfluenza-Antigen codiert, und ein(en) pharmazeutisch oder veterinärmedizinisch verträglichen/es Träger, Excipienten oder Vehikel in einer wirksamen Menge, um eine schützende Immunantwort auszulösen, für die Herstellung eines Medikaments zur Auslösung einer schützenden Immunantwort gegen Influenza bei einem Hund;
wobei der Vogelpocken-Expressionsvektor ein abgeschwächte Vogelpocken-Expressionsvektor ist, wobei der Vogelpocken-Expressionsvektor ein Kanarienpockenvektor ist, und wobei der Kanarienpockenvektor ALVAC ist; und
wobei das Pferdeinfluenza-Antigen ein Hämagglutinin ist, wobei das Hämagglutinin H3 ist.

2. Formulierung, umfassend einen Vogelpocken-Expressionsvektor, der ein Polynucleotid umfasst, das ein Pferdeinfluenza-Antigen codiert, und ein(en) pharmazeutisch oder veterinärmedizinisch verträglichen/es Träger, Excipienten oder Vehikel in einer wirksamen Menge, um eine schützende Immunantwort auszulösen, zur Verwendung bei der Auslösung einer schützenden Immunantwort gegen Influenza bei einem Hund;
wobei der Vogelpocken-Expressionsvektor ein abgeschwächte Vogelpocken-Expressionsvektor ist, wobei der Vogelpocken-Expressionsvektor ein Kanarienpockenvektor ist, und wobei der Kanarienpockenvektor ALVAC ist; und
wobei das Influenzaantigen ein Hämagglutinin ist, wobei das Hämagglutinin H3 ist.

3. Verwendung nach Anspruch 1 oder Formulierung nach Anspruch 2, wobei die Formulierung des Weiteren ein Adjuvans umfasst.

4. Verwendung nach Anspruch 1 oder 3 oder Formulierung nach Anspruch 2 oder 3, wobei das Influenzaantigen ein Fragment des Hämagglutinins ist.

5. Verwendung nach einem der Ansprüche 1, 3 oder 4 oder Formulierung nach einem der Ansprüche 2 bis 4, wobei das Influenzaantigen aus einem mit Influenza infizierten Hund isoliert ist.

6. Verwendung nach Anspruch 5 oder Formulierung nach Anspruch 5, wobei das Influenzaantigen aus einer bronchoalveolären Spülung oder aus Lungengewebe des infizierten Hundes isoliert ist.

7. Verwendung nach einem der Ansprüche 1, 3 oder 4 oder Formulierung nach einem der Ansprüche 2 bis 4, wobei das Influenzaantigen aus einem Pferdeinfluenzavirus-Isolat isoliert ist.

8. Verwendung nach Anspruch 7 oder Formulierung nach Anspruch 7, wobei das Pferdeinfluenzavirus ausgewählt ist aus der Gruppe bestehend aus einem Ohio-Pferdeinfluenzavirus-Isolat, einem Kentucky-Pferdeinfluenzavirus-Isolat, einem Newmarket-Pferdeinfluenzavirus-Isolat oder Gemischen daraus.

9. Verwendung nach Anspruch 1 oder 3 oder Formulierung nach Anspruch 2 oder 3, wobei der Kanarienpockenvektor ausgewählt ist aus der Gruppe bestehend aus CP1529 oder CP1533.

10. Verwendung einer Formulierung, umfassend einen inaktivierten Influenzaimpfstoff und einen pharmazeutisch verträglichen Träger in einer wirksamen Menge, um eine schützende Immunantwort bei einem Hund auszulösen, für die Herstellung eines Medikaments zur Auslösung einer schützenden Immunantwort gegen Influenza bei einem Hund, wobei der inaktivierte Influenzaimpfstoff ein inaktiviertes Pferdeinfluenza vom Serotyp H3 ist; wobei der inaktivierte Influenzaimpfstoff ein inaktiviertes Pferdeinfluenza-Isolat ist und wobei das Pferdeinfluenza-Isolat ausgewählt ist aus der Gruppe bestehend aus einem Ohio-Pferdeinfluenza-Isolat, einem Kentucky-Pferdeinfluenza-Isolat, einem Newmarket-Pferdeinfluenza-Isolat oder Gemischen daraus.

11. Formulierung, umfassend einen inaktivierten Influenzaimpfstoff und einen pharmazeutisch verträglichen Träger in einer wirksamen Menge, um eine schützende Immunantwort bei einem Hund auszulösen, zur Verwendung bei der Auslösung einer schützenden Immunantwort gegen Influenza bei einem Hund; wobei der inaktivierte Influenzaimpfstoff ein inaktiviertes Pferdeinfluenza vom Serotyp H3 ist; wobei der inaktivierte Influenzaimpfstoff ein inaktiviertes Pferdeinfluenza-Isolat ist und wobei das Pferdeinfluenza-Isolat ausgewählt ist aus der Gruppe bestehend aus Ohio-Pferdeinfluenza-Isolat, einem Kentucky-Pferdeinfluenza-Isolat, einem Newmarket-Pferdeinfluenza-Isolat oder Gemischen daraus.

12. Verwendung nach Anspruch 10 oder Formulierung nach Anspruch 11, wobei die Formulierung des Weiteren ein Adjuvans umfasst.

13. Verwendung nach Anspruch 12 oder Formulierung nach Anspruch 12, wobei das Adjuvans Aluminiumhydroxid, Aluminiumphosphat, ein Carbomer oder eine Öl-in-Wasser-Emulsion ist und gegebenenfalls des Weiteren CpG umfasst.

14. Verwendung nach einem der Ansprüche 10, 12 oder 13 oder Formulierung nach einem der Ansprüche 11 bis 13, wobei der inaktivierte Influenzaimpfstoff mit Formalin oder beta-Propiolacton inaktiviert ist.

15. Verwendung nach einem der Ansprüche 10 oder 12 bis 14 oder Formulierung nach einem der Ansprüche 11 bis 14, wobei das Medikament oder die Formulierung für subkutane oder intramuskuläre Verabreichung formuliert ist.

## Revendications

1. Utilisation d'une formulation comprenant un vecteur d'expression d'avipox comprenant un polynucléotide codant pour un antigène de grippe équine et un support, excipient ou véhicule acceptable sur le plan pharmaceutique ou vétérinaire, en une quantité efficace pour déclencher une réponse immunitaire protectrice, pour la fabrication d'un médicament destiné à déclencher une réponse immunitaire protectrice contre la grippe chez un canidé;
où le vecteur d'expression d'avipox est un vecteur d'expression d'avipox atténué,
où le vecteur d'expression d'avipox est un vecteur de canarypox et le vecteur de canarypox est ALVAC; et
où l'antigène de grippe équine est une hémagglutinine, l'hémagglutinine étant H3.

2. Formulation comprenant un vecteur d'expression d'avipox comprenant un polynucléotide codant pour un antigène de grippe équine et un support, excipient ou véhicule acceptable sur le plan pharmaceutique ou vétérinaire, en une quantité efficace pour déclencher une réponse immunitaire protectrice, pour son utilisation pour déclencher une réponse immunitaire protectrice contre la grippe chez un canidé;
où le vecteur d'expression d'avipox est un vecteur d'expression d'avipox atténué,
où le vecteur d'expression d'avipox est un vecteur de canarypox et le vecteur de canarypox est ALVAC; et
où l'antigène de grippe équine est une hémagglutinine, l'hémagglutinine étant H3.

3. Utilisation selon la revendication 1 ou formulation selon la revendication 2, où la formulation comprend en outre un adjuvant.

4. Utilisation selon la revendication 1 ou 3 ou formulation selon la revendication 2 ou 3, où l'antigène de grippe est un fragment de l'hémagglutinine.

5. Utilisation selon l'une quelconque des revendications 1, 3 ou 4, ou formulation selon l'une quelconque des revendications 2 à 4, où l'antigène de grippe est isolé à partir d'un canidé infecté par la grippe.

6. Utilisation selon la revendication 5 ou formulation selon la revendication 5, où l'antigène de grippe est insolé à partir d'un lavage bronchoalvéolaire ou de tissu pulmonaire du canidé infecté.

7. Utilisation selon l'une quelconque des revendications 1, 3 ou 4 ou formulation selon l'une quelconque des revendications 2 à 4, où l'antigène de grippe est isolé à partir d'un isolat de virus de grippe équine.

8. Utilisation selon la revendication 7 ou formulation selon la revendication 7, où le virus de grippe équine est choisi dans le groupe constitué par un isolat de virus de grippe équine Ohio, un isolat de virus de grippe équine Kentucky, un isolat de virus de grippe équine Newmarket ou leurs mélanges.

9. Utilisation selon la revendication 1 ou 3 ou formulation selon la revendication 2 ou 3, où le vecteur de canarypox est choisi dans le groupe constitué par CP1529 ou CP1533.

10. Utilisation d'une formulation comprenant un vaccin grippal inactivé et un support pharmaceutiquement acceptable en une quantité efficace pour déclencher une réponse immunitaire protectrice chez un canidé, pour la fabrication d'un médicament destiné à déclencher une réponse immunitaire protectrice contre la grippe chez un canidé, où ledit vaccin grippal inactivé est un virus de grippe équine inactivé de sérotype H3; où le vaccin grippal inactivé est un isolat de grippe équine inactivé et où l'isolat de grippe équine inactivé est choisi dans le groupe constitué par un isolat de virus de grippe équine Ohio, un isolat de virus de grippe équine Kentucky, un isolat de virus de grippe équine Newmarket ou leurs mélanges.

11. Formulation comprenant un vaccin grippal inactivé et un support pharmaceutiquement acceptable en une quantité efficace pour déclencher une réponse immunitaire protectrice chez un canidé, pour son utilisation pour déclencher une réponse immunitaire protectrice contre la grippe chez un canidé, où ledit vaccin grippal inactivé est un virus de grippe équine inactivé de sérotype H3; où le vaccin grippal inactivé est un isolat de grippe équine inactivé et où l'isolat de grippe équine inactivé est choisi dans le groupe constitué par un isolat de virus de grippe équine Ohio, un isolat de virus de grippe équine Kentucky, un isolat de virus de grippe équine Newmarket ou leurs mélanges.

12. Utilisation selon la revendication 10 ou formulation selon la revendication 11, où la formulation comprend en outre un adjuvant.

13. Utilisation selon la revendication 12 ou formulation selon la revendication 12, où l'adjuvant est l'hydroxyde d'aluminium, le phosphate d'aluminium, un carbomère ou une émulsion de type huile dans l'eau, et comprend éventuellement en outre du CpG.

14. Utilisation selon l'une quelconque des revendications 10, 12 ou 13 ou formulation selon l'une quelconque des revendications 11 à 13, où le vaccin grippal inactivé est inactivé avec de la formaline ou de la β-propiolactone.

15. Utilisation selon l'une quelconque des revendications 10 ou 12 à 14 ou formulation selon l'une quelconque des revendications 11 à 14, où le médicament ou la formulation sont formulés en vue d'une administration sous-cutanée ou intramusculaire.
